# EUROPEAN PATENT APPLICATION

(11) **EP 4 067 377 A1**
(43) Date of publication of application: **05.10.2022**
(21) Application number: 20892522.2
(22) Date of filing: 13.11.2020
(51) Int. Cl.: C07K 16/24, C12N 15/13, C12N 5/18, A61K 39/395, A61P 19/02, A61P 37/06, A61P 37/08

(54) **DEVELOPMENT AND APPLICATION OF THERAPEUTIC AGENTS FOR TSLP-RELATED DISEASES**

(30) Priority: 29.11.2019 CN 201911207253
(71) Applicant: Keymed Biosciences Co.,Ltd., Chengdu, Sichuan 610219 (CN); Shanghai Lingyue Biopharma Co., Ltd., Shanghai 201203 (CN)
(72) Inventor: WANG, Jingkun, Shanghai 201203 (CN); XU, Gang, Chengdu, Sichuan 610219 (CN); CHEN, Bo, Chengdu, Sichuan 610219 (CN)
(74) Representative: Elzaburu S.L.P.
(86) International application number: PCT/CN2020/128821
(87) International publication number: WO 2021/104053

(57) **Abstract**

Provided are an antibody binding to a TSLP protein or an antigen binding part thereof, a manufacturing method therefor and an application thereof. The antibody can bind to human TSLP and/or machin TSLP with high affinity, block the binding of TSLP and TSLPR, and inhibit the transduction of TSLP stimulus signals by means of an STAT5 pathway.

SEQUENCE LISTING

<110>  Keymed Biosciences Co.,Ltd
       Shanghai Lingyue Biopharma Co.,Ltd

<120>  Development and Application of therapeutic agents for TSLP-related
diseases

<130>  MTPIEP19043

<160>  176

<170>  SIPOSequenceListing 1.0

<210>  1
<211>  393
<212>  DNA
<213>  Homo sapiens

<400>  1
tacgacttca ctaactgtga ctttgagaag attaaagcag cctatctcag tactatttct      60

aaagacctga ttacatatat gagtgggacc aaaagtaccg agttcaacaa caccgtctct     120

tgtagcaatc ggccacattg ccttactgaa atccagagcc taaccttcaa tcccaccgcc     180

ggctgcgcgt cgctcgccaa agaaatgttc gccatgaaaa ctaaggctgc cttagctatc     240

tggtgcccag gctattcgga aactcagata aatgctactc aggcaatgaa gaagaggaga     300

aaaaggaaag tcacaaccaa taaatgtctg gaacaagtgt cacaattaca aggattgtgg     360

cgtcgcttca atcgaccttt actgaaacaa cag                                  393


<210>  2
<211>  393
<212>  DNA
<213>  Macaca fascicularis

<400>  2
tacgacttca ccaactgcga cttccagaag atcgaggccg actacctgag aaccatcagc      60

aaggacctga tcacctacat gagcggcacc aagagcaccg acttcaacaa caccgtgtcc     120

tgcagcaaca gaccccactg cctgacagag atccagagcc tgacattcaa ccccacacct     180

agatgtgcca gcctggccaa agagatgttc gccagaaaga ccaaggccac actggccctg     240

tggtgtcctg gctactctga gacacagatc aacgccacac aggccatgaa gaaaaggaga     300

aaaaggaaag tcaccaccaa caagtgcctg gaacaggtgt cccagctgct tggactgtgg     360

cggagattca tcagaaccct gctgaagcag cag                                393

<210>  3
<211>  1116
<212>  DNA
<213>  Homo sapiens

<400>  3
atggggcggc tggttctgct gtggggagct gccgtctttc tgctgggagg ctggatggct      60

ttggggcaag gaggagcagc agaaggagta cagattcaga tcatctactt caatttagaa     120

accgtgcagg tgacatggaa tgccagcaaa tactccagga ccaacctgac tttccactac     180

agattcaacg gtgatgaggc ctatgaccag tgcaccaact accttctcca ggaaggtcac     240

acttcagggt gcctcctaga cgcagagcag cgagacgaca ttctctattt ctccatcagg     300

aatgggacgc accccgtttt caccgcaagt cgctggatgg tttattacct gaaacccagt     360

tccccgaagc acgtgagatt ttcgtggcat caggatgcag tgacggtgac gtgttctgac     420

ctgtcctacg gggatctcct ctatgaggtt cagtaccgga gccccttcga caccgagtgg     480

cagtccaaac aggaaaatac ctgcaacgtc accatagaag gcttggatgc cgagaagtgt     540

tactctttct gggtcagggt gaaggctatg gaggatgtat atgggccaga cacataccca     600

agcgactggt cagaggtgac atgctggcag agaggcgaga ttcgggatgc ctgtgcagag     660

acaccaacgc ctcccaaacc aaagctgtcc aaatttattt taatttccag cctggccatc     720

cttctgatgg tgtctctcct ccttctgtct ttatggaaat tatggagagt gaagaagttt     780

ctcattccca gcgtgccaga cccgaaatcc atcttccccg ggctctttga gatacaccaa     840

gggaacttcc aggagtggat cacagacacc cagaacgtgg cccacctcca caagatggca     900

ggtgcagagc aagaaagtgg ccccgaggag cccctggtag tccagttggc caagactgaa     960

gccgagtctc ccaggatgct ggacccacag accgaggaga aagaggcctc tgggggatcc    1020

ctccagcttc cccaccagcc cctccaaggc ggtgatgtgg tcacaatcgg gggcttcacc    1080

tttgtgatga atgaccgctc ctacgtggcg ttgtga                              1116

<210>  4
<211>  771
<212>  DNA

<213>  Macaca fascicularis

<400>  4
atgggacggt tggttctgct gtggggagct gctgtctttc tgctgggaag ctggatggct      60

ttggggcaag tagcaacagg agaaggacta cagattcaga tcatctactt taatctagaa     120

acggtgcagg tgacatggaa tgccagccac taccccagga gtaacctgag tttccactac     180

aaattcagtc gagatgaggc ctatgaccag tgcaccgtct acattctcca ggaaggtcac     240

acctcggggt gcctcctaga cgcagagcag caagacgata ttctgtattt ctccatcagg     300

aacgggacgc accccgtttt caccgccagt cgctggatct tttattacct gaagcccagt     360

tctccgaagc aggtgagctt ttcgtggcat caggacgcgg tgacagtgac gtgctctgac     420

ctgtcctaca ggggtctcct ctatgaggtt cagtaccgga gccccttcga cacggagtgg     480

cagtccaaac aggaaaatac ctgcaatgtc actatagaag acttggatgc cgagaagtgt     540

tatgctttcc gggcccgggt gaaggccatg gaggatgcgt atgggccaga cacgtacccg     600

agcgactggt cagaggtgac gtgctggcag agaggcaaga ctcgcgactc gtgcccagag     660

cctcgcacgc ctcccaaacc gaagctgtcc aaatttatgt tagtttccag cctggccatc     720

cttctgatgg tgtgtcttct ccttctgtct ttacggaaat tatggaggtg a              771


<210>  5
<211>  209
<212>  PRT
<213>  Homo sapiens

<400>  5

Gln Gly Gly Ala Ala Glu Gly Val Gln Ile Gln Ile Ile Tyr Phe Asn
1               5                   10                  15


Leu Glu Thr Val Gln Val Thr Trp Asn Ala Ser Lys Tyr Ser Arg Thr
            20                  25                  30


Asn Leu Thr Phe His Tyr Arg Phe Asn Gly Asp Glu Ala Tyr Asp Gln
        35                  40                  45


Cys Thr Asn Tyr Leu Leu Gln Glu Gly His Thr Ser Gly Cys Leu Leu
    50                  55                  60

Asp Ala Glu Gln Arg Asp Asp Ile Leu Tyr Phe Ser Ile Arg Asn Gly
65                  70                  75                  80

Thr His Pro Val Phe Thr Ala Ser Arg Trp Met Val Tyr Tyr Leu Lys
                85                  90                  95

Pro Ser Ser Pro Lys His Val Arg Phe Ser Trp His Gln Asp Ala Val
            100                 105                 110

Thr Val Thr Cys Ser Asp Leu Ser Tyr Gly Asp Leu Leu Tyr Glu Val
        115                 120                 125

Gln Tyr Arg Ser Pro Phe Asp Thr Glu Trp Gln Ser Lys Gln Glu Asn
    130                 135                 140

Thr Cys Asn Val Thr Ile Glu Gly Leu Asp Ala Glu Lys Cys Tyr Ser
145                 150                 155                 160

Phe Trp Val Arg Val Lys Ala Met Glu Asp Val Tyr Gly Pro Asp Thr
                165                 170                 175

Tyr Pro Ser Asp Trp Ser Glu Val Thr Cys Trp Gln Arg Gly Glu Ile
            180                 185                 190

Arg Asp Ala Cys Ala Glu Thr Pro Thr Pro Pro Lys Pro Lys Leu Ser
        195                 200                 205

Lys

<210>  6
<211>  209
<212>  PRT
<213>  Macaca fascicularis

<400>  6

Gln Val Ala Thr Gly Glu Gly Leu Gln Ile Gln Ile Ile Tyr Phe Asn
1               5                   10                  15

Leu Glu Thr Val Gln Val Thr Trp Asn Ala Ser His Tyr Pro Arg Ser
            20                  25                  30

Asn Leu Ser Phe His Tyr Lys Phe Ser Arg Asp Glu Ala Tyr Asp Gln
        35                  40                  45

Cys Thr Val Tyr Ile Leu Gln Glu Gly His Thr Ser Gly Cys Leu Leu
    50                  55                  60

Asp Ala Glu Gln Gln Asp Asp Ile Leu Tyr Phe Ser Ile Arg Asn Gly
65                  70                  75                  80

Thr His Pro Val Phe Thr Ala Ser Arg Trp Ile Phe Tyr Tyr Leu Lys
                85                  90                  95

Pro Ser Ser Pro Lys Gln Val Ser Phe Ser Trp His Gln Asp Ala Val
            100                 105                 110

Thr Val Thr Cys Ser Asp Leu Ser Tyr Arg Gly Leu Leu Tyr Glu Val
        115                 120                 125

Gln Tyr Arg Ser Pro Phe Asp Thr Glu Trp Gln Ser Lys Gln Glu Asn
    130                 135                 140

Thr Cys Asn Val Thr Ile Glu Asp Leu Asp Ala Glu Lys Cys Tyr Ala
145                 150                 155                 160

Phe Arg Ala Arg Val Lys Ala Met Glu Asp Ala Tyr Gly Pro Asp Thr
                165                 170                 175

Tyr Pro Ser Asp Trp Ser Glu Val Thr Cys Trp Gln Arg Gly Lys Thr
            180                 185                 190

Arg Asp Ser Cys Pro Glu Pro Arg Thr Pro Pro Lys Pro Lys Leu Ser
        195                 200                 205

Lys

<210>  7
<211>  117
<212>  PRT
<213>  Mus musculus

<400>  7

Gln Val Gln Leu Gln Gln Ser Gly Ala Glu Leu Val Met Pro Gly Ala
1               5                   10                  15

Ser Val Lys Met Ser Cys Lys Ala Ser Gly Tyr Thr Phe Thr Asp Tyr
            20                  25                  30

Trp Met His Trp Val Lys Gln Arg Pro Gly Gln Gly Leu Glu Trp Ile
        35                  40                  45

Gly Thr Ile Asp Asn Ser Asp Ser Asp Thr Thr Tyr Asn Gln Lys Phe
    50                  55                  60

Lys Gly Lys Val Thr Leu Thr Val Asp Glu Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Ser Val Asp Gly Tyr Leu Asp Tyr Trp Gly Gln Gly Thr Thr
            100                 105                 110

Leu Thr Val Ser Ser
        115

<210>  8
<211>  6
<212>  PRT
<213>  Mus musculus

<400>  8

Thr Asp Tyr Trp Met His
1               5

<210>  9
<211>  17

<212>  PRT
<213>  Mus musculus

<400>  9

Thr Ile Asp Asn Ser Asp Ser Asp Thr Thr Tyr Asn Gln Lys Phe Lys
1               5                   10                  15

Gly

<210>  10
<211>  8
<212>  PRT
<213>  Mus musculus

<400>  10

Ser Val Asp Gly Tyr Leu Asp Tyr
1               5

<210>  11
<211>  351
<212>  DNA
<213>  Mus musculus

<400>  11
caggttcagc tgcagcagtc tggggctgag cttgtgatgc ctggggcttc agtgaagatg      60

tcctgcaagg cttctggcta cacattcact gactactgga tgcactgggt gaagcagagg     120

cctggacaag gccttgagtg gattggaacg attgataatt ctgatagtga tacaacctac     180

aatcaaaagt tcaagggcaa ggtcacattg actgtagacg aatcctccag cacagcctac     240

atgcagctca gcagcctgac atctgaggac tctgcggtct attactgtgc aagatcggtt     300

gatggttacc ttgactactg gggccaaggc accactctca cagtctcctc a              351

<210>  12
<211>  107
<212>  PRT
<213>  Mus musculus

<400>  12

Glu Ile Gln Met Thr Gln Ser Pro Ala Ser Leu Ser Ala Ser Val Gly
1               5                   10                  15

Glu Thr Val Thr Ile Thr Cys Arg Thr Ser Glu Asn Ile Tyr Ser Tyr
            20                  25                  30

Leu Ala Trp Tyr Gln Gln Lys Gln Gly Lys Ser Pro Gln Leu Leu Val
        35                  40                  45

Tyr Phe Ala Lys Thr Leu Thr Asp Gly Val Pro Ser Arg Leu Ser Gly
    50                  55                  60

Ser Gly Ser Gly Thr Gln Phe Ser Leu Lys Ile Asn Ser Leu Gln Pro
65                  70                  75                  80

Glu Asp Phe Gly Ser Tyr Tyr Cys Gln His His Tyr Gly Thr Pro Trp
                85                  90                  95

Thr Phe Gly Gly Gly Thr Lys Leu Glu Ile Lys
            100                 105

<210>  13
<211>  11
<212>  PRT
<213>  Mus musculus

<400>  13

Arg Thr Ser Glu Asn Ile Tyr Ser Tyr Leu Ala
1               5                   10

<210>  14
<211>  7
<212>  PRT
<213>  Mus musculus

<400>  14

Phe Ala Lys Thr Leu Thr Asp
1               5

<210>  15
<211>  9
<212>  PRT
<213>  Mus musculus

<400>  15

Gln His His Tyr Gly Thr Pro Trp Thr
1               5

<210>  16
<211>  321
<212>  DNA
<213>  Mus musculus

<400>  16
gaaatccaga tgacccagtc tccagcctcc ctatctgcat ctgtgggaga aactgtcacc        60

atcacatgtc gaacaagtga gaatatttac agttatttag catggtatca gcagaaacag       120

ggaaaatctc ctcagctcct ggtctatttt gcaaaaacct taacagacgg tgtgccatca       180

aggctcagtg gcagtggatc aggcacacag ttttctctga agatcaacag cctgcagcct       240

gaagattttg ggagttatta ctgtcaacat cattatggta ctccgtggac gttcggtgga       300

ggcaccaagc tggaaatcaa a                                                 321

<210>  17
<211>  117
<212>  PRT
<213>  Mus musculus

<400>  17

Gln Val Gln Leu Gln Gln Ser Gly Ala Glu Leu Val Met Pro Gly Ala
1               5                   10                  15

Ser Val Lys Met Ser Cys Lys Ala Ser Gly Tyr Thr Phe Thr Gly Tyr
            20                  25                  30

Trp Met His Trp Val Lys Gln Arg Pro Gly Gln Gly Leu Glu Trp Ile
        35                  40                  45

Gly Thr Ile Asp Asn Ser Asp Ser Asp Thr Thr Tyr Asn Gln Lys Phe
    50                  55                  60

Lys Gly Lys Val Thr Leu Thr Val Asp Glu Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Ser Val Asp Gly Tyr Leu Asp Tyr Trp Gly Gln Gly Thr Thr
            100                 105                 110

Leu Thr Val Ser Ser
        115

<210>  18
<211>  6
<212>  PRT
<213>  Mus musculus

<400>  18

Thr Gly Tyr Trp Met His
1               5

<210>  19
<211>  17
<212>  PRT
<213>  Mus musculus

<400>  19

Thr Ile Asp Asn Ser Asp Ser Asp Thr Thr Tyr Asn Gln Lys Phe Lys
1               5                   10                  15

Gly

<210>  20
<211>  8
<212>  PRT
<213>  Mus musculus

<400>  20

Ser Val Asp Gly Tyr Leu Asp Tyr
1               5

<210>  21
<211>  351

<212>  DNA
<213>  Mus musculus

<400>  21
caggtccagc tgcagcagtc tggggctgag cttgtgatgc ctggggcttc agtgaagatg      60

tcctgcaagg cttctggcta cacattcact ggctactgga tgcactgggt gaagcagagg     120

cctggacaag gccttgagtg gattggaacg attgataatt ctgatagtga tacaacctac     180

aatcaaaagt tcaagggcaa ggtcacattg actgtagacg aatcctccag cacagcctac     240

atgcagctca gcagcctgac atctgaggac tctgcggtct attactgtgc aagatcggtt     300

gatggttacc ttgactactg gggccaaggc accactctca cagtctcctc a              351


<210>  22
<211>  107
<212>  PRT
<213>  Mus musculus

<400>  22

Asp Ile Gln Met Ile Gln Ser Pro Ala Ser Leu Ser Ala Ser Val Gly
1               5                   10                  15


Glu Thr Val Thr Ile Thr Cys Arg Ala Ser Glu Asn Ile Tyr Ser Tyr
            20                  25                  30


Leu Ala Trp Tyr Gln Gln Ile Gln Gly Lys Ser Pro Gln Leu Leu Val
        35                  40                  45


Tyr Tyr Ala Lys Thr Leu Pro Glu Gly Val Pro Ser Arg Phe Ser Gly
    50                  55                  60


Ser Gly Ser Gly Thr Gln Phe Ser Leu Lys Ile Asn Ser Leu Gln Pro
65                  70                  75                  80


Glu Asp Phe Gly Ser Tyr Tyr Cys Gln His His Tyr Gly Thr Pro Trp
                85                  90                  95


Thr Phe Gly Gly Gly Thr Lys Leu Glu Ile Lys
            100                 105

<210>  23
<211>  11
<212>  PRT
<213>  Mus musculus

<400>  23

Arg Ala Ser Glu Asn Ile Tyr Ser Tyr Leu Ala
1               5                   10


<210>  24
<211>  7
<212>  PRT
<213>  Mus musculus

<400>  24

Tyr Ala Lys Thr Leu Pro Glu
1               5


<210>  25
<211>  9
<212>  PRT
<213>  Mus musculus

<400>  25

Gln His His Tyr Gly Thr Pro Trp Thr
1               5


<210>  26
<211>  321
<212>  DNA
<213>  Mus musculus

<400>  26
gacatccaga tgattcagtc tccagcctcc ctatctgcat ctgtgggaga aactgtcacc      60

atcacatgcc gagcaagtga gaatatttac agttatttag catggtatca gcagatacag     120

ggaaaatctc ctcagctcct ggtctattat gcaaaaacct taccagaagg tgtgccatca     180

aggttcagtg gcagtggatc aggcacacag ttttctctga agatcaacag cctgcagcct     240

gaagattttg ggagttatta ctgtcaacat cattacggta ccccgtggac gttcggtgga     300

ggcaccaagc tggaaatcaa a                                               321

<210>  27
<211>  117
<212>  PRT
<213>  Mus musculus

<400>  27

Gln Val Gln Leu Gln Gln Ser Gly Pro Gln Leu Val Arg Pro Gly Ala
1               5                   10                  15

Ser Val Lys Ile Ser Cys Lys Ala Ser Gly Tyr Ser Phe Thr Ser Tyr
            20                  25                  30

Trp Met His Trp Val Lys Gln Arg Pro Gly Gln Gly Leu Glu Trp Ile
        35                  40                  45

Gly Leu Ile Asp Pro Ser Asp Ser Glu Thr Gly Leu Asn Gln Arg Phe
    50                  55                  60

Lys Asp Lys Ala Thr Leu Thr Val Asp Asn Pro Ser Ser Thr Ala Tyr
65                  70                  75                  80

Met Gln Leu Ser Ser Pro Thr Ser Glu Asp Ser Ala Val Tyr Tyr Cys
                85                  90                  95

Ala Arg Ser Leu Asp Gly Tyr Tyr Asp Tyr Trp Gly Gln Gly Thr Thr
            100                 105                 110

Leu Thr Val Ser Ser
        115

<210>  28
<211>  6
<212>  PRT
<213>  Mus musculus

<400>  28

Thr Ser Tyr Trp Met His
1               5

<210>  29
<211>  17

<212>  PRT
<213>  Mus musculus

<400>  29

Leu Ile Asp Pro Ser Asp Ser Glu Thr Gly Leu Asn Gln Arg Phe Lys
1               5                   10                  15

Asp

<210>  30
<211>  8
<212>  PRT
<213>  Mus musculus

<400>  30

Ser Leu Asp Gly Tyr Tyr Asp Tyr
1               5

<210>  31
<211>  351
<212>  DNA
<213>  Mus musculus

<400>  31
caggtccagc tgcagcagtc tgggcctcag ctggttaggc ctggggcttc agtaaagata      60

tcctgcaagg cttctggtta ctcattcacc agctactgga tgcactgggt gaagcagagg     120

cctggacaag gtcttgagtg gattggcttg attgatcctt ccgatagtga aactgggtta     180

aatcagaggt tcaaggacaa ggccacattg actgtagaca atccctccag cacagcctac     240

atgcaactca gcagcccgac atctgaggac tctgcggtct attactgtgc aagatccctt     300

gatggttact atgactactg gggccaaggc accactctca cagtctcctc a              351

<210>  32
<211>  107
<212>  PRT
<213>  Mus musculus

<400>  32

Asp Ile Gln Met Ile Gln Ser Pro Ala Ser Leu Ser Ala Ser Val Gly
1               5                   10                  15

Glu Thr Val Thr Ile Thr Cys Arg Ala Ser Gly Asn Ile His Asn Tyr
            20                  25                  30

Leu Ala Trp Tyr Gln Gln Lys Gln Gly Lys Ser Pro Gln Leu Leu Val
        35                  40                  45

Tyr Asn Ala Lys Thr Leu Ala Asp Gly Val Pro Ser Arg Phe Ser Gly
    50                  55                  60

Ser Gly Ser Gly Thr Gln Tyr Ser Leu Lys Ile Asn Ser Leu Gln Pro
65                  70                  75                  80

Glu Asp Phe Gly Ser Tyr Tyr Cys Gln His Phe Trp Ser Thr Pro Trp
                85                  90                  95

Thr Phe Gly Gly Gly Thr Arg Leu Glu Ile Lys
            100                 105

<210>  33
<211>  11
<212>  PRT
<213>  Mus musculus

<400>  33

Arg Ala Ser Gly Asn Ile His Asn Tyr Leu Ala
1               5                   10

<210>  34
<211>  7
<212>  PRT
<213>  Mus musculus

<400>  34

Asn Ala Lys Thr Leu Ala Asp
1               5

<210>  35
<211>  9
<212>  PRT
<213>  Mus musculus

<400>  35

Gln His Phe Trp Ser Thr Pro Trp Thr
1               5

<210>  36
<211>  321
<212>  DNA
<213>  Mus musculus

<400>  36
gacatccaga tgattcagtc tccagcctcc ctatctgcat ctgtgggaga aactgtcacc        60

atcacatgtc gagcaagtgg gaatattcac aattatttag catggtatca gcagaaacag       120

ggaaaatctc ctcagctcct ggtctataat gcaaaaacct tagcagatgg tgtgccatca       180

aggttcagtg gcagtggatc aggaacacaa tattctctca agatcaacag cctgcagcct       240

gaagattttg ggagttatta ctgtcaacat ttttggagta ctccgtggac gttcggtgga       300

ggcaccagac tggaaataaa a                                                 321

<210>  37
<211>  117
<212>  PRT
<213>  Mus musculus

<400>  37

Gln Val Gln Leu Lys Gln Ser Gly Pro Gln Leu Val Arg Pro Gly Ala
1               5                   10                  15

Ser Val Lys Ile Ser Cys Lys Ala Ser Gly Tyr Ser Phe Thr Ser Tyr
            20                  25                  30

Trp Met His Trp Val Lys Gln Arg Pro Gly Gln Gly Leu Glu Trp Ile
        35                  40                  45

Gly Met Ile Asp Pro Ser Asp Ser Glu Thr Thr Leu Asn Gln Lys Phe
    50                  55                  60

Lys Asp Lys Ala Thr Leu Thr Val Asp Lys Ser Ser Thr Thr Ala Tyr
65                  70                  75                  80

Met Gln Leu Ser Ser Pro Thr Ser Glu Asp Ser Ala Val Tyr Tyr Cys
                85                  90                  95

Ala Arg Ser Leu Asp Gly Tyr Tyr Asp Tyr Trp Gly Gln Gly Thr Thr
            100                 105                 110

Leu Thr Val Ser Ser
        115

<210>  38
<211>  6
<212>  PRT
<213>  Mus musculus

<400>  38

Thr Ser Tyr Trp Met His
1               5

<210>  39
<211>  17
<212>  PRT
<213>  Mus musculus

<400>  39

Met Ile Asp Pro Ser Asp Ser Glu Thr Thr Leu Asn Gln Lys Phe Lys
1               5                   10                  15

Asp

<210>  40
<211>  8
<212>  PRT
<213>  Mus musculus

<400>  40

Ser Leu Asp Gly Tyr Tyr Asp Tyr
1               5

<210>  41
<211>  351

<212>  DNA
<213>  Mus musculus

<400>  41
caggtccagc tgaagcagtc tgggcctcag ttggttaggc ctggggcttc agtgaagata      60

tcctgcaagg cttctggtta ctcattcacc agctactgga tgcactgggt gaagcagagg     120

cctggtcaag gtcttgagtg gattggcatg attgatcctt ccgatagtga aactacgtta     180

aatcagaagt tcaaggacaa ggccacattg actgtagaca aatcctccac cacagcctac     240

atgcaactca gcagcccgac atctgaggac tctgcggtct attactgtgc aagatccctt     300

gatggttact acgactactg gggccaaggc accactctca cagtctcctc a              351


<210>  42
<211>  107
<212>  PRT
<213>  Mus musculus

<400>  42

Asp Val Gln Met Ile Gln Ser Pro Ala Ser Leu Ser Ala Ser Val Gly
1               5                   10                  15


Glu Thr Val Thr Ile Thr Cys Arg Thr Ser Glu Asn Ile Tyr Ser Tyr
            20                  25                  30


Leu Ala Trp Tyr Gln Gln Lys Gln Gly Lys Ser Pro Gln Leu Leu Val
        35                  40                  45


Tyr Phe Ala Lys Thr Leu Thr Asp Gly Val Pro Ser Arg Phe Ser Gly
    50                  55                  60


Ser Gly Ser Gly Thr Gln Phe Ser Leu Lys Ile Asn Ser Leu Gln Pro
65                  70                  75                  80


Glu Asp Phe Gly Ser Tyr Tyr Cys Gln His His Tyr Gly Thr Pro Trp
                85                  90                  95


Thr Phe Gly Gly Gly Thr Lys Leu Glu Ile Lys
            100                 105

<210>  43
<211>  11
<212>  PRT
<213>  Mus musculus

<400>  43

Arg Thr Ser Glu Asn Ile Tyr Ser Tyr Leu Ala
1               5                   10


<210>  44
<211>  7
<212>  PRT
<213>  Mus musculus

<400>  44

Phe Ala Lys Thr Leu Thr Asp
1               5


<210>  45
<211>  9
<212>  PRT
<213>  Mus musculus

<400>  45

Gln His His Tyr Gly Thr Pro Trp Thr
1               5


<210>  46
<211>  321
<212>  DNA
<213>  Mus musculus

<400>  46
gatgtccaga tgattcagtc tccagcctcc ctatctgcat ctgtgggaga aactgtcacc      60

atcacatgtc gaacaagtga gaatatttac agttatttag catggtatca gcagaaacag     120

ggaaaatctc ctcagctcct ggtctatttt gcaaaaacct taacagacgg tgtgccatca     180

aggttcagtg gcagtggatc aggcacacag ttttctctga agatcaacag cctgcagcct     240

gaagattttg ggagttatta ctgtcaacat cattatggta ctccgtggac gttcggtgga     300

ggcaccaagc tggaaatcaa a                                               321

<210>  47
<211>  117
<212>  PRT
<213>  Mus musculus

<400>  47

Gln Val Gln Leu Gln Gln Ser Gly Pro Gln Leu Val Arg Pro Gly Ala
1               5                   10                  15

Ser Val Lys Ile Ser Cys Lys Ala Ser Gly Tyr Ser Phe Thr Ser Tyr
            20                  25                  30

Trp Met His Trp Val Lys Gln Arg Pro Gly Gln Gly Leu Glu Trp Ile
        35                  40                  45

Gly Val Ile Asp Pro Ser Asp Ser Glu Thr Ile Leu Asn Gln Lys Phe
    50                  55                  60

Lys Asp Lys Ala Thr Leu Thr Val Asp Lys Ser Ser Ser Thr Ala Tyr
65                  70                  75                  80

Met Gln Phe Ser Ser Pro Thr Ser Glu Asp Ser Ala Val Tyr Tyr Cys
                85                  90                  95

Ala Arg Ser Leu Asp Gly Tyr Tyr Asp Tyr Trp Gly Gln Gly Thr Thr
            100                 105                 110

Leu Thr Val Ser Ser
        115

<210>  48
<211>  6
<212>  PRT
<213>  Mus musculus

<400>  48

Thr Ser Tyr Trp Met His
1               5

<210>  49
<211>  17

<212>  PRT
<213>  Mus musculus

<400>  49

Val Ile Asp Pro Ser Asp Ser Glu Thr Ile Leu Asn Gln Lys Phe Lys
1               5                   10                  15

Asp

<210>  50
<211>  8
<212>  PRT
<213>  Mus musculus

<400>  50

Ser Leu Asp Gly Tyr Tyr Asp Tyr
1               5

<210>  51
<211>  351
<212>  DNA
<213>  Mus musculus

<400>  51
caggttcagc tgcagcagtc tgggcctcag ctggttaggc ctggggcttc agtgaagata      60

tcctgcaagg cttctggtta ctcattcacc agctactgga tgcactgggt gaagcagagg     120

cctggacaag gtcttgagtg gattggcgtg attgatcctt ccgatagtga gactatatta     180

aatcagaagt tcaaggacaa ggccacattg actgtagaca aatcctccag tacagcctac     240

atgcaattca gcagcccgac atctgaggac tctgcggtct attactgtgc aagatccctt     300

gatggttact acgactactg gggccaaggc accactctca cagtctcctc a              351

<210>  52
<211>  107
<212>  PRT
<213>  Mus musculus

<400>  52

Asp Ile Gln Met Thr Gln Ser Pro Ala Ser Leu Ser Ala Ser Val Gly
1               5                   10                  15

Glu Thr Val Thr Ile Thr Cys Arg Ala Gly Glu Asn Ile Tyr Ser Tyr
            20                  25                  30

Leu Ala Trp Tyr Gln Gln Lys Gln Gly Lys Ser Pro Gln Leu Leu Val
        35                  40                  45

Tyr Asn Ala Lys Thr Leu Ala Glu Gly Val Pro Ser Arg Phe Ser Gly
    50                  55                  60

Ser Ala Ser Gly Thr Gln Phe Ser Leu Lys Ile Asn Ser Leu Gln Pro
65                  70                  75                  80

Glu Asp Phe Gly Ser Tyr Tyr Cys Gln His Phe Trp Gly Thr Pro Trp
                85                  90                  95

Ala Phe Gly Gly Gly Thr Arg Leu Glu Ile Lys
            100                 105

<210>  53
<211>  11
<212>  PRT
<213>  Mus musculus

<400>  53

Arg Ala Gly Glu Asn Ile Tyr Ser Tyr Leu Ala
1               5                   10

<210>  54
<211>  7
<212>  PRT
<213>  Mus musculus

<400>  54

Asn Ala Lys Thr Leu Ala Glu
1               5

<210>  55
<211>  9
<212>  PRT
<213>  Mus musculus

<400>  55

Gln His Phe Trp Gly Thr Pro Trp Ala
1               5

<210>  56
<211>  321
<212>  DNA
<213>  Mus musculus

<400>  56
gacatccaga tgacacagtc tccagcctcc ctatctgcat ctgtgggaga aactgtcacc        60

atcacatgtc gagcaggtga gaatatttac agttatttag catggtatca gcagaaacag       120

ggaaaatctc ctcagctcct ggtctataat gcaaaaacct tagcagaagg tgtgccatca       180

aggttcagtg gcagtgcatc aggcacacag ttttctctga agatcaacag cctgcagcct       240

gaagattttg ggagttatta ctgtcaacat ttttggggta ctccgtgggc gttcggtgga       300

ggcaccagac tggaaataaa a                                                 321

<210>  57
<211>  117
<212>  PRT
<213>  Mus musculus

<400>  57

Gln Val Gln Leu Gln Gln Ser Gly Pro Gln Leu Val Arg Pro Gly Ala
1               5                   10                  15

Ser Val Lys Ile Ser Cys Lys Ala Ser Gly Tyr Ser Phe Thr Thr Tyr
            20                  25                  30

Trp Met His Trp Val Lys Gln Arg Pro Gly Gln Gly Leu Glu Trp Ile
        35                  40                  45

Gly Leu Ile Asp Pro Ser Asp Gly Glu Ala Gly Leu Asn Gln Asn Phe
    50                  55                  60

Lys Asp Lys Ala Thr Leu Thr Val Asp Ile Ser Ser Arg Thr Ala Tyr
65                  70                  75                  80

Met Gln Ile Ser Ser Pro Thr Ser Glu Asp Ser Ala Val Tyr Phe Cys
                85                  90                  95

Ala Arg Ser Leu Asp Gly Tyr Tyr Asp Tyr Trp Gly Gln Gly Thr Thr
            100                 105                 110

Leu Thr Val Ser Ser
        115

<210>  58
<211>  6
<212>  PRT
<213>  Mus musculus

<400>  58

Thr Thr Tyr Trp Met His
1               5

<210>  59
<211>  17
<212>  PRT
<213>  Mus musculus

<400>  59

Leu Ile Asp Pro Ser Asp Gly Glu Ala Gly Leu Asn Gln Asn Phe Lys
1               5                   10                  15

Asp

<210>  60
<211>  8
<212>  PRT
<213>  Mus musculus

<400>  60

Ser Leu Asp Gly Tyr Tyr Asp Tyr
1               5

<210>  61
<211>  351

<212>  DNA
<213>  Mus musculus

<400>  61
caggtccagc tgcagcagtc tgggcctcag ctggttaggc ctggggcttc agtgaagata      60

tcctgcaagg cttctggtta ctcattcacc acctactgga tgcactgggt gaagcagagg      120

cctggacaag gtcttgagtg gattggcttg attgatcctt ccgatggtga agctgggtta      180

aatcagaact tcaaggacaa ggccacattg actgtagaca tttcctccag aacagcctac      240

atgcaaatca gcagcccgac atctgaggac tctgcggttt atttctgtgc aagatccctt      300

gatggttact acgactactg gggccaaggc accactctca cagtctcctc a               351


<210>  62
<211>  107
<212>  PRT
<213>  Mus musculus

<400>  62

Asp Ile Gln Met Thr Gln Ser Pro Ala Ser Leu Ser Ala Ser Val Gly
1               5                   10                  15


Glu Thr Val Thr Ile Thr Cys Arg Ala Ser Glu Asp Ile Tyr Ser Tyr
            20                  25                  30


Leu Ala Trp Tyr Gln Gln Lys Gln Gly Lys Ser Pro Gln Leu Leu Val
        35                  40                  45


Tyr Asn Ala Lys Thr Leu Ala Glu Gly Val Pro Ser Arg Phe Ser Gly
    50                  55                  60


Ser Gly Ser Gly Thr Gln Phe Ser Leu Lys Ile Asn Ser Leu Gln Pro
65                  70                  75                  80


Glu Asp Phe Gly Ser Tyr Tyr Cys Gln His Phe Trp Ser Thr Pro Trp
                85                  90                  95


Thr Phe Gly Gly Gly Thr Lys Leu Glu Leu Lys
            100                 105

<210>  63
<211>  11
<212>  PRT
<213>  Mus musculus

<400>  63

Arg Ala Ser Glu Asp Ile Tyr Ser Tyr Leu Ala
1               5                   10


<210>  64
<211>  7
<212>  PRT
<213>  Mus musculus

<400>  64

Asn Ala Lys Thr Leu Ala Glu
1               5


<210>  65
<211>  9
<212>  PRT
<213>  Mus musculus

<400>  65

Gln His Phe Trp Ser Thr Pro Trp Thr
1               5


<210>  66
<211>  321
<212>  DNA
<213>  Mus musculus

<400>  66
gacatccaga tgacccagtc tccagcctcc ctgtctgcat ctgtgggaga aactgtcacc      60

atcacatgtc gagcaagtga ggatatttac agttatttag catggtatca gcagaaacag     120

ggaaaatctc ctcagctcct ggtctataat gcaaaaacct tagcagaagg tgtgccatca     180

aggttcagtg gcagtggatc aggcacacag ttttctctga agatcaacag cctgcagcct     240

gaagattttg ggagttatta ctgtcaacat ttttggagta ctccgtggac gttcggtgga     300

ggcaccaagc tggagctgaa a                                               321

<210>  67
<211>  117
<212>  PRT
<213>  Mus musculus

<400>  67

Gln Val Gln Leu Leu Gln Ser Gly Pro Gln Leu Val Arg Pro Gly Ala
1               5                   10                  15

Ser Val Lys Ile Ser Cys Lys Ala Ser Gly Tyr Ser Phe Ile Ser Tyr
            20                  25                  30

Trp Ile His Trp Val Lys Gln Arg Pro Gly Gln Gly Leu Glu Trp Ile
        35                  40                  45

Gly Ile Ile Asp Pro Ser Asp Ser Asp Thr Ser Leu Asn Gln Lys Phe
    50                  55                  60

Lys Asp Lys Ala Thr Leu Thr Val Asp Lys Ser Ser Thr Thr Ala Tyr
65                  70                  75                  80

Ile Gln Leu Ser Ser Pro Thr Ser Glu Asp Ser Ala Val Tyr Tyr Cys
                85                  90                  95

Ala Arg Ser Leu Asp Gly Tyr Tyr Asp Tyr Trp Gly Gln Gly Thr Thr
            100                 105                 110

Leu Thr Val Ser Ser
        115

<210>  68
<211>  6
<212>  PRT
<213>  Mus musculus

<400>  68

Ile Ser Tyr Trp Ile His
1               5

<210>  69
<211>  17

<212>  PRT
<213>  Mus musculus

<400>  69

Ile Ile Asp Pro Ser Asp Ser Asp Thr Ser Leu Asn Gln Lys Phe Lys
1               5                   10                  15

Asp

<210>  70
<211>  8
<212>  PRT
<213>  Mus musculus

<400>  70

Ser Leu Asp Gly Tyr Tyr Asp Tyr
1               5

<210>  71
<211>  351
<212>  DNA
<213>  Mus musculus

<400>  71
caggtgcaac tactgcagtc tgggcctcag ctggttaggc ctggggcttc agtgaagata      60

tcctgcaagg cttctggtta ctcattcatt agttactgga tacactgggt gaagcagagg     120

cctggacaag gtcttgagtg gattggcatt attgatcctt ccgatagtga cactagctta     180

aatcagaagt tcaaggacaa ggccacattg actgtggaca aatcctccac cacagcctac     240

attcaactca gcagcccgac atctgaggac tctgcggtct attactgtgc aagatccctt     300

gatggttact acgactactg gggccaaggc accactctca cagtctcctc a              351

<210>  72
<211>  107
<212>  PRT
<213>  Mus musculus

<400>  72

Asp Ile Gln Met Thr Gln Ser Pro Ala Ser Leu Ser Ala Ser Val Gly
1               5                   10                  15

Glu Thr Val Thr Ile Thr Cys Arg Ala Ser Glu Asn Ile Tyr Ser Tyr
            20                  25                  30

Leu Ala Trp Tyr Gln Gln Lys Gln Gly Lys Ser Pro Gln Leu Leu Val
        35                  40                  45

Tyr Asn Ala Arg Thr Leu Pro Glu Gly Val Pro Ser Arg Phe Ser Gly
    50                  55                  60

Ser Gly Ser Gly Thr Gln Phe Ser Leu Lys Ile Asn Ser Leu Gln Pro
65                  70                  75                  80

Glu Asp Phe Gly Ile Tyr Tyr Cys Gln His His Tyr Thr Thr Pro Trp
                85                  90                  95

Thr Phe Gly Gly Gly Thr Lys Leu Glu Ile Lys
            100                 105

<210>  73
<211>  11
<212>  PRT
<213>  Mus musculus

<400>  73

Arg Ala Ser Glu Asn Ile Tyr Ser Tyr Leu Ala
1               5                   10

<210>  74
<211>  7
<212>  PRT
<213>  Mus musculus

<400>  74

Asn Ala Arg Thr Leu Pro Glu
1               5

<210>  75
<211>  9
<212>  PRT
<213>  Mus musculus

<400>  75

Gln His His Tyr Thr Thr Pro Trp Thr
1               5

<210>  76
<211>  321
<212>  DNA
<213>  Mus musculus

<400>  76
gacatccaga tgactcagtc tccagcctcc ctatctgcat ctgtgggaga aactgtcacc        60

atcacatgtc gagcaagtga gaatatttac agttatttag catggtatca gcagaaacag       120

ggaaaatctc ctcaactcct ggtctataat gcaagaacct taccagaagg tgtgccatca       180

cggttcagtg gcagtggatc aggcacacag ttttctctga agatcaacag cctgcagcct       240

gaagattttg ggatttatta ctgtcaacat cattatacta ctccgtggac gttcggtgga       300

ggcaccaagc tggaaatcaa a                                                 321

<210>  77
<211>  117
<212>  PRT
<213>  Homo sapiens

<400>  77

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Ser Phe Ile Ser Tyr
            20                  25                  30

Trp Ile His Trp Val Arg Gln Ala Pro Gly Gln Gly Leu Glu Trp Met
        35                  40                  45

Gly Ile Ile Asp Pro Ser Asp Ser Asp Thr Ser Leu Asn Gln Lys Phe
    50                  55                  60

Gln Gly Arg Val Thr Leu Thr Val Asp Lys Ser Ser Thr Thr Ala Tyr
65                  70                  75                  80

Met Glu Leu Ser Ser Leu Arg Ser Glu Asp Thr Ala Val Tyr Tyr Cys
                85                  90                  95

Ala Arg Ser Leu Asp Gly Tyr Tyr Asp Tyr Trp Gly Gln Gly Thr Leu
            100                 105                 110

Val Thr Val Ser Ser
        115

<210>  78
<211>  6
<212>  PRT
<213>  Homo sapiens

<400>  78

Ile Ser Tyr Trp Ile His
1               5

<210>  79
<211>  17
<212>  PRT
<213>  Homo sapiens

<400>  79

Ile Ile Asp Pro Ser Asp Ser Asp Thr Ser Leu Asn Gln Lys Phe Gln
1               5                   10                  15

Gly

<210>  80
<211>  8
<212>  PRT
<213>  Homo sapiens

<400>  80

Ser Leu Asp Gly Tyr Tyr Asp Tyr
1               5

<210>  81
<211>  351

<212>  DNA
<213>  Homo sapiens

<400>  81
caggttcagc tggttcagtc tggcgccgaa gtgaagaaac ctggcagcag cgtgaaggtg      60

tcctgcaagg ctagcggcta cagcttcatc agctactgga tccactgggt ccgacaggcc     120

cctggacaag gacttgagtg gatgggcatc atcgacccca gcgacagcga cacaagcctg     180

aaccagaaat tccagggcag agtgaccctg accgtggaca agtctagcac caccgcctac     240

atggaactga gcagcctgag aagcgaggac accgccgtgt actactgtgc cagatctctg     300

gacggctact acgactactg gggccaggga acactggtca ccgttagctc t              351


<210>  82
<211>  117
<212>  PRT
<213>  Homo sapiens

<400>  82

Gln Val Gln Leu Val Gln Ser Gly Ala Glu Val Lys Lys Pro Gly Ser
1               5                   10                  15


Ser Val Lys Val Ser Cys Lys Ala Ser Gly Tyr Ser Phe Ile Ser Tyr
            20                  25                  30


Trp Ile His Trp Val Arg Gln Ala Pro Gly Gln Gly Leu Glu Trp Met
        35                  40                  45


Gly Ile Ile Asp Pro Ser Asp Ser Asp Thr Ser Leu Asn Gln Lys Phe
    50                  55                  60


Gln Gly Arg Val Thr Ile Thr Ala Asp Glu Ser Thr Ser Thr Ala Tyr
65                  70                  75                  80


Met Glu Leu Ser Ser Leu Arg Ser Glu Asp Thr Ala Val Tyr Tyr Cys
                85                  90                  95


Ala Arg Ser Leu Asp Gly Tyr Tyr Asp Tyr Trp Gly Gln Gly Thr Leu
            100                 105                 110

Val Thr Val Ser Ser
        115

<210>  83
<211>  6
<212>  PRT
<213>  Homo sapiens

<400>  83

Ile Ser Tyr Trp Ile His
1               5

<210>  84
<211>  17
<212>  PRT
<213>  Homo sapiens

<400>  84

Ile Ile Asp Pro Ser Asp Ser Asp Thr Ser Leu Asn Gln Lys Phe Gln
1               5                   10                  15

Gly

<210>  85
<211>  8
<212>  PRT
<213>  Homo sapiens

<400>  85

Ser Leu Asp Gly Tyr Tyr Asp Tyr
1               5

<210>  86
<211>  351
<212>  DNA
<213>  Homo sapiens

<400>  86
caggttcagc tggttcagtc tggcgccgaa gtgaagaaac ctggcagcag cgtgaaggtg      60

tcctgcaagg ctagcggcta cagcttcatc agctactgga tccactgggt ccgacaggcc     120

cctggacaag gacttgagtg gatgggcatc atcgacccca gcgacagcga cacaagcctg     180

aaccagaaat tccagggcag agtgaccatc accgccgacg agtctaccag caccgcctac 240

atggaactga gcagcctgag aagcgaggac accgccgtgt actactgtgc cagatctctg 300

gacggctact acgattactg gggccaggga accctggtca ccgtttcttc t 351

<210> 87
<211> 117
<212> PRT
<213> Homo sapiens

<400> 87

Glu Val Gln Leu Val Gln Ser Gly Ala Glu Val Lys Lys Pro Gly Glu
1               5                   10                  15

Ser Leu Arg Ile Ser Cys Lys Ala Ser Gly Tyr Ser Phe Ile Ser Tyr
            20                  25                  30

Trp Ile His Trp Val Arg Gln Met Pro Gly Lys Gly Leu Glu Trp Met
        35                  40                  45

Gly Ile Ile Asp Pro Ser Asp Ser Asp Thr Ser Leu Asn Gln Ser Phe
    50                  55                  60

Gln Gly His Val Thr Leu Ser Val Asp Lys Ser Ser Thr Thr Ala Tyr
65                  70                  75                  80

Leu Gln Trp Ser Ser Leu Lys Ala Ser Asp Thr Ala Met Tyr Tyr Cys
                85                  90                  95

Ala Arg Ser Leu Asp Gly Tyr Tyr Asp Tyr Trp Gly Gln Gly Thr Leu
            100                 105                 110

Val Thr Val Ser Ser
        115

<210> 88
<211> 6
<212> PRT
<213> Homo sapiens

<400>  88

Ile Ser Tyr Trp Ile His
1               5

<210>  89
<211>  17
<212>  PRT
<213>  Homo sapiens

<400>  89

Ile Ile Asp Pro Ser Asp Ser Asp Thr Ser Leu Asn Gln Ser Phe Gln
1               5                   10                  15

Gly

<210>  90
<211>  8
<212>  PRT
<213>  Homo sapiens

<400>  90

Ser Leu Asp Gly Tyr Tyr Asp Tyr
1               5

<210>  91
<211>  351
<212>  DNA
<213>  Homo sapiens

<400>  91
gaagtgcagc tggttcagtc tggcgccgaa gtgaagaagc ctggcgagag cctgagaatc      60

agctgcaagg ccagcggcta cagcttcatc agctactgga tccactgggt ccgacagatg     120

cctggcaaag gcctggaatg gatgggcatc atcgacccca gcgacagcga cacaagcctg     180

aaccagtctt tccagggcca cgtgacactg agcgtggaca agtctagcac caccgcctac     240

ctgcagtggt ctagcctgaa ggcctctgac accgccatgt actactgcgc cagatctctg     300

gacggctact acgattactg gggccaggga accctggtca ccgtttcttc t              351

<210>  92

<211>  117
<212>  PRT
<213>  Homo sapiens

<400>  92

Glu Val Gln Leu Val Gln Ser Gly Ala Glu Val Lys Lys Pro Gly Glu
1               5                   10                  15

Ser Leu Arg Ile Ser Cys Lys Ala Ser Gly Tyr Ser Phe Ile Ser Tyr
            20                  25                  30

Trp Ile His Trp Val Arg Gln Met Pro Gly Lys Gly Leu Glu Trp Met
        35                  40                  45

Gly Ile Ile Asp Pro Ser Asp Ser Asp Thr Ser Leu Asn Gln Ser Phe
    50                  55                  60

Gln Gly His Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Ala Arg Ser Leu Asp Gly Tyr Tyr Asp Tyr Trp Gly Gln Gly Thr Leu
            100                 105                 110

Val Thr Val Ser Ser
        115

<210>  93
<211>  6
<212>  PRT
<213>  Homo sapiens

<400>  93

Ile Ser Tyr Trp Ile His
1               5

<210>  94
<211>  17
<212>  PRT

<213>  Homo sapiens

<400>  94

Ile Ile Asp Pro Ser Asp Ser Asp Thr Ser Leu Asn Gln Ser Phe Gln
1               5                   10                  15

Gly

<210>  95
<211>  8
<212>  PRT
<213>  Homo sapiens

<400>  95

Ser Leu Asp Gly Tyr Tyr Asp Tyr
1               5

<210>  96
<211>  351
<212>  DNA
<213>  Homo sapiens

<400>  96
gaagtgcagc tggttcagtc tggcgccgaa gtgaagaagc ctggcgagag cctgagaatc      60

agctgcaagg ccagcggcta cagcttcatc agctactgga tccactgggt ccgacagatg     120

cctggcaaag gcctggaatg gatgggcatc atcgacccca gcgacagcga cacaagcctg     180

aaccagtctt tccagggcca cgtgacaatc agcgccgaca agagcatcag caccgcctac     240

ctgcagtggt ctagcctgaa ggcctctgac accgccatgt actactgcgc cagatctctg     300

gacggctact acgactactg gggccaggga acactggtca ccgttagctc t              351

<210>  97
<211>  117
<212>  PRT
<213>  Homo sapiens

<400>  97

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Phe Thr Asp Tyr
            20                  25                  30

Trp Met His Trp Val Arg Gln Ala Pro Gly Gln Gly Leu Glu Trp Met
        35                  40                  45

Gly Ile Ile Asp Pro Ser Asp Ser Asp Thr Ser Leu Asn Gln Lys Phe
    50                  55                  60

Gln Gly Arg Val Thr Leu Thr Val Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Ser Leu Asp Gly Tyr Tyr Asp Tyr Trp Gly Gln Gly Thr Leu
            100                 105                 110

Val Thr Val Ser Ser
        115

<210>  98
<211>  6
<212>  PRT
<213>  Homo sapiens

<400>  98

Thr Asp Tyr Trp Met His
1               5

<210>  99
<211>  17
<212>  PRT
<213>  Homo sapiens

<400>  99

Ile Ile Asp Pro Ser Asp Ser Asp Thr Ser Leu Asn Gln Lys Phe Gln
1               5                   10                  15

Gly

<210>  100
<211>  8
<212>  PRT
<213>  Homo sapiens

<400>  100

Ser Leu Asp Gly Tyr Tyr Asp Tyr
1               5


<210>  101
<211>  351
<212>  DNA
<213>  Homo sapiens

<400>  101
caggttcagc tggttcagtc tggcgccgaa gtgaagaaac ctggcagcag cgtgaaggtg        60

tcctgcaagg ctagcggcta cacattcacc gactactgga tgcactgggt ccgacaggct       120

ccaggacagg gacttgagtg gatgggcatc atcgacccca gcgacagcga cacaagcctg       180

aaccagaaat tccagggcag agtgaccctg accgtggaca cctctacaag caccgcctac       240

atggaactga gcagcctgag aagcgaggac accgccgtgt actactgtgc cagatctctg       300

gacggctact acgattactg gggccaggga accctggtca ccgtttcttc t                351


<210>  102
<211>  117
<212>  PRT
<213>  Homo sapiens

<400>  102

Gln Val Gln Leu Val Gln Ser Gly Ala Glu Val Lys Lys Pro Gly Ser
1               5                   10                  15


Ser Val Lys Val Ser Cys Lys Ala Ser Gly Tyr Thr Phe Thr Asp Tyr
            20                  25                  30


Trp Met His Trp Val Arg Gln Ala Pro Gly Gln Gly Leu Glu Trp Met
        35                  40                  45

Gly Thr Ile Asp Asn Ser Asp Ser Asp Thr Ser Leu Asn Gln Lys Phe
    50                  55                  60

Gln Gly Arg Val Thr Leu Thr Val Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Ser Leu Asp Gly Tyr Tyr Asp Tyr Trp Gly Gln Gly Thr Leu
            100                 105                 110

Val Thr Val Ser Ser
        115

<210>  103
<211>  6
<212>  PRT
<213>  Homo sapiens

<400>  103

Thr Asp Tyr Trp Met His
1               5

<210>  104
<211>  17
<212>  PRT
<213>  Homo sapiens

<400>  104

Thr Ile Asp Asn Ser Asp Ser Asp Thr Ser Leu Asn Gln Lys Phe Gln
1               5                   10                  15

Gly

<210>  105
<211>  8
<212>  PRT
<213>  Homo sapiens

<400>  105

Ser Leu Asp Gly Tyr Tyr Asp Tyr
1               5

<210>  106
<211>  351
<212>  DNA
<213>  Homo sapiens

<400>  106
caggttcagc tggttcagtc tggcgccgaa gtgaagaaac ctggcagcag cgtgaaggtg        60

tcctgcaagg ctagcggcta cacattcacc gactactgga tgcactgggt ccgacaggct       120

ccaggacagg gacttgagtg gatgggcacc atcgacaaca gcgacagcga cacaagcctg       180

aaccagaaat tccagggcag agtgaccctg accgtggaca cctctacaag caccgcctac       240

atggaactga gcagcctgag aagcgaggac accgccgtgt actactgtgc cagatctctg       300

gacggctact acgattactg gggccaggga accctggtca ccgtttcttc t                351

<210>  107
<211>  117
<212>  PRT
<213>  Homo sapiens

<400>  107

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Phe Thr Asp Tyr
            20                  25                  30

Trp Met His Trp Val Arg Gln Ala Pro Gly Gln Gly Leu Glu Trp Met
        35                  40                  45

Gly Thr Ile Asp Asn Ser Asp Ser Asp Thr Ser Leu Asn Gln Lys Phe
    50                  55                  60

Gln Gly Arg Val Thr Leu Thr Val Asp Glu Ser Ser Ser Thr Ala Tyr
65                  70                  75                  80

Met Glu Leu Ser Ser Leu Arg Ser Glu Asp Thr Ala Val Tyr Tyr Cys

                85                  90                  95

Ala Arg Ser Leu Asp Gly Tyr Tyr Asp Tyr Trp Gly Gln Gly Thr Leu
            100                 105                 110

Val Thr Val Ser Ser
        115

<210>  108
<211>  6
<212>  PRT
<213>  Homo sapiens

<400>  108

Thr Asp Tyr Trp Met His
1               5

<210>  109
<211>  17
<212>  PRT
<213>  Homo sapiens

<400>  109

Thr Ile Asp Asn Ser Asp Ser Asp Thr Ser Leu Asn Gln Lys Phe Gln
1               5                   10                  15

Gly

<210>  110
<211>  8
<212>  PRT
<213>  Homo sapiens

<400>  110

Ser Leu Asp Gly Tyr Tyr Asp Tyr
1               5

<210>  111
<211>  351
<212>  DNA
<213>  Homo sapiens

<400>  111
caggttcagc tggttcagtc tggcgccgaa gtgaagaaac ctggcagcag cgtgaaggtg      60

tcctgcaagg ctagcggcta cacattcacc gactactgga tgcactgggt ccgacaggct      120

ccaggacagg gacttgagtg gatgggcacc atcgacaaca gcgacagcga cacaagcctg      180

aaccagaaat tccagggcag agtgaccctg accgtggaca cctctacaag caccgcctac      240

atggaactga gcagcctgag aagcgaggac accgccgtgt actactgtgc cagatctctg      300

gacggctact acgattactg gggccaggga accctggtca ccgtttcttc t               351


<210>  112
<211>  117
<212>  PRT
<213>  Homo sapiens

<400>  112

Glu Val Gln Leu Val Gln Ser Gly Ala Glu Val Lys Lys Pro Gly Glu
1               5                   10                  15


Ser Leu Lys Ile Ser Cys Lys Ala Ser Gly Tyr Thr Phe Thr Asp Tyr
            20                  25                  30


Trp Met His Trp Val Arg Gln Met Pro Gly Lys Gly Leu Glu Trp Met
        35                  40                  45


Gly Ile Ile Asp Pro Ser Asp Ser Asp Thr Ser Leu Asn Gln Ser Phe
    50                  55                  60


Gln Gly His Val Thr Leu Ser Val Asp Lys Ser Ser Thr Thr Ala Tyr
65                  70                  75                  80


Leu Gln Trp Ser Ser Leu Lys Ala Ser Asp Thr Ala Met Tyr Tyr Cys
                85                  90                  95


Ala Arg Ser Leu Asp Gly Tyr Tyr Asp Tyr Trp Gly Gln Gly Thr Leu
            100                 105                 110


Val Thr Val Ser Ser
        115

<210>  113
<211>  6
<212>  PRT
<213>  Homo sapiens

<400>  113

Thr Asp Tyr Trp Met His
1               5

<210>  114
<211>  17
<212>  PRT
<213>  Homo sapiens

<400>  114

Ile Ile Asp Pro Ser Asp Ser Asp Thr Ser Leu Asn Gln Ser Phe Gln
1               5                   10                  15

Gly

<210>  115
<211>  8
<212>  PRT
<213>  Homo sapiens

<400>  115

Ser Leu Asp Gly Tyr Tyr Asp Tyr
1               5

<210>  116
<211>  351
<212>  DNA
<213>  Homo sapiens

<400>  116
gaagtgcagc tggttcagtc tggcgccgaa gtgaagaagc ctggcgagag cctgaagatc      60

agctgcaagg ccagcggcta caccttcacc gactactgga tgcactgggt ccgacagatg     120

cctggcaaag gcctggaatg gatgggcatc atcgacccca gcgacagcga cacaagcctg     180

aaccagtctt tccagggcca cgtgacactg agcgtggaca agtctagcac caccgcctac     240

ctgcagtggt ctagcctgaa ggcctctgac accgccatgt actactgcgc cagatctctg 300

gacggctact acgattactg gggccaggga accctggtca ccgtttcttc t 351

<210> 117
<211> 117
<212> PRT
<213> Homo sapiens

<400> 117

Glu Val Gln Leu Val Gln Ser Gly Ala Glu Val Lys Lys Pro Gly Glu
1               5                   10                  15

Ser Leu Lys Ile Ser Cys Lys Ala Ser Gly Tyr Thr Phe Thr Asp Tyr
            20                  25                  30

Trp Met His Trp Val Arg Gln Met Pro Gly Lys Gly Leu Glu Trp Met
        35                  40                  45

Gly Thr Ile Asp Asn Ser Asp Ser Asp Thr Ser Leu Asn Gln Ser Phe
    50                  55                  60

Gln Gly His Val Thr Leu Ser Val Asp Lys Ser Ser Thr Thr Ala Tyr
65                  70                  75                  80

Leu Gln Trp Ser Ser Leu Lys Ala Ser Asp Thr Ala Met Tyr Tyr Cys
                85                  90                  95

Ala Arg Ser Leu Asp Gly Tyr Tyr Asp Tyr Trp Gly Gln Gly Thr Leu
            100                 105                 110

Val Thr Val Ser Ser
        115

<210> 118
<211> 6
<212> PRT
<213> Homo sapiens

<400> 118

Thr Asp Tyr Trp Met His
1               5

<210>  119
<211>  17
<212>  PRT
<213>  Homo sapiens

<400>  119

Thr Ile Asp Asn Ser Asp Ser Asp Thr Ser Leu Asn Gln Ser Phe Gln
1               5                   10                  15

Gly

<210>  120
<211>  8
<212>  PRT
<213>  Homo sapiens

<400>  120

Ser Leu Asp Gly Tyr Tyr Asp Tyr
1               5

<210>  121
<211>  351
<212>  DNA
<213>  Homo sapiens

<400>  121
gaagtgcagc tggttcagtc tggcgccgaa gtgaagaagc ctggcgagag cctgaagatc      60

agctgcaagg ccagcggcta caccttcacc gactactgga tgcactgggt ccgacagatg     120

cctggcaaag gcctggaatg gatgggcacc atcgacaaca gcgacagcga cacaagcctg     180

aaccagagct ttcagggcca cgtgacactg agcgtggaca agtctagcac caccgcctac     240

ctgcagtggt ctagcctgaa ggcctctgac accgccatgt actactgcgc cagatctctg     300

gacggctact acgattactg gggccaggga accctggtca ccgtttcttc t              351

<210>  122
<211>  117
<212>  PRT

<213>  Homo sapiens

<400>  122

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Phe Thr Asp Tyr
            20                  25                  30

Trp Met His Trp Val Arg Gln Ala Pro Gly Gln Gly Leu Glu Trp Met
        35                  40                  45

Gly Ile Ile Asp Pro Ser Asp Ser Asp Thr Ser Leu Asn Gln Lys Phe
    50                  55                  60

Gln Gly Arg Val Thr Ile Thr Ala Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Ser Leu Asp Gly Tyr Tyr Asp Tyr Trp Gly Gln Gly Thr Leu
            100                 105                 110

Val Thr Val Ser Ser
        115

<210>  123
<211>  6
<212>  PRT
<213>  Homo sapiens

<400>  123

Thr Asp Tyr Trp Met His
1               5

<210>  124
<211>  17
<212>  PRT
<213>  Homo sapiens

<400>  124

Ile Ile Asp Pro Ser Asp Ser Asp Thr Ser Leu Asn Gln Lys Phe Gln
1               5                   10                  15

Gly

<210>  125
<211>  8
<212>  PRT
<213>  Homo sapiens

<400>  125

Ser Leu Asp Gly Tyr Tyr Asp Tyr
1               5

<210>  126
<211>  351
<212>  DNA
<213>  Homo sapiens

<400>  126
caggttcagc tggttcagtc tggcgccgaa gtgaagaaac ctggcagcag cgtgaaggtg      60

tcctgcaagg ctagcggcta cacattcacc gactactgga tgcactgggt ccgacaggct     120

ccaggacagg gacttgagtg gatgggcatc atcgacccca gcgacagcga cacaagcctg     180

aaccagaaat tccagggcag agtgaccatc accgccgaca cctctacaag caccgcctac     240

atggaactga gcagcctgag aagcgaggac accgccgtgt actactgtgc cagatctctg     300

gacggctact acgattactg gggccaggga accctggtca ccgtttcttc t              351

<210>  127
<211>  117
<212>  PRT
<213>  Homo sapiens

<400>  127

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Phe Thr Asp Tyr

20                  25                  30

Trp Met His Trp Val Arg Gln Ala Pro Gly Gln Gly Leu Glu Trp Met
        35                  40                  45

Gly Thr Ile Asp Asn Ser Asp Ser Asp Thr Ser Leu Asn Gln Lys Phe
    50                  55                  60

Gln Gly Arg Val Thr Ile Thr Ala Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Ser Leu Asp Gly Tyr Tyr Asp Tyr Trp Gly Gln Gly Thr Leu
            100                 105                 110

Val Thr Val Ser Ser
        115

<210>  128
<211>  6
<212>  PRT
<213>  Homo sapiens

<400>  128

Thr Asp Tyr Trp Met His
1               5

<210>  129
<211>  17
<212>  PRT
<213>  Homo sapiens

<400>  129

Thr Ile Asp Asn Ser Asp Ser Asp Thr Ser Leu Asn Gln Lys Phe Gln
1               5                   10                  15

Gly

<210>  130
<211>  8
<212>  PRT
<213>  Homo sapiens

<400>  130

Ser Leu Asp Gly Tyr Tyr Asp Tyr
1               5


<210>  131
<211>  351
<212>  DNA
<213>  Homo sapiens

<400>  131
caggttcagc tggttcagtc tggcgccgaa gtgaagaaac ctggcagcag cgtgaaggtg        60

tcctgcaagg ctagcggcta cacattcacc gactactgga tgcactgggt ccgacaggct       120

ccaggacagg gacttgagtg gatgggcacc atcgacaaca gcgacagcga cacaagcctg       180

aaccagaaat tccagggcag agtgaccatc accgccgaca cctctacaag caccgcctac       240

atggaactga gcagcctgag aagcgaggac accgccgtgt actactgtgc cagatctctg       300

gacggctact acgattactg gggccaggga accctggtca ccgtttcttc t                351


<210>  132
<211>  117
<212>  PRT
<213>  Homo sapiens

<400>  132

Gln Val Gln Leu Val Gln Ser Gly Ala Glu Val Lys Lys Pro Gly Ser
1               5                   10                  15


Ser Val Lys Val Ser Cys Lys Ala Ser Gly Tyr Thr Phe Thr Asp Tyr
            20                  25                  30


Trp Met His Trp Val Arg Gln Ala Pro Gly Gln Gly Leu Glu Trp Met
        35                  40                  45


Gly Thr Ile Asp Asn Ser Asp Ser Asp Thr Ser Leu Asn Gln Lys Phe
    50                  55                  60

Gln Gly Arg Val Thr Ile Thr Ala Asp Glu Ser Ser Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Ser Leu Asp Gly Tyr Tyr Asp Tyr Trp Gly Gln Gly Thr Leu
            100                 105                 110

Val Thr Val Ser Ser
        115

<210>  133
<211>  6
<212>  PRT
<213>  Homo sapiens

<400>  133

Thr Asp Tyr Trp Met His
1               5

<210>  134
<211>  17
<212>  PRT
<213>  Homo sapiens

<400>  134

Thr Ile Asp Asn Ser Asp Ser Asp Thr Ser Leu Asn Gln Lys Phe Gln
1               5                   10                  15

Gly

<210>  135
<211>  8
<212>  PRT
<213>  Homo sapiens

<400>  135

Ser Leu Asp Gly Tyr Tyr Asp Tyr

1               5


<210>  136
<211>  351
<212>  DNA
<213>  Homo sapiens

<400>  136
caggttcagc tggttcagtc tggcgccgaa gtgaagaaac ctggcagcag cgtgaaggtg        60

tcctgcaagg ctagcggcta cacattcacc gactactgga tgcactgggt ccgacaggct       120

ccaggacagg gacttgagtg gatgggcacc atcgacaaca gcgacagcga cacaagcctg       180

aaccagaaat tccagggcag agtgaccatc accgccgacg agtctagcag caccgcctac       240

atggaactga gcagcctgag aagcgaggac accgccgtgt actactgtgc cagatctctg       300

gacggctact acgattactg gggccaggga accctggtca ccgtttcttc t                351


<210>  137
<211>  117
<212>  PRT
<213>  Homo sapiens

<400>  137

Glu Val Gln Leu Val Gln Ser Gly Ala Glu Val Lys Lys Pro Gly Glu
1               5                   10                  15


Ser Leu Lys Ile Ser Cys Lys Ala Ser Gly Tyr Thr Phe Thr Asp Tyr
            20                  25                  30


Trp Met His Trp Val Arg Gln Met Pro Gly Lys Gly Leu Glu Trp Met
        35                  40                  45


Gly Ile Ile Asp Pro Ser Asp Ser Asp Thr Ser Leu Asn Gln Ser Phe
    50                  55                  60


Gln Gly His Val Thr Ile Ser Ala Asp Lys Ser Ile Ser Thr Ala Tyr
65                  70                  75                  80


Leu Gln Trp Ser Ser Leu Lys Ala Ser Asp Thr Ala Met Tyr Tyr Cys
                85                  90                  95

Ala Arg Ser Leu Asp Gly Tyr Tyr Asp Tyr Trp Gly Gln Gly Thr Leu
            100                 105                 110

Val Thr Val Ser Ser
        115

<210>  138
<211>  6
<212>  PRT
<213>  Homo sapiens

<400>  138

Thr Asp Tyr Trp Met His
1               5

<210>  139
<211>  17
<212>  PRT
<213>  Homo sapiens

<400>  139

Ile Ile Asp Pro Ser Asp Ser Asp Thr Ser Leu Asn Gln Ser Phe Gln
1               5                   10                  15

Gly

<210>  140
<211>  8
<212>  PRT
<213>  Homo sapiens

<400>  140

Ser Leu Asp Gly Tyr Tyr Asp Tyr
1               5

<210>  141
<211>  351
<212>  DNA
<213>  Homo sapiens

<400>  141

gaagtgcagc tggttcagtc tggcgccgaa gtgaagaagc ctggcgagag cctgaagatc 60

agctgcaagg ccagcggcta caccttcacc gactactgga tgcactgggt ccgacagatg 120

cctggcaaag gcctggaatg gatgggcatc atcgacccca gcgacagcga cacaagcctg 180

aaccagtctt tccagggcca cgtgacaatc agcgccgaca agagcatcag caccgcctac 240

ctgcagtggt ctagcctgaa ggcctctgac accgccatgt actactgcgc cagatctctg 300

gacggctact acgactactg gggccaggga acactggtca ccgttagctc t 351

<210> 142
<211> 117
<212> PRT
<213> Homo sapiens

<400> 142

Glu Val Gln Leu Val Gln Ser Gly Ala Glu Val Lys Lys Pro Gly Glu
1               5                   10                  15

Ser Leu Lys Ile Ser Cys Lys Ala Ser Gly Tyr Thr Phe Thr Asp Tyr
            20                  25                  30

Trp Met His Trp Val Arg Gln Met Pro Gly Lys Gly Leu Glu Trp Met
        35                  40                  45

Gly Thr Ile Asp Asn Ser Asp Ser Asp Thr Ser Leu Asn Gln Ser Phe
    50                  55                  60

Gln Gly His Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Ala Arg Ser Leu Asp Gly Tyr Tyr Asp Tyr Trp Gly Gln Gly Thr Leu
            100                 105                 110

Val Thr Val Ser Ser
        115

<210>  143
<211>  6
<212>  PRT
<213>  Homo sapiens

<400>  143

Thr Asp Tyr Trp Met His
1               5

<210>  144
<211>  17
<212>  PRT
<213>  Homo sapiens

<400>  144

Thr Ile Asp Asn Ser Asp Ser Asp Thr Ser Leu Asn Gln Ser Phe Gln
1               5                   10                  15

Gly

<210>  145
<211>  8
<212>  PRT
<213>  Homo sapiens

<400>  145

Ser Leu Asp Gly Tyr Tyr Asp Tyr
1               5

<210>  146
<211>  351
<212>  DNA
<213>  Homo sapiens

<400>  146
gaagtgcagc tggttcagtc tggcgccgaa gtgaagaagc ctggcgagag cctgaagatc      60

agctgcaagg ccagcggcta caccttcacc gactactgga tgcactgggt ccgacagatg     120

cctggcaaag gcctggaatg gatgggcacc atcgacaaca gcgacagcga cacaagcctg     180

aaccagagct ttcagggcca cgtgacaatc agcgccgaca agtctatcag caccgcctac     240

ctgcagtggt ctagcctgaa ggcctctgac accgccatgt actactgcgc cagatctctg     300

gacggctact acgactactg gggccaggga acactggtca ccgttagctc t                351

<210>  147
<211>  117
<212>  PRT
<213>  Homo sapiens

<400>  147

Glu Val Gln Leu Val Gln Ser Gly Ala Glu Val Lys Lys Pro Gly Glu
1               5                   10                  15

Ser Leu Lys Ile Ser Cys Lys Ala Ser Gly Tyr Thr Phe Thr Asp Tyr
            20                  25                  30

Trp Met His Trp Val Arg Gln Met Pro Gly Lys Gly Leu Glu Trp Met
        35                  40                  45

Gly Thr Ile Asp Asn Ser Asp Ser Asp Thr Ser Leu Asn Gln Ser Phe
    50                  55                  60

Gln Gly His Val Thr Ile Ser Ala Asp Lys Ser Ser Thr Thr Ala Tyr
65                  70                  75                  80

Leu Gln Trp Ser Ser Leu Lys Ala Ser Asp Thr Ala Met Tyr Tyr Cys
                85                  90                  95

Ala Arg Ser Leu Asp Gly Tyr Tyr Asp Tyr Trp Gly Gln Gly Thr Leu
            100                 105                 110

Val Thr Val Ser Ser
        115

<210>  148
<211>  6
<212>  PRT
<213>  Homo sapiens

<400>  148

Thr Asp Tyr Trp Met His
1               5

<210>  149
<211>  17
<212>  PRT
<213>  Homo sapiens

<400>  149

Thr Ile Asp Asn Ser Asp Ser Asp Thr Ser Leu Asn Gln Ser Phe Gln
1               5                   10                  15

Gly

<210>  150
<211>  8
<212>  PRT
<213>  Homo sapiens

<400>  150

Ser Leu Asp Gly Tyr Tyr Asp Tyr
1               5

<210>  151
<211>  351
<212>  DNA
<213>  Homo sapiens

<400>  151
gaagtgcagc tggttcagtc tggcgccgaa gtgaagaagc ctggcgagag cctgaagatc      60

agctgcaagg ccagcggcta caccttcacc gactactgga tgcactgggt ccgacagatg     120

cctggcaaag gcctggaatg gatgggcacc atcgacaaca gcgacagcga cacaagcctg     180

aaccagagct ttcagggcca cgtgacaatc agcgccgaca agtctagcac caccgcctac     240

ctgcagtggt ctagcctgaa ggcctctgac accgccatgt actactgcgc cagatctctg     300

gacggctact acgattactg gggccaggga accctggtca ccgtttcttc t              351

<210>  152
<211>  117
<212>  PRT
<213>  Homo sapiens

<400>  152

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Phe Thr Asp Tyr
            20                  25                  30

Trp Met His Trp Val Arg Gln Ala Pro Gly Gln Gly Leu Glu Trp Met
        35                  40                  45

Gly Thr Ile Asp Asn Ser Asp Ser Asp Thr Thr Tyr Asn Pro Ser Phe
    50                  55                  60

Gln Gly His Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Ala Arg Ser Val Asp Gly Tyr Leu Asp Tyr Trp Gly Gln Gly Thr Leu
            100                 105                 110

Val Thr Val Ser Ser
        115

<210>  153
<211>  6
<212>  PRT
<213>  Homo sapiens

<400>  153

Thr Asp Tyr Trp Met His
1               5

<210>  154
<211>  17
<212>  PRT
<213>  Homo sapiens

<400>  154

Thr Ile Asp Asn Ser Asp Ser Asp Thr Thr Tyr Asn Pro Ser Phe Gln
1               5                   10                  15

Gly

<210>  155
<211>  8
<212>  PRT
<213>  Homo sapiens

<400>  155

Ser Val Asp Gly Tyr Leu Asp Tyr
1               5

<210>  156
<211>  351
<212>  DNA
<213>  Homo sapiens

<400>  156
caggttcagc tggttcagtc tggcgccgaa gtgaagaaac ctggcagcag cgtgaaggtg      60

tcctgcaagg ctagcggcta cacattcacc gactactgga tgcactgggt ccgacaggct     120

ccaggacagg gacttgagtg gatgggcacc atcgacaaca gcgacagcga cacaacctac     180

aacccatctt tccagggcca cgtgacaatc agcgccgaca agagcatcag caccgcctac     240

ctgcagtggt ctagcctgaa ggcctctgac accgccatgt actactgtgc cagatctgtg     300

gacggctacc tggattactg gggccaggga accctggtca ccgtttcttc t              351

<210>  157
<211>  117
<212>  PRT
<213>  Homo sapiens

<400>  157

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Phe Thr Asp Tyr
            20                  25                  30

Trp Met His Trp Val Arg Gln Ala Pro Gly Gln Gly Leu Glu Trp Met
        35                  40                  45

Gly Thr Ile Asp Asn Ser Asp Ser Asp Thr Thr Tyr Asn Gln Lys Phe
    50                  55                  60

Gln Gly Arg Val Thr Leu Thr Val Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Ser Val Asp Gly Tyr Leu Asp Tyr Trp Gly Gln Gly Thr Leu
            100                 105                 110

Val Thr Val Ser Ser
        115

<210>  158
<211>  6
<212>  PRT
<213>  Homo sapiens

<400>  158

Thr Asp Tyr Trp Met His
1               5

<210>  159
<211>  17
<212>  PRT
<213>  Homo sapiens

<400>  159

Thr Ile Asp Asn Ser Asp Ser Asp Thr Thr Tyr Asn Gln Lys Phe Gln
1               5                   10                  15

Gly

<210>  160

<211>  8
<212>  PRT
<213>  Homo sapiens

<400>  160

Ser Val Asp Gly Tyr Leu Asp Tyr
1               5


<210>  161
<211>  351
<212>  DNA
<213>  Homo sapiens

<400>  161
caggttcagc tggttcagtc tggcgccgaa gtgaagaaac ctggcagcag cgtgaaggtg      60

tcctgcaagg ctagcggcta cacattcacc gactactgga tgcactgggt ccgacaggct     120

ccaggacagg gacttgagtg gatgggcacc atcgacaaca gcgacagcga cacaacctac     180

aaccagaaat tccagggcag agtgaccctg accgtggaca cctctacaag caccgcctac     240

atggaactga gcagcctgag aagcgaggac accgccgtgt actactgtgc cagatctgtg     300

gacggctacc tggattactg gggccaggga accctggtca ccgtttcttc t              351


<210>  162
<211>  117
<212>  PRT
<213>  Homo sapiens

<400>  162

Gln Val Gln Leu Val Gln Ser Gly Ala Glu Val Lys Lys Pro Gly Ser
1               5                   10                  15


Ser Val Lys Val Ser Cys Lys Ala Ser Gly Tyr Thr Phe Thr Asp Tyr
            20                  25                  30


Trp Met His Trp Val Arg Gln Ala Pro Gly Gln Gly Leu Glu Trp Met
        35                  40                  45


Gly Thr Ile Asp Asn Ser Asp Ser Asp Thr Thr Tyr Asn Gln Lys Phe
    50                  55                  60

Gln Gly Arg Val Thr Met Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Arg Ser Val Asp Gly Tyr Leu Asp Tyr Trp Gly Gln Gly Thr Leu
            100                 105                 110

Val Thr Val Ser Ser
        115

<210>  163
<211>  6
<212>  PRT
<213>  Homo sapiens

<400>  163

Thr Asp Tyr Trp Met His
1               5

<210>  164
<211>  17
<212>  PRT
<213>  Homo sapiens

<400>  164

Thr Ile Asp Asn Ser Asp Ser Asp Thr Thr Tyr Asn Gln Lys Phe Gln
1               5                   10                  15

Gly

<210>  165
<211>  8
<212>  PRT
<213>  Homo sapiens

<400>  165

Ser Val Asp Gly Tyr Leu Asp Tyr
1               5

<210>  166
<211>  351
<212>  DNA
<213>  Homo sapiens

<400>  166
caggttcagc tggttcagtc tggcgccgaa gtgaagaaac ctggcagcag cgtgaaggtg      60

tcctgcaagg ctagcggcta cacattcacc gactactgga tgcactgggt ccgacaggct     120

ccaggacagg gacttgagtg gatgggcacc atcgacaaca gcgacagcga cacaacctac     180

aaccagaaat tccagggcag agtgaccatg accagagaca cctctacaag caccgtctac     240

atggaactga gcagcctgag aagcgaggac accgccgtgt actactgtgc cagatctgtg     300

gacggctacc tggattactg gggccaggga accctggtca ccgtttcttc t              351


<210>  167
<211>  107
<212>  PRT
<213>  Homo sapiens

<400>  167

Asp Ile Gln Met Thr Gln Ser Pro Ser Ser Leu Ser Ala Ser Val Gly
1               5                   10                  15


Asp Arg Val Thr Ile Thr Cys Arg Ala Ser Glu Asn Ile Tyr Ser Tyr
            20                  25                  30


Leu Ala Trp Tyr Gln Gln Lys Pro Gly Lys Ala Pro Lys Leu Leu Ile
        35                  40                  45


Tyr Asn Ala Arg Thr Leu Pro Glu Gly Val Pro Ser Arg Phe Ser Gly
    50                  55                  60


Ser Gly Ser Gly Thr Asp Phe Thr Leu Thr Ile Ser Ser Leu Gln Pro
65                  70                  75                  80


Glu Asp Phe Ala Thr Tyr Tyr Cys Gln His His Tyr Thr Thr Pro Trp
                85                  90                  95


Thr Phe Gly Gln Gly Thr Lys Val Glu Ile Lys

            100                 105

<210> 168
<211> 11
<212> PRT
<213> Homo sapiens

<400> 168

Arg Ala Ser Glu Asn Ile Tyr Ser Tyr Leu Ala
1               5                   10

<210> 169
<211> 7
<212> PRT
<213> Homo sapiens

<400> 169

Asn Ala Arg Thr Leu Pro Glu
1               5

<210> 170
<211> 9
<212> PRT
<213> Homo sapiens

<400> 170

Gln His His Tyr Thr Thr Pro Trp Thr
1               5

<210> 171
<211> 321
<212> DNA
<213> Homo sapiens

<400> 171
gacatccaga tgacacagag ccctagcagc ctgtctgcca gcgtgggaga cagagtgacc 60

atcacctgta gagccagcga gaacatctac agctacctgg cctggtatca gcagaagcct 120

ggcaaggctc ccaagctgct gatctacaac gccagaacac tgcctgaggg cgtgccctct 180

agattcagcg gatctggctc tggcaccgac ttcaccctga caatctctag cctgcagcct 240

gaggacttcg ccacctacta ctgccagcac cactacacca caccttggac attcggccag 300

ggcaccaagg tggaaatcaa g                                                321

<210>  172
<211>  107
<212>  PRT
<213>  Homo sapiens

<400>  172

Asp Ile Gln Met Thr Gln Ser Pro Ser Ser Leu Ser Ala Ser Val Gly
1               5                   10                  15

Asp Arg Val Thr Ile Thr Cys Arg Thr Ser Glu Asn Ile Tyr Ser Tyr
            20                  25                  30

Leu Ala Trp Tyr Gln Gln Lys Pro Gly Lys Ala Pro Lys Leu Leu Ile
        35                  40                  45

Tyr Phe Ala Lys Thr Leu Thr Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln His His Tyr Gly Thr Pro Trp
                85                  90                  95

Thr Phe Gly Gln Gly Thr Lys Val Glu Ile Lys
            100                 105


<210>  173
<211>  11
<212>  PRT
<213>  Homo sapiens

<400>  173

Arg Thr Ser Glu Asn Ile Tyr Ser Tyr Leu Ala
1               5                   10


<210>  174
<211>  7
<212>  PRT

<213>  Homo sapiens

<400>  174

Phe Ala Lys Thr Leu Thr Asp
1               5



<210>  175
<211>  9
<212>  PRT
<213>  Homo sapiens

<400>  175

Gln His His Tyr Gly Thr Pro Trp Thr
1               5



<210>  176
<211>  321
<212>  DNA
<213>  Homo sapiens

<400>  176
gacatccaga tgacacagag ccctagcagc ctgtctgcca gcgtgggaga cagagtgacc      60

atcacctgta gaaccagcga gaacatctac agctacctgg cctggtatca gcagaagcct     120

ggcaaggctc ccaagctgct gatctacttc gccaagacac tgaccgacgg cgtgccctct     180

agattcagcg gatctggctc tggcaccgac ttcaccctga caatctctag cctgcagcct     240

gaggacttcg ccacctacta ctgccagcac cactacggca caccttggac attcggccag     300

ggcaccaagg tggaaatcaa g                                               321

## Description

### Technical Field

The present disclosure relates to an antibody that recognizes TSLP protein, and a manufacturing method and application thereof.

### Background Art

Thymic stromal lymphopoietin (TSLP)is a cytokine of the interleukin-7 cytokine family. It is primarily produced from epithelial cells and keratinocytes located in the skin, intestinal tract or the lung, and is involved in maintaining the stability of the mucosal immune system. Upon stimulation or destruction of epithelial tissue by allergens or pathogens, dendritic cells present antigens to naive CD4+ T cells, releasing epithelial cell factors, TSLP, and IL-33. These important co-stimulatory cytokines lead to the development of allergen-specific TH2 cells, which then produce the cytokines interleukins IL-4, IL-5, and IL-13. This group of TH2-derived cytokines plays a critical role in the pathogenesis of allergic diseases.

TSLP is a multifunctional cytokine that can exert biological functions through a variety of TSLPR/IL-7Ra receptors on the surface of immune cells, including DC cells, CD4, and CD8+ T cells, B cells, mast cells, basophils, eosinophils, and NKT cells (Ziegler SF, 2013). TSLP stimulates immature dendritic cells (iDCs) activation, increases antigen presentation, and produces IL-8 (Interleukin 8 or otherwise known as Chemokine (C-X-C motif) ligand 8 (CXCL 8)), eotaxin 2 (or otherwise known as Chemokine (C-C motif) ligand 24 (CCL 24)), TARC (thymus and activation-regulated chemokine or otherwise known as Chemokine (C-C motif) ligand 17 (CCL 17)) and MDC (macrophage-derived chemokine or otherwise known as Chemokine (C-C motif) 22 (CCL 22)), attracts eosinophils, neutrophils, and Th2 cell aggregating; TSLP may promote the differentiation of naive T cells into TH2 cells, which depended on the expression of OX40L by DC cells induced by TSLP; TSLP can induce mast cell proliferation, prolong the life cycle of eosinophils, and specifically release inflammatory cytokines and chemokines; TSLP can also stimulate another important group of mucosal immune cells, group 2 innate lymphoid cells (ILC2) to secrete Th2 cytokines IL-4, IL5, and IL-13, and promote skin inflammation in vivo. Smooth muscle cells also secrete IL-8 and eotaxin after being stimulated by TSLP. TSLP has also been demonstrated to increase the production of cytokines of a variety of innate immune cells, including ILC2, mast cells, natural killer cells, and eosinophils, and to promote the development and function of basophil subsets. Therefore, TSLP, which may be one of the initiators of the inflammatory cascade, inhibition of TSLP could intervene from an early phase of inflammation, thus preventing the release of pro-inflammatory cytokines by immune cells, which is more effective than the inhibition of IL-4, IL-5, and IL-13 alone.

TSLP transduces signals via the JAK/STAT (JAK kinase-signal transducer and activator of transcription) pathway. TSLP binds to TSLPR on the cell membrane and then binds to IL-7Rα to form a stable TSLP-TSLPR-IL7Rα receptor complex, in which the intracellular segment of TSLPR receptor recruits and activates JAK2, and interacts with JAK1 recruited by IL7Rα to activate downstream signaling molecules. Studies have shown that in CD11c+ DC cells derived from human peripheral blood, TSLP can activate several STAT signaling molecules, such as STAT1, STAT3, STAT4, STAT5, and STAT6, in which STAT5 activation signal is the key to promote TH2 cell differentiation and secretion of TH2 factors. In addition, the activation of JAK/STAT5 signaling pathway stimulated by TSLP can render ILC2 cells unresponsive to inhibition effect of glucocorticoid, suggesting that inhibition of TSLP may be helpful to restore sensitivity to glucocorticoid in patients.

TSLP is closely related to the occurrence of type II inflammatory diseases. Studies have shown that TSLP is abundantly expressed in damaged skin of patients with atopic dermatitis, but is not detected in non-damaged skin. Large numbers of TSLP mRNA-positive cells were also detected in the lung epithelia and submucosa of patients with asthma and chronic obstructive pulmonary disease (COPD), who had higher concentrations of TSLP in alveolar lavage samples than healthy controls. The expression level of TSLP in patients with asthma is directly correlated with the expression of TH2 cytokines and chemokines, but negatively correlated with the state of lung function retained by patients. In biopsies from patients with allergic rhinitis, increased expression of TSLP in nasal epithelium was found and correlated with TH2 cytokine production and eosinophil infiltration in epithelial related tissues.

The genetic polymorphisms of TSLP and TSLPR have been implicated in the pathogenesis of eosinophilic esophagitis (EoE). In esophagus samples from EoE patients, high expression of TSLP and increased proportion of basophils (lin-, CD49b+, FcεRI+, c-kit-, 2D7+) were detected. Interestingly, the mouse EoE model is dependent on TSLP and basophils, but not on IgE. Use of TSLP neutralizing antibodies or clearance of basophils both are effective in reducing EoE symptoms, suggesting that inhibition of TSLP-basophils, rather than IgE, may be an effective approach to the treatment of EoE.

In addition, studies have shown that TSLP may contribute to TH1/TH17-related autoimmune diseases such as rheumatoid arthritis and multiple sclerosis. Cells expressing TSLP and TSLPR are increased in the joints of patients with rheumatoid arthritis. In a proteoglycan-induced rheumatoid arthritis mouse model, TSLPR-deficient mice show decreased levels of IL-17, IL-1β, and IL-6, increased levels of INFγ and IL-10, and reduced disease symptoms. These results suggest that TSLP and its receptor may be new therapeutic targets for treating rheumatoid arthritis.

### Summary of the Invention

The present inventors immunized mice with recombinant TSLP protein, gene gun, or a combination thereof to obtain multiple strains of high-affinity antibodies that recognize human and Cynomolgus macaques TSLP recombinant protein. The antibodies of the present disclosure having high affinity can effectively block the binding of TSLP to its receptor TSLPR, and can block TSLP-induced STAT5 signal transduction in reporter cells, and at the same time can block TSLP-induced cell proliferation, wherein the blocking efficiency is stronger than that of other antibodies of the same type, and thus the antibodies can be used for the diagnosis and treatment of allergic inflammatory diseases.

In one aspect, the present disclosure provides an antibody or antigen-binding portion thereof that binds to a TSLP protein.

In one aspect, the present disclosure provides a nucleic acid encoding any of the aforementioned antibody or antigen-binding portion thereof.

In one aspect, the present disclosure provides a vector comprising the nucleic acid molecule of the preceding aspect.

In one aspect, the present disclosure provides a cell comprising the vector of the preceding aspect. An antibody or antigen-binding portion thereof according to any one of the preceding aspects is provided, wherein the antibody or antigen-binding portion thereof is humanized.

In one aspect, the present disclosure provides a pharmaceutical composition or kit comprising an antibody or antigen-binding portion thereof or nucleic acids encoding the same according to any of the preceding aspects and a pharmaceutically acceptable carrier.

In one aspect, the present disclosure provides a method of treating a TSLP-related disorder comprising the steps of administering to the mammal a therapeutically effective amount of an antibody or antigen-binding fragment thereof, or a nucleic acid molecule, or a vector, or a cell and/or a pharmaceutical composition according to any of the preceding aspects.

Use of the antibody or antigen-binding fragment thereof or the nucleic acid molecule or the vector and/or the cell or the pharmaceutical composition of any of the preceding aspects in the manufacture of a medicament or kit for the treatment of a TSLP-related disorder in a mammal is provided.

The antibody may bind to human TSLP and/or Cynomolgus macaques TSLP with high affinity, block the binding of TSLP to TSLPR, and inhibit the transduction of TSLP stimulatory signals via the STAT5 pathway.

### Brief Description of the Drawings

FIG. 1 illustrates the recombinant expression of TSLP protein in prokaryotic cells;
FIG. 2 illustrates the recombinant expression of TSLP protein in HEK 293 cells;
FIG. 3 illustrates the binding of TSLP recombinant protein to receptor TSLPR by ELISA assay;
FIG. 4 illustrates FACS assay of Ba/F3-hTSLPR cells;
FIG. 5 illustrates proliferation assay of Ba/F3-hTSLPR-IL7Rα cells;
FIG. 6 illustrates reporter gene assay of Ba/F3-hTSLPR-IL7Rα-STAT5 luc cells
FIG. 7 illustrates the binding of chimeric antibodies by ELISA assay;
FIG. 8 illustrates the blockade of chimeric antibodies by ELISA assay;
FIG. 9 illustrates the blockade of chimeric antibodies at the cellular level;
FIG. 10 illustrates the inhibition of chimeric antibodies on cell proliferation;
FIG. 11 illustrates the inhibition of chimeric antibodies on reporter gene expression;
FIG. 12 illustrates the binding of humanized antibodies to TSLP in human and Cynomolgus macaques by ELISA assay;
FIG. 13 illustrates the blockade of humanized antibodies by ELISA assay;
FIG. 14 illustrates the blockade of humanized antibodies at the cellular level;
FIG. 15 illustrates the inhibition of humanized antibodies on cell proliferation; and
FIG. 16 illustrates the inhibition of humanized antibodies on reporter gene expression;

### Detailed Description of the Invention

### I. Definitions

In the present disclosure, unless otherwise specified, scientific and technical terms used herein have the meanings commonly understood by those skilled in the art. Also, as used herein, protein and nucleic acid chemistry, molecular biology, cell and tissue culture, microbiology, immunology, and laboratory procedures used herein are terms and routine procedures widely used in the corresponding fields. Meanwhile, in order to better understand the present invention, definitions and explanations of related terms are provided below.

In one aspect, provided herein are antibodies (e. g., monoclonal antibodies) that specifically bind to TSLP and antigen-binding fragments thereof. In particular aspects, provided herein are anti-TSLP monoclonal antibodies that specifically bind to TSLP, wherein the anti-TSLP antibodies include variants of the parent antibodies. In particular aspects, provided herein are antibodies that specifically bind to TSLP (e. g., human TSLP). In particular aspects, provided herein are anti-TSLP antibodies comprising modifications in one or more amino acid residues (e. g., 5-13 amino acid substitutions in the framework region of the heavy chain variable region) that retain affinity for an antigen as compared to the parent antibody without the modification.

The term "about" or "approximately" as used herein means within plus or minus 10% of a given value or range, unless otherwise specified. Where integers are required, the term refers to integers within plus or minus 10% of a given value or range, rounded up or down to the nearest integer. The phrase "substantially identical" with respect to an antibody chain polypeptide sequence may be construed as an antibody chain exhibiting at least 60%, 65%, 70%, 75%, 80%, 85%, 90%, 95%, 96%, 97%, 98%, 99% or more sequence identity to a reference polypeptide sequence. The term with respect to a nucleic acid sequence may be construed as a sequence of nucleotides exhibiting at least about 60%, 65%, 70%, 75%, 80%, 85%, 90%, 95%, 96%, 97%, 98%, 99% or more sequence identity to the reference nucleic acid sequence.

Sequence "identical" or "identity" has the recognized meaning in this field, and the percentage of sequence identity between two nucleic acid or polypeptide molecules or regions can be calculated using published techniques. Sequence identity can be measured along the entire length of a polynucleotide or polypeptide or along a region of the molecule (see, for example: Computational Molecular Biology, Lesk, A.M., ed., Oxford University Pres, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987; and Sequence Analysis Primer, Gribskov, M.and Devereux, J., eds., M Stockton Press, New York, 1991). While there are many methods of measuring identity between two polynucleotides or polypeptides, the term "identity" is well known to those skilled in the art (Carrillo, H. & Lipman, D. SIAM J Applied Math 48: 1073 (1988)).

"Substitution" variants are those in which at least one amino acid residue in the native sequence has been removed and inserted by a different amino acid at the same position. Said substitution may be single, with only one amino acid in the molecule being substituted; or may be multiple, with two or more amino acids being substituted in the same molecule. Multiple substitutions may be at consecutive positions. Likewise, an amino acid may be substituted with multiple residues, wherein such variants include both substitutions and insertions. "Insertion" variants are those in which one or more amino acids are inserted immediately adjacent to an amino acid at a particular position in a native sequence. By immediately adjacent amino acid is meant attached to the α-carboxy or α-amino functional group of the amino acid. "Deletion" variants are those in which one or more amino acids in the native amino acid sequence have been removed. Typically, one or two amino acids are deleted in a particular region of the molecules of deletion variants.

With respect to the variable domains of antibodies, the term "variable" refers to certain portions of related molecules that differ widely in sequence between antibodies and are used for specifically recognizing and binding to a specific target of a particular antibody. However, the variability is not uniformly distributed throughout the variable domain of the antibody. Variability is concentrated on three segments called complementarity determining regions (CDR; i.e., CDR1, CDR2, and CDR3) or hypervariable regions, all of which are located within the variable domains of the light and heavy chains. The more conserved portions within the variable domains are referred to as framework (FR) regions or framework sequences. Each variable domain of native heavy and light chains comprises four FR regions, predominantly in a β -sheet configuration, connected by three CDRs, which form loops connecting and in some cases forming part of the β -sheet structure. The CDRs of each chain are typically joined together by adjacent FR regions and aid in the formation of antibody target binding sites (epitopes or determinants) by means of CDR from other chains (see Kabat et al. Sequences of Proteins of Immunological Interest, nationalInstitute of Health, bethesda, MD (1987)). As used herein, the immunoglobulin amino acid residue numbering is in accordance with the Kabat numbering scheme (Kabat et al.) for numbering amino acid residues in the immunoglobulin, unless otherwise indicated. One CDR may have the ability to specifically bind to a cognate epitope.

As used herein, an "antibody fragment" or "antigen-binding fragment" of an antibody refers to any portion of a full-length antibody that is less than full-length, but that comprises at least a portion of the variable region (e. g., one or more CDRs and/or one or more antibody binding sites) of the antibody that binds antigen, thus retaining binding specificity as well as at least a portion of the specific binding capacity of the full-length antibody. Thus, an antigen-binding fragment refers to an antibody fragment that comprises an antigen-binding portion that binds the same antigen as the antibody from which the antibody fragment was derived. Antibody fragments include antibody derivatives produced by enzymatic treatment of full-length antibodies, as well as synthetically produced derivatives, e. g., recombinantly produced derivatives. Antibodies include antibody fragments. Examples of antibody fragments include, but are not limited to, Fab, Fab', F(ab')₂, single chain Fv (scFv), Fv, dsFv, diabodies, Fd, and Fd' fragments and other fragments, including modified fragments (see, e. g., Methods in Molecular Biology, Vol 207: Recombinant Antibodies for Cancer Therapy Methods and Protocols (2003); Chapter 1; p 3-25, Kipriyanov). The fragments may comprise multiple chains linked together, for example, by disulfide bonds and/or by peptide linkers. Antibody fragments generally comprise at least or about 50 amino acids, and typically at least or about 200 amino acids. Antigen-binding fragments include any antibody fragment that, when inserted into an antibody framework (e. g., by displacement of the corresponding region), results in an antibody that immunospecifically binds (i. e., exhibiting a Ka of at least or at least about 10⁷-10⁸M-1) an antigen. A "functional fragment" or "analog of an anti-TSLP antibody" is a fragment or analog that prevents or substantially reduces the ability of the receptor to bind a ligand or initiate signal transduction. As used herein, functional fragments generally have the same meaning as "antibody fragments" and, in the case of antibodies, can refer to fragments that prevent or substantially reduce the ability of the receptor to bind a ligand or initiate signal transduction, e. g., Fv, Fab, F(ab')₂, and the like. An "Fv" fragment consists of a dimer (V_{H}-V_{L} dimer) of a variable domain of a heavy chain and a variable domain of a light chain formed by non-covalent association. In this configuration, the three CDRs of each variable domain interact to define a target binding site on the surface of the V_{H}-V_{L} dimer, as is the case with intact antibodies. The six CDRs together confer target binding specificity to the intact antibody. However, even a single variable domain (or half of an Fv comprising only three target-specific CDRs) may still have the ability to recognize and bind to targets.

As used herein, the term "bispecific antibody (BsAb)" refers to an antibody and/or antigen-binding molecule capable of specifically binding to two different antigenic determinants. Generally, a bispecific antibody and/or antigen-binding molecule comprises two antigen-binding sites, each of which is specific for different antigenic determinants. In some embodiments, the bispecific antibody and/or antigen binding molecule is capable of binding two antigenic determinants simultaneously, particularly two antigenic determinants expressed on two different cells.

As used herein, "monoclonal antibody" refers to a population of identical antibodies, meaning that each individual antibody molecule in the population of monoclonal antibodies is identical to another antibody molecule. This property is in contrast to the property of a population of polyclonal antibodies comprising antibodies having a plurality of different sequences. Monoclonal antibodies can be prepared by a number of well-known methods (Smith et al. (2004) J.Clin.Pathol.57, 912-917; and Nelson et al., J Clin Pathol( 2000), 53, 111-117). For example, monoclonal antibodies can be prepared by immortalizing B cells, for example, by fusing B cells with myeloma cells to generate hybridoma cell lines or by infecting B cells with a virus such as EBV. Recombinant techniques can also be used to prepare antibodies from clonal populations of host cells in vitro by transforming host cells with plasmids carrying artificial sequences encoding the nucleotides of the antibodies.

As used herein, the term "hybridoma" or "hybridoma cell" refers to a cell or cell line (typically a myeloma or lymphoma cell) resulting from the fusion of antibody-producing lymphocytes and non-antibody-producing cancer cells. As known to those of ordinary skill in the art, hybridomas can be propagated and continuously supplied to produce a particular monoclonal antibody. Methods for producing hybridomas are known in the art (see, e. g., Harlow & Lane, 1988). When referring to the term "hybridoma" or "hybridoma cell", it also includes subclones and progeny cells of hybridomas.

As used herein, a full-length antibody is an antibody having two full-length heavy chains (e. g. VH-CH1-CH2-CH3 or VH-CH1-CH2-CH3-CH4) and two full-length light chains (VL-CL) and a hinge region, e. g., an antibody naturally produced by an antibody secreting B cell as well as a synthetically produced antibody having the same domain.

The term "chimeric antibody" refers to an antibody in which the variable region sequences are derived from one species and the constant region sequences are derived from another species, such as an antibody in which the variable region sequences are derived from a mouse antibody and the constant region sequences are derived from a human antibody.

A "humanized" antibody refers to a form of non-human (e. g., mouse) antibody that is a chimeric immunoglobulin, immunoglobulin chain or fragment thereof (e. g., Fv, Fab, Fab', F(ab')₂ or other antigen-binding subsequence of an antibody), containing minimal sequence derived from a non-human immunoglobulin. Preferably, the humanized antibody is a human immunoglobulin (recipient antibody) in which residues from a complementarity determining region (CDR) of the recipient antibody are replaced by CDR residues from a non-human species (donor antibody) such as mouse, rat, or rabbit having the desired specificity, affinity, and capacity.

Furthermore, in humanization it is also possible to mutate amino acid residues within the CDR1, CDR2 and/or CDR3 regions of VH and/or VL, thereby improving one or more binding properties (e. g., affinity) of the antibody. Mutations can be introduced, e. g. by PCR-mediated mutagenesis, and their effect on antibody binding or other functional properties can be assessed using the in vitro or in vivo assays described herein. Typically, conservative mutations are introduced. Such mutations may be amino acid substitutions, additions, or deletions. In addition, mutations within CDR typically do not exceed one or two. Thus, humanized antibodies of the present disclosure also encompass antibodies comprising one or two amino acid mutations within CDR.

As used herein, the term "CDR" refers to a complementarity-determining region known to have three CDRs per heavy and light chain of an antibody molecule. CDR is also known as a hypervariable region and is present in the variable region of each of the heavy and light chains of an antibody with a very high site of variability in the primary structure of CDR. In the present specification, the CDR of the heavy chain is represented by CDR1, CDR2, CDR3 from the amino terminus of the amino terminal sequence of the heavy chain, and the CDR of the light chain is represented by CDR1, CDR2, CDR3 from the amino terminus of the amino terminal sequence of the light chain. These sites are adjacent to each other in the tertiary structure and determine the specificity of the antigen to which the antibody binds.

As used herein, the term "epitope" refers to any antigenic determinant on an antigen to which the paratope of an antibody binds. Epitopic determinants usually comprise chemically active surface types of molecules, such as amino acids or sugar side chains, and usually have specific three-dimensional structural characteristics as well as specific charge characteristics.

As used herein, "specific binding" or "immunospecifically binding" with respect to an antibody or antigen-binding fragment thereof is used interchangeably herein and refers to the ability of an antibody or antigen-binding fragment to form one or more non-covalent bonds with the same antigen through non-covalent interactions between the antibody and the antibody binding site of the antigen. The antigen may be an isolated antigen or present in a tumor cell. Typically, an antibody that immunospecifically binds (or specifically binds) to an antigen is one that binds to the antigen with an affinity constant Ka of about or 1×10⁷M⁻¹ or 1×10⁸M⁻¹ or more (or a dissociation constant (Kd) of 1×10⁻⁷M or 1×10⁻⁸M or less). Affinity constants can be determined by standard kinetic methods for antibody reactions, e. g., immunoassays, surface plasmon resonance (SPR) (Rich and Myszka (2000) Curr. Opin.Biotechnol 11:54; Englebienne (1998) Analyst. 123:1599), isothermal titration calorimetry (ITC), or other kinetic interaction assays known in the art (see, e. g., Paul, ed., Fundamental Immunology, 2nd ed., Raven Press, New York, pages 332-336 (1989); see also U.S. Patent No. 7, 229, 619) which describes an exemplary SPR and ITC method for calculating the binding affinity of an antibody. Instruments and methods for real-time detecting and monitoring the rate of binding are known and commercially available (see, BiaCore 2000, Biacore AB, Upsala, Sweden and GE Healthcare Life Sciences; Malmqvist (2000) Biochem.Soc.Trans. 27:335).

As used herein, the terms "polynucleotide" and "nucleic acid molecule" refer to an oligomer or polymer comprising at least two linked nucleotides or nucleotide derivatives, including deoxyribonucleic acid (DNA) and ribonucleic acid (RNA), typically linked together by phosphodiester bonds. As used herein, the term "nucleic acid molecule" is intended to include DNA molecules and RNA molecules. The nucleic acid molecule may be single-stranded or double-stranded, and may be cDNA.

As used herein, an isolated nucleic acid molecule is a nucleic acid molecule isolated from other nucleic acid molecules present in the natural source of the nucleic acid molecule. An "isolated" nucleic acid molecule, such as a cDNA molecule, can be substantially free of other cellular material or culture medium when prepared by recombinant techniques, or substantially free of chemical precursors or other chemical components when chemically synthesized. Exemplary isolated nucleic acid molecules provided herein include isolated nucleic acid molecules encoding a provided antibody or antigen-binding fragment.

As used herein, "operably linked" with respect to a nucleic acid sequence, region, element or domain means that the nucleic acid regions are functionally related to one another. For example, a promoter may be operably linked to a nucleic acid encoding a polypeptide such that the promoter regulates or mediates transcription of the nucleic acid.

Also provided are "conservative sequence modifications" of the sequences set forth in the sequence listings described herein, i. e., nucleotide and amino acid sequence modifications that do not eliminate binding of an antibody encoded by a nucleotide sequence or containing an amino acid sequence to an antigen. These conservative sequence modifications include substitutions of conservative nucleotide and amino acid and additions and deletions of nucleotide and amino acid. For example, modifications can be introduced into the sequence listings described herein by standard techniques known in the art, such as site-directed mutagenesis and PCR-mediated mutagenesis. Conservative sequence modifications include conservative amino acid substitutions in which an amino acid residue is replaced with an amino acid residue having a similar side chain. Families of amino acid residues having similar side chains have been defined in the art. These families include amino acids containing basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), non-polar side chains (e.g., alanine, valine, leucine, isoleucine, proline, phenylalanine, methionine), β-branched side chains (e.g., threonine, valine, isoleucine), and aromatic side chains (e.g., tyrosine, phenylalanine, tryptophan, histidine). Thus, a predicted nonessential amino acid residue in an anti-TSLP antibody is preferably replaced with another amino acid residue from the same side chain family. Methods for identifying conservative substitutions of nucleotides and amino acids that do not eliminate antigen binding are well known in the art (see, e. g., Brummell et al., Biochem. 32: 1180-1187 (1993); Kobayashi et al., Protein Eng. 12(10): 879-884 (1999); Burks et al., Proc. Natl. Acad. Sci. USA 94: 412-417 (1997)).

Alternatively, in another embodiment, mutations can be randomly introduced along all or a portion of the anti-TSLP antibody coding sequence, e. g., by saturation mutagenesis, and the resulting modified anti-TSLP antibodies can be screened for improved binding activity.

As used herein, "expression" refers to a process of producing a polypeptide by transcription and translation of a polynucleotide. The level of expression of a polypeptide can be assessed using any method known in the art, including, for example, methods of determining the amount of polypeptide produced from a host cell. Such methods may include, but are not limited to, quantitation of polypeptides in cell lysates by ELISA, gel electrophoresis followed by Coomassie blue staining, Lowry protein assay, and Bradford protein assay.

As used herein, a "host cell" is a cell for receiving, maintaining, replicating, and expanding a vector. The host cell may also be used to express a polypeptide encoded by the vector. The nucleic acid contained in the vector replicates during the host cell division, thereby amplifying the nucleic acid. The host cell may be a eukaryotic cell or a prokaryotic cell. Suitable host cells include, but are not limited to, CHO cells, various COS cells, HeLa cells, HEK cells such as HEK 293 cells. As used herein, a "vector" is a replicable nucleic acid from which one or more heterologous proteins can be expressed when the vector is transformed into an appropriate host cell. References to vectors include those into which a nucleic acid encoding a polypeptide or fragment thereof may be introduced, typically by restriction digestion and ligation. References to vectors also include those comprising a nucleic acid encoding a polypeptide. Vectors are used to introduce a nucleic acid encoding a polypeptide into a host cell, to amplify the nucleic acid, or to express/display the polypeptide encoded by the nucleic acid. The vector generally remains episomal, but can be designed to allow integration of a gene or portion thereof into the chromosome of the genome. Also contemplated are vectors for artificial chromosomes, such as yeast artificial vectors and mammalian artificial chromosomes. The selection and use of such vehicles is well known to those skilled in the art.

As used herein, a vector also includes a "virus vector" or a "viral vector". A viral vector is an engineered virus that is operably linked to a foreign gene to transfer (as a vehicle or shuttle) the foreign gene into a cell.

As used herein, an "expression vector" includes a vector capable of expressing a DNA operably linked to regulatory sequences, such as a promoter region, capable of affecting the expression of such DNA fragments. Such additional fragments may include promoter and terminator sequences, and optionally may include one or more origins of replication, one or more selectable markers, enhancers, polyadenylation signals, etc. Expression vectors are typically derived from plasmid or viral DNA, or may contain elements of both. Thus, an expression vector refers to a recombinant DNA or RNA construct, such as a plasmid, phage, recombinant virus or other vectors, which results in the expression of a cloned DNA when introduced into an appropriate host cell. Appropriate expression vectors are well known to those skilled in the art and include expression vectors that are replicable in eukaryotic and/or prokaryotic cells as well as expression vectors that remain episomal or that are integrated into the host cell genome.

As used herein, "treating" a subject with a disease or condition means that the subject's symptoms are partially or completely alleviated or remain unchanged after the treatment. Thus, treatment includes prevention, treatment and/or cure. The prevention refers to preventing the underlying disease and/or preventing the worsening of symptoms or the progression of the disease. The treatment also includes any antibody or antigen-binding fragment thereof provided and any pharmaceutical use of the compositions provided herein.

As used herein, "therapeutic effect" refers to an effect resulting from treatment of a subject that alters, typically improves or ameliorates a symptom of a disease or condition, or cures a disease or condition.

As used herein, a "therapeutically effective amount" or "therapeutically effective dose" refers to an amount of a substance, compound, material, or composition comprising a compound that is at least sufficient to produce a therapeutic effect after being administered to a subject. Thus, it is an amount necessary to prevent, cure, ameliorate, block, or partially block the symptoms of a disease or disorder.

As used herein, a "prophylactically effective amount" or "prophylactically effective dose" refers to an amount of a substance, compound, material, or composition comprising a compound that, when administered to a subject, has the desired prophylactic effect, e. g., preventing or delaying the occurrence or recurrence of a disease or condition, reducing the likelihood of the occurrence or recurrence of a disease or condition. A fully prophylactically effective dose need not occur by administration of one dose, and may occur only after administration of a series of doses. Thus, a prophylactically effective amount may be administered one or more times As used herein, the term "patient" refers to a mammal, such as a human.

### DETAILED DESCRIPTION

In one aspect, the present disclosure provides an antibody or antigen-binding portion thereof that binds to TSLP, comprising a heavy chain CDR selected from the amino acid sequences of SEQ ID NOs:8-10, 18-20, 28-30, 38-40, 48-50, 58-60, 68-70, 78-80, 83-85, 88-90, 93-95, 98-100, 103-105, 108-110, 113-115, 118-120, 123-125, 128-130, 133-135, 138-140, 143-145, 148-150, 153-155, 158-160, 163-165, or any variant thereof, and/or a light chain CDR selected from the amino acid sequences of SEQ ID NOs:13-15, 23-25, 33-35, 43-45, 53-55, 63-65, 73-75, 168-170, 173-175, or any variant thereof.

In one aspect, the antibody or antigen-binding portion thereof according to the aforementioned aspect comprises a heavy chain CDR1 selected from the amino acid sequences of SEQ ID NOs: 8, 18, 28, 38, 48, 58, 68, 78, 83, 88, 93, 98, 103, 108, 113, 118, 123, 128, 133, 138, 143, 148, 153, 158, 163, or any variant thereof, a heavy chain CDR2 selected from the amino acid sequences of SEQ ID NOs: 9, 19, 29, 39, 49, 59, 69, 79, 84, 89, 94, 99, 104, 109, 114, 119, 124, 129, 134, 139, 144, 149, 154, 159, 164, or any variant thereof, and a heavy chain CDR3 selected from the amino acid sequences of SEQ ID NOs: 10, 20, 30, 40, 50, 60, 70, 80, 85, 90, 95, 100, 105, 110, 115, 120, 125, 130, 135, 140, 145, 150, 155, 160, 165, or any variant thereof; and/or a light chain CDR1 selected from the amino acid sequences of SEQ ID NOs: 13, 23, 33, 43, 53, 63, 73, 168, 173, or any variant thereof, a light chain CDR2 selected from the amino acid sequence SEQ ID NOs: 14, 24, 34, 44, 54, 64, 74, 169, 173, or any variant thereof, and a light chain CDR3 selected from the amino acid sequence SEQ ID NOs: 15, 25, 35, 45, 55, 65, 75, 170, 175, or any variant thereof.

In one aspect, the present disclosure relates to the antibody or antigen-binding portion thereof according to the aforementioned aspect, comprising a CDR combination of heavy and light chains selected from the group consisting of:
(1) the heavy chain CDR1, CDR2, and CDR3 sequences including SEQ ID NOs: 8-10, respectively, and/or the light chain CDR1, CDR2, and CDR3 sequences including SEQ ID NOs: 13-15, respectively;
(2) the heavy chain CDR1, CDR2, and CDR3 sequences including SEQ ID NOs: 18-20, respectively, and/or the light chain CDR1, CDR2, and CDR3 sequences including SEQ ID NOs: 23-25, respectively;
(3) the heavy chain CDR1, CDR2, and CDR3 sequences including SEQ ID NOs: 28-30, respectively, and/or the light chain CDR1, CDR2, and CDR3 sequences including SEQ ID NOs: 33-35, respectively;
(4) the heavy chain CDR1, CDR2, and CDR3 sequences including SEQ ID NOs: 38-40, respectively, and/or the light chain CDR1, CDR2, and CDR3 sequences including SEQ ID NOs: 43-45, respectively;
(5) the heavy chain CDR1, CDR2, and CDR3 sequences including SEQ ID NOs: 48-50, respectively, and/or the light chain CDR1, CDR2, and CDR3 sequences including SEQ ID NOs: 53-55, respectively;
(6) the heavy chain CDR1, CDR2, and CDR3 sequences including SEQ ID NOs: 58-60, respectively, and/or the light chain CDR1, CDR2, and CDR3 sequences including SEQ ID NOs: 63-65, respectively;
(7) the heavy chain CDR1, CDR2, and CDR3 sequences including SEQ ID NOs: 68-70, respectively, and/or the light chain CDR1, CDR2, and CDR3 sequences including SEQ ID NOs: 73-75, respectively;
(8) the heavy chain CDR1, CDR2, and CDR3 sequences including SEQ ID NOs: 78-80, respectively, and/or the light chain CDR1, CDR2, and CDR3 sequences including SEQ ID NOs: 168-170, respectively;
(9) the heavy chain CDR1, CDR2, and CDR3 sequences including SEQ ID NOs: 83-85, respectively, and/or the light chain CDR1, CDR2, and CDR3 sequences including SEQ ID NOs: 168-170, respectively;
(10) the heavy chain CDR1, CDR2, and CDR3 sequences including SEQ ID NOs: 88-90, respectively, and/or the light chain CDR1, CDR2, and CDR3 sequences including SEQ ID NOs: 168-170, respectively;
(11) the heavy chain CDR1, CDR2, and CDR3 sequences including SEQ ID NOs: 93-95, respectively, and/or the light chain CDR1, CDR2, and CDR3 sequences including SEQ ID NOs: 168-170, respectively;
(12) the heavy chain CDR1, CDR2, and CDR3 sequences including SEQ ID NOs: 98-100, respectively, and/or the light chain CDR1, CDR2, and CDR3 sequences including SEQ ID NOs: 168-170, respectively;
(13) the heavy chain CDR1, CDR2, and CDR3 sequences including SEQ ID NOs: 103-105, respectively, and/or the light chain CDR1, CDR2, and CDR3 sequences including SEQ ID NOs: 168-170, respectively;
(14) the heavy chain CDR1, CDR2, and CDR3 sequences including SEQ ID NOs: 108-110, respectively, and/or the light chain CDR1, CDR2, and CDR3 sequences including SEQ ID NOs: 168-170, respectively;
(15) the heavy chain CDR1, CDR2, and CDR3 sequences including SEQ ID NOs: 113-115, respectively, and/or the light chain CDR1, CDR2, and CDR3 sequences including SEQ ID NOs: 168-170, respectively;
(16) the heavy chain CDR1, CDR2, and CDR3 sequences including SEQ ID NOs: 118-120, respectively, and/or the light chain CDR1, CDR2, and CDR3 sequences including SEQ ID NOs: 168-170, respectively;
(17) the heavy chain CDR1, CDR2, and CDR3 sequences including SEQ ID NOs: 123-125, respectively, and/or the light chain CDR1, CDR2, and CDR3 sequences including SEQ ID NOs: 168-170, respectively;
(18) the heavy chain CDR1, CDR2, and CDR3 sequences including SEQ ID NOs: 128-130, respectively, and/or the light chain CDR1, CDR2, and CDR3 sequences including SEQ ID NOs: 168-170, respectively;
(19) the heavy chain CDR1, CDR2, and CDR3 sequences including SEQ ID NOs: 133-135, respectively, and/or the light chain CDR1, CDR2, and CDR3 sequences including SEQ ID NOs: 168-170, respectively;
(20) the heavy chain CDR1, CDR2, and CDR3 sequences including SEQ ID NOs: 138-140, respectively, and/or the light chain CDR1, CDR2, and CDR3 sequences including SEQ ID NOs: 168-170, respectively;
(21) the heavy chain CDR1, CDR2, and CDR3 sequences including SEQ ID NOs: 143-145, respectively, and/or the light chain CDR1, CDR2, and CDR3 sequences including SEQ ID NOs: 168-170, respectively;
(22) the heavy chain CDR1, CDR2, and CDR3 sequences including SEQ ID NOs: 148-150, respectively, and/or the light chain CDR1, CDR2, and CDR3 sequences including SEQ ID NOs: 168-170, respectively;
(23) the heavy chain CDR1, CDR2, and CDR3 sequences including SEQ ID NOs: 153-155, respectively, and/or the light chain CDR1, CDR2, and CDR3 sequences including SEQ ID NOs: 168-170, respectively;
(24) the heavy chain CDR1, CDR2, and CDR3 sequences including SEQ ID NOs: 158-160, respectively, and/or the light chain CDR1, CDR2, and CDR3 sequences including SEQ ID NOs: 168-170, respectively;
(25) the heavy chain CDR1, CDR2, and CDR3 sequences including SEQ ID NOs: 163-165, respectively, and/or the light chain CDR1, CDR2, and CDR3 sequences including SEQ ID NOs: 168-170, respectively;
(26) the heavy chain CDR1, CDR2, and CDR3 sequences including SEQ ID NOs: 153-155, respectively, and/or the light chain CDR1, CDR2, and CDR3 sequences including SEQ ID NOs: 173-175, respectively;
(27) the heavy chain CDR1, CDR2, and CDR3 sequences including SEQ ID NOs: 158-160, respectively, and/or the light chain CDR1, CDR2, and CDR3 sequences including SEQ ID NOs: 173-175, respectively;
(28) the heavy chain CDR1, CDR2, and CDR3 sequences including SEQ ID NOs: 163-165, respectively, and/or the light chain CDR1, CDR2, and CDR3 sequences including SEQ ID NOs: 173-175, respectively;
(29) the heavy chain CDR1, CDR2, and CDR3 sequences including SEQ ID NOs: 78-80, respectively, and/or the light chain CDR1, CDR2, and CDR3 sequences including SEQ ID NOs: 173-175, respectively;
(30) the heavy chain CDR1, CDR2, and CDR3 sequences including SEQ ID NOs: 83-85, respectively, and/or the light chain CDR1, CDR2, and CDR3 sequences including SEQ ID NOs: 173-175, respectively;
(31) the heavy chain CDR1, CDR2, and CDR3 sequences including SEQ ID NOs: 98-100, respectively, and/or the light chain CDR1, CDR2, and CDR3 sequences including SEQ ID NOs: 173-175, respectively;
(32) the heavy chain CDR1, CDR2, and CDR3 sequences including SEQ ID NOs: 103-105, respectively, and/or the light chain CDR1, CDR2, and CDR3 sequences including SEQ ID NOs: 173-175, respectively;
(33) the heavy chain CDR1, CDR2, and CDR3 sequences including SEQ ID NOs: 123-125, respectively, and/or the light chain CDR1, CDR2, and CDR3 sequences including SEQ ID NOs: 173-175, respectively; and
(34) the heavy chain CDR1, CDR2, and CDR3 sequences including SEQ ID NOs: 128-130, respectively, and/or the light chain CDR1, CDR2, and CDR3 sequences including SEQ ID NOs: 173-175, respectively.

In one aspect, the present disclosure relates to an antibody or antigen-binding portion thereof according to the aforementioned aspect, comprising a heavy chain variable region selected from the amino acid sequence of SEQ ID NOs: 7, 17, 27, 37, 47, 57, 67, 77, 82, 87, 92, 97, 102, 107, 112, 117, 122, 127, 132, 137, 142, 147, 152, 157, 162, or any variant thereof, and/or a light chain variable region selected from the amino acid sequence of SEQ ID NOs: 12, 22, 32, 42, 52, 62, 72, 167, 172, or any variant thereof.

In one aspect, the present disclosure relates to an antibody or antigen-binding portion thereof that binds human TSLP, comprising a heavy chain variable region of the amino acid sequence of SEQ ID NO: 7 or any variant thereof, and a light chain variable region of the amino acid sequence of SEQ ID NO: 12 or any variant thereof.

In one aspect, the present disclosure relates to an antibody or antigen-binding portion thereof that binds human TSLP, comprising a heavy chain variable region of the amino acid sequence of SEQ ID NO: 17 or any variant thereof, and a light chain variable region of the amino acid sequence of SEQ ID NO: 22 or any variant thereof.

In one aspect, the present disclosure relates to an antibody or antigen-binding portion thereof that binds human TSLP, comprising a heavy chain variable region of the amino acid sequence of SEQ ID NO: 27 or any variant thereof, and a light chain variable region of the amino acid sequence of SEQ ID NO: 32 or any variant thereof.

In one aspect, the present disclosure relates to an antibody or antigen-binding portion thereof that binds human TSLP, comprising a heavy chain variable region of the amino acid sequence of SEQ ID NO: 37 or any variant thereof, and a light chain variable region of the amino acid sequence of SEQ ID NO: 42 or any variant thereof.

In one aspect, the present disclosure relates to an antibody or antigen-binding portion thereof that binds human TSLP, comprising a heavy chain variable region of the amino acid sequence of SEQ ID NO: 47 or any variant thereof, and a light chain variable region of the amino acid sequence of SEQ ID NO: 52 or any variant thereof.

In one aspect, the present disclosure relates to an antibody or antigen-binding portion thereof that binds human TSLP, comprising a heavy chain variable region of the amino acid sequence of SEQ ID NO: 57 or any variant thereof, and a light chain variable region of the amino acid sequence of SEQ ID NO: 62 or any variant thereof.

In one aspect, the present disclosure relates to an antibody or antigen-binding portion thereof that binds human TSLP, comprising a heavy chain variable region of the amino acid sequence of SEQ ID NO: 67 or any variant thereof, and a light chain variable region of the amino acid sequence of SEQ ID NO: 72 or any variant thereof.

In one aspect, the present disclosure relates to an antibody or antigen-binding portion thereof that binds human TSLP, comprising a heavy chain variable region of the amino acid sequence of SEQ ID NO: 77 or any variant thereof, and a light chain variable region of the amino acid sequence of SEQ ID NO: 167 or any variant thereof.

In one aspect, the present disclosure relates to an antibody or antigen-binding portion thereof that binds human TSLP, comprising a heavy chain variable region of the amino acid sequence of SEQ ID NO: 82 or any variant thereof, and a light chain variable region of the amino acid sequence of SEQ ID NO: 167 or any variant thereof.

In one aspect, the present disclosure relates to an antibody or antigen-binding portion thereof that binds human TSLP, comprising a heavy chain variable region of the amino acid sequence of SEQ ID NO: 87 or any variant thereof, and a light chain variable region of the amino acid sequence of SEQ ID NO: 167 or any variant thereof.

In one aspect, the present disclosure relates to an antibody or antigen-binding portion thereof that binds human TSLP, comprising a heavy chain variable region of the amino acid sequence of SEQ ID NO: 92 or any variant thereof, and a light chain variable region of the amino acid sequence of SEQ ID NO: 167 or any variant thereof.

In one aspect, the present disclosure relates to an antibody or antigen-binding portion thereof that binds human TSLP, comprising a heavy chain variable region of the amino acid sequence of SEQ ID NO: 97 or any variant thereof, and a light chain variable region of the amino acid sequence of SEQ ID NO: 167 or any variant thereof.

In one aspect, the present disclosure relates to an antibody or antigen-binding portion thereof that binds human TSLP, comprising a heavy chain variable region of the amino acid sequence of SEQ ID NO: 102 or any variant thereof, and a light chain variable region of the amino acid sequence of SEQ ID NO: 167 or any variant thereof.

In one aspect, the present disclosure relates to an antibody or antigen-binding portion thereof that binds human TSLP, comprising a heavy chain variable region of the amino acid sequence of SEQ ID NO: 107 or any variant thereof, and a light chain variable region of the amino acid sequence of SEQ ID NO: 167 or any variant thereof.

In one aspect, the present disclosure relates to an antibody or antigen-binding portion thereof that binds human TSLP, comprising a heavy chain variable region of the amino acid sequence of SEQ ID NO: 112 or any variant thereof, and a light chain variable region of the amino acid sequence of SEQ ID NO: 167 or any variant thereof.

In one aspect, the present disclosure relates to an antibody or antigen-binding portion thereof that binds human TSLP, comprising a heavy chain variable region of the amino acid sequence of SEQ ID NO: 117 or any variant thereof, and a light chain variable region of the amino acid sequence of SEQ ID NO: 167 or any variant thereof.

In one aspect, the present disclosure relates to an antibody or antigen-binding portion thereof that binds human TSLP, comprising a heavy chain variable region of the amino acid sequence of SEQ ID NO: 122 or any variant thereof, and a light chain variable region of the amino acid sequence of SEQ ID NO: 167 or any variant thereof.

In one aspect, the present disclosure relates to an antibody or antigen-binding portion thereof that binds human TSLP, comprising a heavy chain variable region of the amino acid sequence of SEQ ID NO: 127 or any variant thereof, and a light chain variable region of the amino acid sequence of SEQ ID NO: 167 or any variant thereof.

In one aspect, the present disclosure relates to an antibody or antigen-binding portion thereof that binds human TSLP, comprising a heavy chain variable region of the amino acid sequence of SEQ ID NO: 132 or any variant thereof, and a light chain variable region of the amino acid sequence of SEQ ID NO: 167 or any variant thereof.

In one aspect, the present disclosure relates to an antibody or antigen-binding portion thereof that binds human TSLP, comprising a heavy chain variable region of the amino acid sequence of SEQ ID NO: 137 or any variant thereof, and a light chain variable region of the amino acid sequence of SEQ ID NO: 167 or any variant thereof.

In one aspect, the present disclosure relates to an antibody or antigen-binding portion thereof that binds human TSLP, comprising a heavy chain variable region of the amino acid sequence of SEQ ID NO: 142 or any variant thereof, and a light chain variable region of the amino acid sequence of SEQ ID NO: 167 or any variant thereof.

In one aspect, the present disclosure relates to an antibody or antigen-binding portion thereof that binds human TSLP, comprising a heavy chain variable region of the amino acid sequence of SEQ ID NO: 147 or any variant thereof, and a light chain variable region of the amino acid sequence of SEQ ID NO: 167 or any variant thereof.

In one aspect, the present disclosure relates to an antibody or antigen-binding portion thereof that binds human TSLP, comprising a heavy chain variable region of the amino acid sequence of SEQ ID NO: 152 or any variant thereof, and a light chain variable region of the amino acid sequence of SEQ ID NO: 167 or any variant thereof.

In one aspect, the present disclosure relates to an antibody or antigen-binding portion thereof that binds human TSLP, comprising a heavy chain variable region of the amino acid sequence of SEQ ID NO: 157 or any variant thereof, and a light chain variable region of the amino acid sequence of SEQ ID NO: 167 or any variant thereof.

In one aspect, the present disclosure relates to an antibody or antigen-binding portion thereof that binds human TSLP, comprising a heavy chain variable region of the amino acid sequence of SEQ ID NO: 162 or any variant thereof, and a light chain variable region of the amino acid sequence of SEQ ID NO: 167 or any variant thereof.

In one aspect, the present disclosure relates to an antibody or antigen-binding portion thereof that binds human TSLP, comprising a heavy chain variable region of the amino acid sequence of SEQ ID NO: 152 or any variant thereof, and a light chain variable region of the amino acid sequence of SEQ ID NO: 172 or any variant thereof.

In one aspect, the present disclosure relates to an antibody or antigen-binding portion thereof that binds human TSLP, comprising a heavy chain variable region of the amino acid sequence of SEQ ID NO: 157 or any variant thereof, and a light chain variable region of the amino acid sequence of SEQ ID NO: 172 or any variant thereof.

In one aspect, the present disclosure relates to an antibody or antigen-binding portion thereof that binds human TSLP, comprising a heavy chain variable region of the amino acid sequence of SEQ ID NO: 162 or any variant thereof, and a light chain variable region of the amino acid sequence of SEQ ID NO: 172 or any variant thereof.

In one aspect, the present disclosure relates to an antibody or antigen-binding portion thereof that binds human TSLP, comprising a heavy chain variable region of the amino acid sequence of SEQ ID NO: 77 or any variant thereof, and a light chain variable region of the amino acid sequence of SEQ ID NO: 172 or any variant thereof.

In one aspect, the present disclosure relates to an antibody or antigen-binding portion thereof that binds human TSLP, comprising a heavy chain variable region of the amino acid sequence of SEQ ID NO: 82 or any variant thereof, and a light chain variable region of the amino acid sequence of SEQ ID NO: 172 or any variant thereof.

In one aspect, the present disclosure relates to an antibody or antigen-binding portion thereof that binds human TSLP, comprising a heavy chain variable region of the amino acid sequence of SEQ ID NO: 97 or any variant thereof, and a light chain variable region of the amino acid sequence of SEQ ID NO: 172 or any variant thereof.

In one aspect, the present disclosure relates to an antibody or antigen-binding portion thereof that binds human TSLP, comprising a heavy chain variable region of the amino acid sequence of SEQ ID NO: 102 or any variant thereof, and a light chain variable region of the amino acid sequence of SEQ ID NO: 172 or any variant thereof.

In one aspect, the present disclosure relates to an antibody or antigen-binding portion thereof that binds human TSLP, comprising a heavy chain variable region of the amino acid sequence of SEQ ID NO: 122 or any variant thereof, and a light chain variable region of the amino acid sequence of SEQ ID NO: 172 or any variant thereof.

In one aspect, the present disclosure relates to an antibody or antigen-binding portion thereof that binds human TSLP, comprising a heavy chain variable region of the amino acid sequence of SEQ ID NO: 127 or any variant thereof, and a light chain variable region of the amino acid sequence of SEQ ID NO: 172 or any variant thereof.

In one aspect, the present disclosure provides a nucleic acid encoding the aforementioned antibody or antigen-binding portion thereof according to any of the preceding aspects. Preferably, the nucleic acid molecule comprises an antibody heavy chain nucleotide sequence selected from SEQ ID NOs: 11, 21, 31, 41, 51, 61, 71, 81, 86, 91, 96, 101, 106, 111, 116, 121, 126, 131, 136, 141, 146, 151, 156, 161, 166, or any variant thereof, and/or an antibody light chain nucleotide sequence selected from SEQ ID NOs: 16, 26, 36, 46, 56, 66, 76, 171, 176, or any variant thereof.

In one aspect, the present disclosure relates to an antibody or antigen-binding portion thereof that binds to human TSLP, having at least greater than 60%, 65%, 70%, 75%, 80%, 85%, 90%, 95%, 96%, 97%, 98%, 99% or more sequence identity to the antibody or antigen-binding portion thereof of any of the preceding aspects.

In one aspect, the present disclosure relates to a nucleic acid molecule encoding the antibody or antigen-binding portion thereof according to any one of the preceding aspects, or a nucleic acid molecule having at least greater than 60%, 65%, 70%, 75%, 80%, 85%, 90%, 95%, 96%, 97%, 98%, 99% or more sequence identity thereto.

In one aspect, the present disclosure provides a vector comprising the nucleic acid according to any one of the preceding aspects.

In one aspect, the present disclosure provides a cell comprising the vector according to any one of the preceding aspects.

In one aspect, the present disclosure provides a pharmaceutical composition comprising an antibody or antigen-binding portion thereof or nucleic acids encoding the same according to any one of the preceding aspects and a pharmaceutically acceptable carrier.

In one aspect, the present disclosure provides a method of treating a TSLP-related disorder comprising the steps of administering to the mammal a therapeutically effective amount of an antibody or antigen-binding fragment thereof, or a nucleic acid molecule, or a vector, or a cell or a pharmaceutical composition according to any of the preceding aspects.

In one aspect, the present disclosure provides a use of the antibody or antigen-binding fragment thereof or the nucleic acid molecule or the vector or the cell or the pharmaceutical composition according to any one of the preceding aspects in the manufacture of a medicament for the treatment of a TSLP-related disorder in a mammal.

According to any one of the preceding aspects, optionally, the TSLP-related disorders are TSLP-related inflammatory disorders as well as an autoimmune disease. Optionally, the antibody is conjugated to other drugs, such as a labeled or cytotoxic conjugate.

In one aspect, the present disclosure also includes kits, e.g., comprising the antibody, the fragment, the homolog, derivative thereof, etc., of the disclosure, e.g., the labeled or cytotoxic conjugate, as well as the instructions for use of the antibody, the conjugate that kills a particular type of cell, and the like. The instructions can include directions for using the antibody, conjugate, etc. in vitro, in vivo, or ex vivo. The antibodies may be in liquid form or in solid form, usually lyophilized. The kit may contain other suitable reagents, such as buffers, reconstitution solutions and other necessary components for the intended use. Combinations of reagents packaged in predetermined amounts with instructions for their use, e. g. for therapeutic use or for conducting diagnostic assays, are contemplated. When the antibody is labeled, e. g. with an enzyme, then the kit can include a substrate and cofactors required for the enzyme (e. g. a substrate precursor providing a detectable chromophore or fluorophore). In addition, other additives such as stabilizers, buffers (e. g. blocking buffers or lysis buffers), and the like may also be included. The relative amounts of the various reagents can be varied to provide a concentrate of reagent solution, which provides user flexibility, and savings space and reagent. These reagents may also be provided in dry powder form, usually in lyophilized form, including excipients which, when dissolved, provide a reagent solution having the appropriate concentration.

In one aspect, the present disclosure provides a use of the antibody or functional fragment thereof and/or the nucleic acid molecule or the vector or the cell or the pharmaceutical composition and/or the kit according to any one of the preceding aspects in the manufacture of a reagent for inhibiting the binding of TSLP to TSLPR.

In addition, the antibodies of the present disclosure may be used in immunoassays, purification methods, and other methods using immunoglobulins or fragments thereof. Such uses are well known in the art.

Accordingly, the present disclosure also provides compositions comprising anti-TSLP antibody of the present disclosure, or fragment thereof, conveniently in combination with a pharmaceutically acceptable carrier, diluent, or excipient, as is conventional in the art.

As used herein, the term "pharmaceutical composition" refers to formulations of various preparations. Formulations containing therapeutically effective amounts of multivalent antibodies are in sterile liquid solution, liquid suspension, or lyophilized form, optionally containing stabilizers or excipients.

The antibodies of the present disclosure may be used as a composition administered alone, or may be used in combination with other active agents.

It will be appreciated that therapeutic agents according to the described embodiments will be administered with suitable pharmaceutically acceptable carriers, excipients, and other agents incorporated into formulations to provide improved transfer, delivery, tolerability, and the like. A large number of suitable formulations can be found in all pharmacopoeias known to pharmaceutical chemists: Remington's Pharmaceutical Sciences (15th ed. Mack Publishing Company, Easton, Pa. (1975)), particularly Chapter 87: Blaug, Seymour. These formulations include, for example, powders, pastes, ointments, gels, waxes, oils, lipids, lipid-containing (cationic or anionic) carriers (e. g., Lipofectin^{™}), DNA conjugates, anhydrous syrups, oil-in-water and water-in-oil emulsions, emulsion polyethylene glycols (polyethylene glycols of various molecular weights), semi-solid gels, and semi-solid mixtures containing polyethylene glycol. Any of the foregoing mixtures may be suitable for the treatment or therapy according to the present disclosure, provided that the active ingredient in the formulation is not inactivated by the formulation and that the formulation is physiologically compatible and tolerates the route of administration.

In one embodiment, the antibody can be used as a therapeutic agent. Such agents will generally be used to treat, alleviate, and/or prevent a disease or pathology associated with aberrant expression, activity, and/or signaling of TSLP in a subject. The therapeutic regimens may be implemented using standard methods by identifying a subject, e. g., a human patient, having (or at risk of or developing) a disease or disorder associated with aberrant expression, activity, and/or signaling of TSLP, e. g., a TSLP-related disorder. An antibody preparation, preferably one with high specificity and affinity for its target antigen, is administered to a subject, which will generally have an effect due to its binding to the target. The administered antibody can eliminate or inhibit or interfere with expression, activity, and/or signaling function of the target (e. g., TSLP). The administered antibody can eliminate or inhibit or interfere with the binding of the target (e. g., TSLP) to the endogenous ligand to which it naturally binds. For example, an antibody binds to a target and modulates, blocks, inhibits, reduces, antagonizes, neutralizes, and/or otherwise interferes with the expression, activity, and/or signaling of TSLP. In some embodiments, to treat a disease or disorder associated with aberrant expression of TSLP, an antibody having heavy and light chain CDR can be administered to a subject.

By way of non-limiting example, TSLP-related disorders associated with aberrant TSLP expression, activity and/or signaling include TSLP-related inflammatory disorders as well as autoimmune diseases. TSLP-related inflammatory conditions include allergic inflammation, asthma, chronic obstructive pulmonary disease, atopic dermatitis, and eosinophilic esophagitis. The allergic inflammation includes allergic rhinitis, allergic sinusitis, and allergic conjunctivitis. The autoimmune diseases include rheumatoid arthritis and multiple sclerosis.

In another embodiment, antibodies against TSLP can be used in methods known in the art relating to localization and/or quantitation of TSLP (e. g., for determining TSLP and/or levels of TSLP in an appropriate physiological sample, for diagnostic methods, for protein imaging, etc.). In a given embodiment, an antibody comprising an antibody-derived antigen-binding domain specific for TSLP or a derivative, fragment, analog or homolog thereof is used as a pharmaceutically active compound (hereinafter "therapeutic agent").

In another embodiment, TSLP polypeptides can be isolated using antibodies specific for TSLP by standard techniques such as immunoaffinity, chromatography, or immunoprecipitation. The protein in a biological sample can be detected with antibodies (or fragments thereof) against the TSLP protein. In some embodiments, TSLP can be detected in a biological sample as part of a clinical testing procedure, for example, to determine the efficacy of a given therapeutic regimen. Detection may be facilitated by coupling (i. e., physically linking) the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazine aminofluorescein, dansyl chloride, or phycoerythrin; one example of a luminescent material includes luminol; examples of bioluminescent materials include luciferase, luciferin and aequorin, and examples of suitable radioactive materials include ¹²⁵I, ¹³¹I, ³⁵S, or ³H.

In another embodiment, antibodies according to the present disclosure can be used as reagents for detecting the presence of TSLP or a protein fragment thereof in a sample. In some embodiments, the antibody comprises a detectable label. The antibody is a polyclonal antibody, or more preferably a monoclonal antibody. Intact antibodies or fragments thereof (e. g., Fab, scFv or F(ab')₂) are used. The term "labeling" with respect to an antibody is intended to encompass direct labeling of the antibody by coupling (i. e., physically linking) a detectable substance to the antibody, as well as indirect labeling of the antibody by reaction with another reagent that is directly labeled. Examples of indirect labeling include detection of a primary antibody using a fluorescently labeled second antibody, and end-labeling of the antibody with biotin to enable detection with fluorescently labeled streptavidin. The term "biological sample" is intended to include tissues, cells, and biological fluids isolated from a subject, as well as tissues, cells, and fluids present in a subject. Thus, the term "biological sample" as used includes blood and fractions or components in blood, including serum, plasma, or lymph. In other words, the detection method of the described embodiments can be used to detect the analyte mRNA, protein, or genomic DNA in a biological sample both in vitro and in vivo. For example, in vitro techniques for detection of mRNA analytes include Norhtern hybridization and in situ hybridization. In vitro techniques for detection of protein analytes include enzyme-linked immunosorbent assays (ELISA), Western blotting, immunoprecipitations, and immunofluorescence. In vitro techniques for detection of genomic DNA analytes include Southern hybridization. Procedures for performing immunoassays are described, for example, in "ELISA: Theory and Practice: Methods in Molecular Biology", vol. 42, J. R. Crowther (ed.) Human Press, Totowa, N. J. 1995; "Immunoassay", E. Diamandis and T. Christopoulus, Academic Press, Inc., San Diego, Calif., 1996; and "Practice and Theory of Enzyme Immunoassays", P. Tijssen, Elsevier Science Publishers, Amsterdam, 1985. In addition, in vivo techniques for detection of protein analytes include introducing into a subject a labeled anti-analyte protein antibody. For example, an antibody can be labeled with a radiolabel, and the presence and location of the radiolabel in the subject can then be detected by standard imaging techniques.

The antibodies described herein and derivatives, fragments, analogs, and homologs thereof can be incorporated into pharmaceutical compositions suitable for administration. The principles and considerations involved in preparing such compositions, as well as guidance in selecting components, are well known in the art, for example, see Remington's Pharmaceutical Sciences: The Science and Practice of Pharmacy, 19th edition (Alfonso R. Gennaro et al. Eds.) Mack Pub. Co. Easton, Pa: 1995; Drug Absorption Enhancement: Concepts, Possibilities, Limitations, and Trends, Harwood Academic Publishers, Langhorne, Pa. 1994; and Peptide and Protein Drug Delivery (Advances In Parenteral Sciences, vol. 4), 1991, M. Dekker, New York.

Such compositions typically comprise the antibody and a pharmaceutically acceptable carrier. When antibody fragments are used, minimal inhibitory fragments that specifically bind to the target protein binding domain may be preferred. For example, based on the variable region sequence of an antibody, peptide molecules can be designed that retain the ability to bind to a target protein sequence. Such peptides can be chemically synthesized and/or produced by recombinant DNA techniques (see, e. g., Marasco et al. Proc .Natl. Acad. Sci. USA, 90:7889-7893 (1993)).

As used herein, the term "pharmaceutically acceptable carrier" is intended to include any and all solvents, dispersion media, coatings, antibacterial and antifungal agents, isotonic and absorption delaying agents, etc., compatible with pharmaceutical administration. Suitable pharmaceutically acceptable carriers are described in the latest edition of Remington's Pharmaceutical Sciences, a standard bibliography in the art, which is incorporated herein by reference. Preferred examples of such carriers or diluents include, but are not limited to, water, saline, Ringer's solution, dextrose solution, and 5% human serum albumin. Liposomes and non-aqueous vehicles such as fixed oils can also be used. The use of such media and agents for pharmaceutically active substances is well known in the art. Except insofar as any conventional media or agent is incompatible with the antibody, its use in the compositions is contemplated.

The pharmaceutical compositions of the embodiments are formulated to be compatible with their intended route of administration. Examples of routes of administration include parenteral, e. g., intravenous, intradermal, subcutaneous, oral (e. g., inhalation), transdermal (i. e., topical), transmucosal, and rectal administration. Solutions or suspensions for parenteral, intradermal or subcutaneous administration may include the following components: sterile diluents for injection such as water, saline solutions, fixed oils, polyethylene glycols, glycerol, propylene glycol or other synthetic solvents; antibacterial agents such as benzyl alcohol or methyl p-hydroxybenzoate; antioxidants such as ascorbic acid or sodium bisulfite; chelating agents such as EDTA; buffers, such as acetates, citrates, or phosphates, and reagents to adjust the osmotic pressure, such as sodium chloride or dextrose. The pH can be adjusted with acids or bases, such as hydrochloric acid or sodium hydroxide. The parenteral preparation can be enclosed in ampoules, disposable syringes, or multiple dose vials made of glass or plastic.

Pharmaceutical compositions suitable for injectable use may include sterile aqueous solutions (where water soluble) or dispersions and sterile powders for the extemporaneous preparation of sterile injectable solutions or dispersions. For intravenous administration, suitable pharmaceutically acceptable carriers include physiological saline, bacteriostatic water, Cremophor EL^{™} (BASF, Parsippany, N. J.) or phosphate buffered saline (PBS). In all cases, the composition must be sterile and should be fluid to the extent that easy syringability exists. It must be stable under the conditions of manufacture and storage and must be preserved against the contaminating from microorganisms such as bacteria and fungi. The carrier can be a solvent or dispersion medium containing, for example, water, ethanol, polyol (for example, glycerol, propylene glycol, and liquid polyethylene glycol, etc.), and suitable mixtures thereof. For example, the desired particle size can be maintained in the case of dispersions by the use of coatings such as lecithin, and proper fluidity can be maintained by the use of surfactants. Prevention of the action of microorganisms can be achieved by various antibacterial and antifungal agents, for example, parabens, chlorobutanol, phenol, ascorbic acid, thimerosal, etc. In many cases, it will be preferable to include isotonic agents, for example, sugars, polyalcohols such as mannitol, sorbitol, and sodium chloride in the composition. The absorption of the injectable compositions can be prolonged by encompassing in the composition an agent which delays absorption, for example, aluminum monostearate and gelatin.

Sterile injectable solutions can be prepared by incorporating the antibody in the required amount in an appropriate solvent with one or a combination of ingredients enumerated above, as required, followed by filtered sterilization. Generally, dispersions are prepared by incorporating the antibody into a sterile vehicle that contains a basic dispersion medium and the required other ingredients from those enumerated above. The resulting powders are vacuum-dried and freeze-dried to prepare sterile powder for injection containing the active ingredient and any additional desired ingredient from a sterile-filtered solution of those ingredients previously described.

For administration by inhalation, the compounds are delivered in the form of an aerosol spray from a pressurized container or dispenser or a nebulizer containing a suitable propellant, e.g., a gas such as carbon dioxide.

Systemic administration can also be carried out by transmucosal or transdermal means. For transmucosal or transdermal administration, penetrants appropriate to permeate the barrier are used in the formulation. Such penetrants are generally known in the art, and include, for example, detergents, bile salts, and fusidic acid derivatives for transmucosal administration. Transmucosal administration can be accomplished through the use of nasal sprays or suppositories. For transdermal administration, one or more of the antibodies can be formulated into ointments, salves, gels, or creams as generally known in the art.

The compounds may also be prepared in the form of suppositories (e. g., with conventional suppository bases such as cocoa butter or other glycerides) or retention enemas for rectal delivery. In one embodiment, the antibodies can be prepared with carriers that prevent their rapid elimination from the body, such as sustained/controlled release formulations, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparing such formulations will be apparent to those skilled in the art.

It is especially advantageous to formulate parenteral compositions in dosage unit form for ease of administration and uniformity of dosage. Dosage unit form, as used herein, refers to physically discrete units suited as unitary dosages for the subject to be treated; each unit contains a predetermined quantity of one or more of the antibodies calculated to produce the desired therapeutic effect in association with the required pharmaceutical carrier. The specification for the dosage unit forms of the described embodiments are dictated by and directly dependent on: the unique characteristics of antibodies and the specific therapeutic effects to be achieved, and the limitations inherent in the art of formulating such antibodies for treating individuals.

The pharmaceutical compositions can be presented in a container, pack, or dispenser together with instructions for administration.

The formulations described herein may also contain more than one of the antibodies, preferably those with complementary activities but without adversely affecting each other, depending on the particular situation to be treated. Alternatively or additionally, the composition may, for example, comprise an agent that enhances its function, such as a cytotoxic agent, a cytokine, a chemotherapeutic agent, or a growth inhibitor. Such molecules are suitably present in combination in amounts effective for the intended purpose. For example, they may be present in combination in a kit or in combination for use.

In one embodiment, one or more of the antibodies can be administered in combination therapy, i. e., in combination with other agents, such as therapeutic agents, which can be used to treat pathological conditions or disorders, such as various forms of cancer, autoimmune disorders, and inflammatory diseases. The term "in combination" means herein that the agents are administered substantially synchronously, simultaneously or sequentially. If administered sequentially, the first of the two compounds is still preferably detected at an effective concentration at the treatment site when the second compound is initially administered. In one aspect, a "combination" can also include both an antibody of the present disclosure and another therapeutic agent in a kit.

For example, combination therapy can comprise coformulation and/or coadministration of one or more antibodies described herein with one or more additional therapeutic agents as described in more detail below, for example, one or more cytokine and growth factor inhibitors, immunosuppressants, anti-inflammatory agents, metabolic inhibitors, enzyme inhibitors, and/or cytotoxins or cytostatic agents. Such combination therapy may advantageously utilize lower dosages of the administered therapeutic agents, thus avoiding possible toxicities or complications associated with each monotherapy.

For purposes of clarity and conciseness, features are described herein as part of the same or separate embodiments, however, it will be understood that the scope of the present disclosure may include some embodiments having a combination of all or some of the features described.

### Example

### Example 1: Preparation of TSLP and TSLPR recombinant proteins

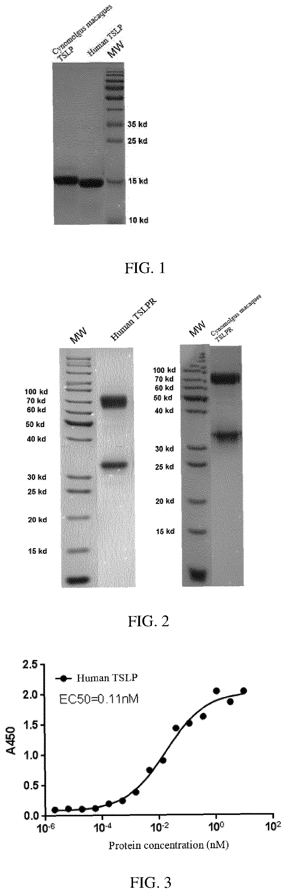

cDNA sequences of human TSLP (Uniprot: Q969D9, SEQ ID NO: 1) and Cynomolgus macaques TSLP (Uniprot: A0A2K5TXV0, SEQ ID NO: 2) were synthesized, then cloned into the eukaryotic expression vector pCMV3 (purchased from Beijing Sinobiological, Cat. No. CG90911-UT), with an insertion of signal peptide MDMRVPAQLLGLLLLWLRGARS at the N-terminal, and a fusion expression of the tag containing six histidines at the carboxy-terminal of TSLP, resulting in plasmids pSect-hTSLP-cHis and pSect-cyTSLP-cHis, which were then transfected into HEK293.6E cells (ATCC CRL-1573), the cell supernatant was collected and subjected to nickel-column affinity chromatography, but no recombinant protein of corresponding length was shown. The full-length fragment of TSLP was then cloned into prokaryotic expression plasmid pET-30a, which was transformed into BL21 *E.coli.,* and the recombinant protein was found to be expressed in inclusion body after induction of expression. The inclusion body proteins were refolded and purified to obtain recombinant proteins of human TSLP and Cynomolgus macaques TSLP. As shown in FIG. 1, the recombinant proteins of human TSLP and Cynomolgus macaques TSLP were purified.

Human TSLPR cDNA (Uniprot: Q9HC73, SEQ ID NO: 3, purchased from Beijing Sinobiological, Cat. No. HG18720-UT) was subjected to PCR amplification to obtain a gene fragment encoding the extracellular domain of human TSLPR (Gln23-Lys231), and the gene fragment was cloned downstream of the promoter of the eukaryotic expression vector pCMV3, with a fusion expression of human IgG1 Fc (knob) fragment at C-terminal, resulting in the plasmid pHC 1-TSLPR-hFc-knob. Meanwhile, the plasmid pHC1-hFc-hole containing only human IgG1 Fc (hole) fragment was constructed, then the eukaryotic expression plasmid was mixed with pHC1-TSLPR-hFc-knob plasmid, the mixture was co-transfected into eukaryotic cell HEK293.6E to obtain human TSLPR-Fc fusion proteins. Similarly, synthetic Cynomolgus macaques TSLPR cDNA (Uniprot: G8F663, SEQ ID NO: 4) was subjected to PCR amplification to obtain a gene fragment encoding the extracellular domain of Cynomolgus macaques TSLPR (Gln23-Lys231), and the gene fragment was cloned into a eukaryotic expression plasmid containing a human IgG1 Fc (knob) fragment, resulting in fusion of the extracellular domain of the Cynomolgus macaques TSLPR with the N-terminus of the human IgG1 Fc (knob), then the eukaryotic expression plasmid was mixed with a plasmid containing only the human IgG1 Fc (hole) fragment, the mixture was co-transfected into the eukaryotic cell HEK293.6E to obtain a Cynomolgus macaques TSLPR-Fc fusion protein. HEK293.6E cells were cultured for 5-7 days after being transfected with the plasmid, the cell supernatant was collected and purified by Protein A affinity chromatography to obtain recombinant proteins of extracellular domain of human TSLPR (SEQ ID NO: 5) and Cynomolgus macaques TSLPR (SEQ ID NO: 6), and the results were shown in FIG. 2.

The binding of the human TSLP recombinant protein to the human TSLPR extracellular domain recombinant protein was examined by ELISA, the result showed an EC₅₀=0.11 nM, indicating that recombinant human TSLP binds to human TSLPR with high affinity (FIG. 3).

### Example 2. Construction of TSLPR stably transfected cells

### 1) Construction of Ba/F3-hTSLPR stably transfected cell line

Ba/F3 cells were maintained in RPMI-1640 medium containing 10% fetal bovine serum, 50 µM 2-mercaptoethanol, 2 mM L-glutamine, 50 µg/mL penicillin-streptomycin, and 10 ng/mL mouse IL-3. The lentivirus expression vector expressing human TSLPR (Guangzhou FulenGen Co., Ltd., EX-W0156-Lv105-B) was used to package the virus by Lenti-Pac HIV Lentivirus Packaging Kit (Guangzhou FulenGen Co., Ltd.), and transfected into Ba/F3 cells, and puromycin was added for resistance screening to obtain the cell line Ba/F3-hTSLPR which can stably express human TSLPR.

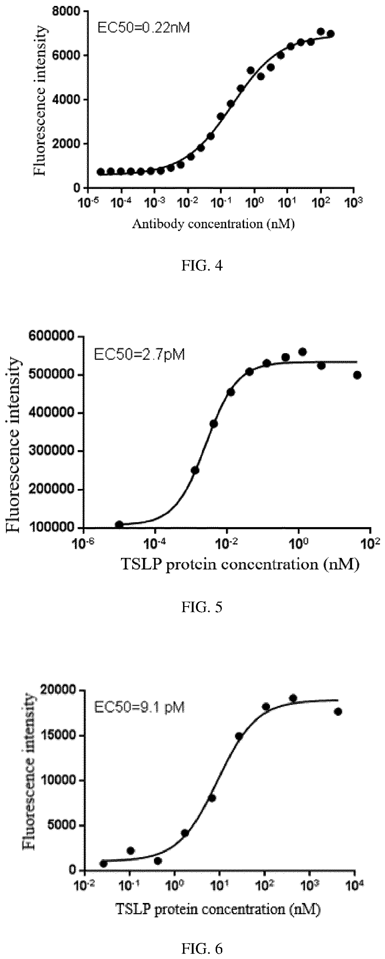

FIG. 4 shows that constructed cells expressed human TSLPR with an EC₅₀ = 0.22 nM as determined by the detection of the binding of biotinylated human TSLP protein to cell surface TSLPR.

### 2) Construction of Ba/F3-hTSLPR-IL7Rα stably transfected cell line

A heterodimeric receptor complex was formed by TSLP binding to TSLPR with IL-7Rα involving in the signaling of TSLP receptor complex, JAK1 in the intracellular segment of IL-7Rα and JAK2 in the intracellular segment of TSLPR were phosphorylated to activate downstream signaling molecules, thereby transducing activation signals to the inside of cells. IRES (GenBank KM077140.1, 99-745) and human IL-7Rα gene were cloned into the lentiviral expression vector expressing human TSLPR, and a double gene co-expression plasmid pLenti-CMV-TSLPR-IRES-IL7Rα-Puro was obtained and then transfected into Ba/F3 cells to construct stably transfected cells with complete human TSLP signal transduction pathway.

Ba/F3 cells were maintained in RPMI-1640 medium containing 10% fetal bovine serum, 2 mM L-glutamine, 50 µg/mL penicillin-streptomycin, and 2 ng/mL mouse IL-3. Human TSLPR and IL-7Rα double-gene co-expression plasmids were packaged into lentiviruses using a Lenti-Pac HIV lentivirus packaging kit, Ba/F3 cells were transduced with the culture supernatant containing recombinant virus, and puromycin was added for resistance screening to obtain the cell line Ba/F3-hTSLPR-IL7Rα which can stably express human TSLPR and IL-7Rα. The constructed Ba/F3-hTSLPR-IL7Rα cell line showed a dose-dependent proliferative response to human TSLP with an EC50 = 0.22 nM as measured by cell proliferative activity assay (FIG. 5).

### 3) Construction of Ba/F3-hTSLPR-IL7Rα-STAT5 luc reporter cell line

STAT 5-luc reporter gene was transferred into Ba/F3-hTSLPR-IL7Rα stably transfected cell line to screen STAT5 luciferase reporter cell line. Firstly, a nucleotide sequence containing five STAT5 response elements (AGTTCTGAGAAAAGT) was synthesized and cloned into pGL4.22-luc2P (Promega, E6761) by restriction endonuclease KpnI and HindIII, while the hygro resistance gene was cloned into the vector for replacing the Puro resistance gene, resulting in pGL4.22-luc2p STAT5 RE-hygro. Secondly, 10 µg of pGL4.22-luc2p STAT5 RE-hygro plasmid was mixed with 5×10⁶ Ba/F3-hTSLPR-IL7Rα stably transfected cells, and then electroporated at 300 V with a capacitance of 950 µF; 200 µg/mL hygromycin B solution was added 48-72 hours after transfection, the selection medium was changed every 3-5 days. After screening with a period of increasing antibiotic concentrations, colonies were formed by the stably transfected cells and subjected to limiting dilution to obtain the monoclonal strains, which can be stimulated with different concentrations of TSLP for 6 hours, and then the fluorescent signal can be detected. FIG. 6 shows that the fluorescence signal increased gradually with the increase of TSLP concentration, indicating that TSLP can activate the STAT 5 signaling pathway of the stably transfected Ba/F3-hTSLPR-IL7Rα-STAT5 luc with an EC50 = 0.22 nm.

### Example 3 Immunization of animals

6-week-old female Balb/C mice were selected for subcutaneous immunization once every two weeks, in which each mouse was alternately immunized with 50 µg of the antigen (TSLP from human and Cynomolgus macaques) that was mixed with an equal volume of Freund's adjuvant. After four-time immunization, blood was collected from the tail, and the titers of mouse serum and the inhibitory effect of serum on the binding of TSLP to TSLPR were determined by ELISA. Alternatively, after the initial immunization, booster immunizations were performed every other week using a gene gun, in which the exposed mouse abdominal bare skin was bombarded with a mixture of human TSLP expression plasmid and gold powder at a pressure of 400 psi for a total of nine times.

### Example 4 Antibody screening

### 1) Fab phage library

Three weeks after the last immunization, the immunized animals of Example 3 were challenging immunized with human TSLP recombinant protein at 10 µg/100 µL/mouse by tail vein injection. Four days later, RNA from mouse lymph nodes and splenocytes was extracted by a TRNzol lysis method, then subjected to reverse transcription to synthesize single-chain cDNA, which was used as a template to amplify variable region sequence of antibody. A TSLP immune library was constructed on the Fab antibody fragment phage display platform, with a library size of more than 5×10⁹, and 96 monoclones were randomly selected for induced expression. The expression rate of light and heavy chains in Western blot was higher than 95%, and the sequence diversity was good. TSLP antibody libraries were screened by coating human TSLP recombinant protein or Cynomolgus macaques TSLP recombinant protein in Maxisorp immunotube. After two rounds of screening, several positive clones were obtained which could specifically recognize TSLP and efficiently block TSLP-TSLPR binding. The monoclonal antibody Fab was expressed in TG1 competent cells, and the binding to human and Cynomolgus macaques TSLP and the blockade of TSLP/TSLPR were verified again, resulting in 6 clones with high affinity that can bind to both human and Cynomolgus macaques simultaneously and block TSLP binding to TSLPR with high efficiency (the results were shown in Table 1).

**Table 1 Screening Fab phage library by ELISA**

| | Binding of Human TSLP | Blockade of human TSLP | Binding of Cynomolgus macaques TSLP | Blockade of Cynomolgus macaques TSLP |
|---|---|---|---|---|
| 71G4 | +++ | ++ | ++ | ++ |
| 79D6 | +++ | +++ | +++ | +++ |
| 76A8 | ++ | ++ | + | ++ |
| 80D12 | +++ | +++ | +++ | +++ |
| 80E11 | +++ | +++ | +++ | +++ |
| 80B12 | +++ | +++ | ++ | ++ |

### 2) Mouse hybridoma

The mice were challenging immunized with human TSLP recombinant protein at 10 µg/100 µL/mouse by tail vein injection. Four days later, lymph nodes and spleens of the mice were collected and triturated in DMEM to obtain B cell suspension, which was mixed with SP2/0 in an appropriate amount, and subjected to cell fusion with an electric fusion instrument. The fusion cells were cultured in DMEM complete medium containing HAT at 5% CO₂, 37°C.

### 3) Screening of hybridomas

### Screening for the binding to human TSLP by Enzyme-linked immunosorbent assay (ELISA)

Human TSLP recombinant protein was coated onto a 96-well microtiter plate at 1 ug/mL in carbonate buffer overnight at 4°C. The plate was washed three times with PBS and blocked by adding 200 µL/well of PBS containing 2% skim milk for 1 hour. After washing once with PBS, 100 µL of hybridoma supernatant or phage supernatant was added to each well and incubated at room temperature for 60 minutes; after washing with PBST (PBS + 0.05% Tween-20) and PBS three times, respectively, 100 µL of HRP-labeled anti-human IgG Fc secondary antibody was added to each well, and the mixture was incubated at room temperature for 60 minutes. After washing with PBST and PBS three times, respectively, 100 µL of TMB substrate was added to each well for color development at 37°C for 10 minutes, the reaction was terminated with 50 µL of 2M sulfuric acid solution per well, and the absorbance was read at a wavelength of 450 nm.

### Screening for the binding to Cynomolgus macaques TSLP by hybridoma cell ELISA

Cynomolgus macaques TSLP recombinant protein was coated onto a 96-well microtiter plate at 1 ug/mL in carbonate buffer overnight at 4°C. The plate was washed three times with PBS and blocked by adding 200 µL/well of PBS containing 2% skim milk for 1 hour. After washing once with PBS, 100 µL of hybridoma supernatant or phage supernatant was added to each well and incubated at room temperature for 60 minutes; after washing with PBST and PBS three times, respectively, 100 µL of HRP-labeled anti-human IgG Fc secondary antibody was added to each well, and the mixture was incubated at room temperature for 60 minutes. After washing with PBST and PBS three times, respectively, 100 µL of TMB substrate was added to each well for color development at 37°C for 10 minutes, the reaction was terminated with 50 µL of 2M sulfuric acid solution per well, and the absorbance was read at a wavelength of 450 nm.

### Screening for blockade of human TSLP- TSLPR binding by ELISA

Human TSLPR recombinant protein was coated onto a 96-well microtiter plate at 1 µg/mL in carbonate buffer overnight at 4°C. The plate was washed three times with PBS and blocked by adding 200 µL/well of PBS containing 2% skim milk for 1 hour. Hybridoma cell supernatants or phage supernatants were incubated with 60 ng/mL biotinylated human TSLP protein in a blocked U-shaped 96-well plate for 30 minutes at room temperature. The 96-well plate was washed three times with PBS, and 100 µL of the incubated mixture was added to each well and incubated for 1 hour at room temperature. After washing with PBST and PBS three times, 100 µL of streptavidin-conjugated horseradish peroxidase (SA-HRP) was added to each well and incubated at room temperature for 30 minutes in the dark. After washing with PBST and PBS three times, respectively, 100 µL of TMB substrate was added to well for color development at 37°C for 10 minutes, the reaction was terminated with 50 µL of 2M sulfuric acid solution per well, and the absorbance was read at a wavelength of 450 nm.

### Screening for blockade of Cynomolgus macaques TSLP and Cynomolgus macaques TSLPR by ELISA

Cynomolgus macaques TSLPR recombinant protein was coated onto a 96-well microtiter plate at 1 µg/mL in carbonate buffer overnight at 4°C. The plate was washed three times with PBS and blocked by adding 200 µL/well of PBS containing 2% skim milk for 1 hour. Hybridoma cell supernatants or phage supernatants were incubated with 60 ng/mL biotinylated Cynomolgus macaques TSLP protein in a blocked U-shaped 96-well plate for 30 minutes at room temperature. The 96-well plate was washed three times with PBS, and 100 µL of the incubated mixture was added to each well and incubated for 1 hour at room temperature. After washing with PBST and PBS three times, respectively, 100 µL of SA-HRP was added to each well and incubated at room temperature for 30 minutes in the dark. After washing with PBST and PBS three times, respectively, 100 µL of TMB substrate was added to well for color development at 37°C for 10 minutes, the reaction was terminated with 50 µL of 2M sulfuric acid solution per well, and the absorbance was read at a wavelength of 450 nm.

### Blockade at the cellular level

Ba/F3-hTSLPR cells were added to a 96-well U-plate at 5×10⁴/well. The antibody was incubated with biotinylated human TSLP protein at 4°C for 30 minutes and then added to the cells, the mixture was incubated at 4°C for 1 hour, then streptavidin-conjugated phycoerythrin fluorescein (SA-PE) was added, the mixture was incubated for 45 minutes at 4°C. The blocking effect of the antibody at the cellular level was detected by flow cytometry.

A total of 712 clones of hybridomas binding to human TSLP were screened by ELISA, wherein 52 clones of hybridomas could block the binding of human TSLP to human TSLPR in both ELISA and cell-based assays. There were 6 positive clones that bound to Cynomolgus macaques TSLP and blocked the binding of Cynomolgus macaques TSLP to Cynomolgus macaques TSLPR (see Table 2 for results).

**Table 2 Screening hybridoma supernatants by ELISA**

| | Binding of Human TSLP | Binding of Cynomolgus macaques TSLP | Blockade of Human TSLP | Blockade of Cynomolgus macaques TSLP |
|---|---|---|---|---|
| 1A7 | +++ | +++ | - | ND |
| 2G8 | +++ | +++ | - | ND |
| 4F4 | +++ | +++ | ++ | - |
| 5A5 | ++ | ++ | - | ND |
| 7E7 | +++ | +++ | - | ND |
| 8C3 | +++ | ++ | - | ND |
| 8B5 | ++ | + | - | ND |
| 9C5 | +++ | +++ | ++ | - |
| 9B7 | +++ | +++ | - | ND |
| 9G11 | +++ | + | - | ND |
| 9E12 | ++ | + | - | ND |
| 10A4 | ++ | - | - | ND |
| 10E4 | ++ | + | - | ND |
| 11E2 | +++ | - | + | ND |
| 11G7 | ++ | + | - | ND |
| 11B12 | ++ | + | - | ND |
| 13A5 | +++ | +++ | - | ND |
| 17H3 | +++ | - | - | ND |
| 17D7 | +++ | +++ | ++ | - |
| 17H8 | +++ | + | - | ND |
| 18G6 | ++ | - | - | ND |
| 19F6 | ++ | +++ | - | ND |
| 19F9 | ++ | + | - | ND |
| 21B5 | ++ | ++ | ++ | - |
| 22C4 | ++ | + | - | ND |
| 22B10 | ++ | + | ++ | + |
| 25G6 | ++ | + | +++ | - |
| 26C8 | ++ | ++ | - | ND |
| 26A9 | +++ | +++ | - | ND |
| 26D11 | ++ | + | ++ | - |
| 27F11 | +++ | +++ | - | ND |
| 27H11 | +++ | +++ | - | ND |
| 29F1 | ++ | ++ | +++ | + |
| 30B4 | ++ | ++ | - | ND |
| 31G6 | ++ | + | +++ | + |
| 35H10 | +++ | +++ | - | ND |
| 36B6 | ++ | - | + | ND |
| 38E2 | ++ | + | - | ND |
| 38A6 | ++ | + | +++ | ++ |
| 39G5 | +++ | ++ | +++ | +++ |
| 39B11 | ++ | + | +++ | + |
| 40B4 | +++ | +++ | - | ND |

| | | | | |
|---|---|---|---|---|
| ND in Table 2 means undetected | | | | |

### Example 5 Acquisition of antibody sequences

According to the results of hybridoma screening, the positive monoclonal cells were centrifuged at 1000 rpm, the cells were collected, and total RNA extracted with Trizol was used as a template to synthesize a first-strand cDNA, which was used as a subsequent template to amplify the variable region DNA sequence corresponding to the hybridoma cells. In a 50 µL reaction system, 1 µL of cDNA, 5 µL of 10 × PCR buffer, 1 µL of each of forward and reverse primers, 1 µL of dNTP, 1 µL of 25 mmol MgCl₂, 39 µL of H₂O, and 1 µL of Taq enzyme were added, and PCR amplification was performed including initial denaturation at 95°C for 10 minutes and temperature cycling. The reaction conditions were 30 cycles of denaturation at 94°C for 1 minute, annealing at 58°C for 1 minute, extension at 72°C for 15 seconds, followed by incubation at 72°C for 10 minutes. Based on the results of phage screening, the variable region sequences of positive clones were amplified. After sequencing, the heavy and light chain variable region sequences of the obtained candidate positive clones were as follows respectively:
Clones: 71G4 SEQ ID Nos:7-16
   Heavy chain VH 
<-------------FR1-----------> CDR1 <----FR2-----> CDR2 <--
QVQLQQSGAELVMPGASVKMSCKASGYTFTDYWMHWVKQRPGQGLEWIGTIDNSDSDTTYNQKFKGKVT
-------------FR3-------------> CDR3 <---FR4--->
LTVDESSSTAYMQLSSLTSEDSAVYYCARSVDGYLDYWGQGTTLTVSS
   Nucleotide sequence 
CAGGTTCAGCTGCAGCAGTCTGGGGCTGAGCTTGTGATGCCTGGGGCTTCAGTGAAGATGTCCTGCAAG
GCTTCTGGCTACACATTCACTGACTACTGGATGCACTGGGTGAAGCAGAGGCCTGGACAAGGCCTTGAG
TGGATTGGAACGATTGATAATTCTGATAGTGATACAACCTACAATCAAAAGTTCAAGGGCAAGGTCACA
TTGACTGTAGACGAATCCTCCAGCACAGCCTACATGCAGCTCAGCAGCCTGACATCTGAGGACTCTGCG
GTCTATTACTGTGCAAGATCGGTTGATGGTTACCTTGACTACTGGGGCCAAGGCACCACTCTCACAGTC
TCCTCA
   Light chain VK 
<-----------FR1--------->    CDR1    <-----FR2-----> CDR2 <-----------
EIQMTQSPASLSASVGETVTITCRTSENIYSYLAWYQQKQGKSPQLLVYFAKTLTDGVPSRLSGSGSGT
---------FR3-------->    CDR3    <---FR4--->
QFSLKINSLQPEDFGSYYCQHHYGTPWTFGGGTKLEIK
 Nucleotide sequence 
GAAATCCAGATGACCCAGTCTCCAGCCTCCCTATCTGCATCTGTGGGAGAAACTGTCACCATCACATGT
CGAACAAGTGAGAATATTTACAGTTATTTAGCATGGTATCAGCAGAAACAGGGAAAATCTCCTCAGCTC
CTGGTCTATTTTGCAAAAACCTTAACAGACGGTGTGCCATCAAGGCTCAGTGGCAGTGGATCAGGCACA
CAGTTTTCTCTGAAGATCAACAGCCTGCAGCCTGAAGATTTTGGGAGTTATTACTGTCAACATCATTAT
GGTACTCCGTGGACGTTCGGTGGAGGCACCAAGCTGGAAATCAAA
Clones: 79D6 SEQ ID Nos:17-26
   Heavy chain VH 
<-------------FR1----------> CDR1 <----FR2-----> CDR2 <--
QVQLQQSGAELVMPGASVKMSCKASGYTFTGYWMHWVKQRPGQGLEWIGTIDNSDSDTTYNQKFKGKVT
------------FR3-------------> CDR3 <---FR4--->
LTVDESSSTAYMQLSSLTSEDSAVYYCARSVDGYLDYWGQGTTLTVSS
   Nucleotide sequence 
CAGGTCCAGCTGCAGCAGTCTGGGGCTGAGCTTGTGATGCCTGGGGCTTCAGTGAAGATGTCCTGCAAG
GCTTCTGGCTACACATTCACTGGCTACTGGATGCACTGGGTGAAGCAGAGGCCTGGACAAGGCCTTGAG
TGGATTGGAACGATTGATAATTCTGATAGTGATACAACCTACAATCAAAAGTTCAAGGGCAAGGTCACA
TTGACTGTAGACGAATCCTCCAGCACAGCCTACATGCAGCTCAGCAGCCTGACATCTGAGGACTCTGCG
GTCTATTACTGTGCAAGATCGGTTGATGGTTACCTTGACTACTGGGGCCAAGGCACCACTCTCACAGTC
TCCTCA
   Light chain VK 
<-----------FR1--------->    CDR1    <-----FR2----->    CDR2 <-----------
DIQMIQSPASLSASVGETVTITCRASENIYSYLAWYQQIQGKSPQLLVYYAKTLPEGVPSRFSGSGSGT
---------FR3-------->    CDR3    <---FR4--->
QFSLKINSLQPEDFGSYYCQHHYGTPWTFGGGTKLEIK
   Nucleotide sequence 
GACATCCAGATGATTCAGTCTCCAGCCTCCCTATCTGCATCTGTGGGAGAAACTGTCACCATCACATGC
CGAGCAAGTGAGAATATTTACAGTTATTTAGCATGGTATCAGCAGATACAGGGAAAATCTCCTCAGCTC
CTGGTCTATTATGCAAAAACCTTACCAGAAGGTGTGCCATCAAGGTTCAGTGGCAGTGGATCAGGCACA
CAGTTTTCTCTGAAGATCAACAGCCTGCAGCCTGAAGATTTTGGGAGTTATTACTGTCAACATCATTAC
GGTACCCCGTGGACGTTCGGTGGAGGCACCAAGCTGGAAATCAAA
Clones: 76A8 SEQ ID Nos:27-36
   Heavy chain VH 
<-------------FR1-----------> CDR1 <----FR2-----> CDR2 <--
QVQLQQSGPQLVRPGASVKISCKASGYSFTSYWMHWVKQRPGQGLEWIGLIDPSDSETGLNQRFKDKAT
-------------FR3---------------> CDR3 <---FR4--->
LTVDNPSSTAYMQLSSPTSEDSAVYYCARSLDGYYDYWGQGTTLTVSS
   Nucleotide sequence 
CAGGTCCAGCTGCAGCAGTCTGGGCCTCAGCTGGTTAGGCCTGGGGCTTCAGTAAAGATATCCTGCAAG
GCTTCTGGTTACTCATTCACCAGCTACTGGATGCACTGGGTGAAGCAGAGGCCTGGACAAGGTCTTGAG
TGGATTGGCTTGATTGATCCTTCCGATAGTGAAACTGGGTTAAATCAGAGGTTCAAGGACAAGGCCACA
TTGACTGTAGACAATCCCTCCAGCACAGCCTACATGCAACTCAGCAGCCCGACATCTGAGGACTCTGCG
GTCTATTACTGTGCAAGATCCCTTGATGGTTACTATGACTACTGGGGCCAAGGCACCACTCTCACAGTC
TCCTCA
   Light chain VK 
<-----------FR1--------->    CDR1    <-----FR2----->  CDR2 <-----------
DIQMIQSPASLSASVGETVTITCRASGNIHNYLAWYQQKQGKSPQLLVYNAKTLADGVPSRFSGSGSGT
---------FR3-------->    CDR3  <---FR4--->
QYSLKINSLQPEDFGSYYCQHFWSTPWTFGGGTRLEIK
   Nucleotide sequence 
GACATCCAGATGATTCAGTCTCCAGCCTCCCTATCTGCATCTGTGGGAGAAACTGTCACCATCACATGT
CGAGCAAGTGGGAATATTCACAATTATTTAGCATGGTATCAGCAGAAACAGGGAAAATCTCCTCAGCTC
CTGGTCTATAATGCAAAAACCTTAGCAGATGGTGTGCCATCAAGGTTCAGTGGCAGTGGATCAGGAACA
CAATATTCTCTCAAGATCAACAGCCTGCAGCCTGAAGATTTTGGGAGTTATTACTGTCAACATTTTTGG
AGTACTCCGTGGACGTTCGGTGGAGGCACCAGACTGGAAATAAAA
Clones: 80D12 SEQ ID Nos:37-46
   Heavy chain VH 
<-------------FR1-----------> CDR1 <----FR2-----> CDR2 <--
QVQLKQSGPQLVRPGASVKISCKASGYSFTSYWMHWVKQRPGQGLEWIGMIDPSDSETTLNQKFKDKAT
------------FR3--------------> CDR3 <---FR4--->
LTVDKSSTTAYMQLSSPTSEDSAVYYCARSLDGYYDYWGQGTTLTVSS
   Nucleotide sequence 
CAGGTCCAGCTGAAGCAGTCTGGGCCTCAGTTGGTTAGGCCTGGGGCTTCAGTGAAGATATCCTGCAAG
GCTTCTGGTTACTCATTCACCAGCTACTGGATGCACTGGGTGAAGCAGAGGCCTGGTCAAGGTCTTGAG
TGGATTGGCATGATTGATCCTTCCGATAGTGAAACTACGTTAAATCAGAAGTTCAAGGACAAGGCCACA
TTGACTGTAGACAAATCCTCCACCACAGCCTACATGCAACTCAGCAGCCCGACATCTGAGGACTCTGCG
GTCTATTACTGTGCAAGATCCCTTGATGGTTACTACGACTACTGGGGCCAAGGCACCACTCTCACAGTC
TCCTCA
   Light chain VK 
<-----------FR1--------->     CDR1    <-----FR2----->  CDR2 <------------
DVQMIQSPASLSASVGETVTITCRTSENIYSYLAWYQQKQGKSPQLLVYFAKTLTDGVPSRFSGSGSGT
---------FR3-------->     CDR3    <---FR4--->
QFSLKINSLQPEDFGSYYCQHHYGTPWTFGGGTKLEIK
   Nucleotide sequence 
GATGTCCAGATGATTCAGTCTCCAGCCTCCCTATCTGCATCTGTGGGAGAAACTGTCACCATCACATGT
CGAACAAGTGAGAATATTTACAGTTATTTAGCATGGTATCAGCAGAAACAGGGAAAATCTCCTCAGCTC
CTGGTCTATTTTGCAAAAACCTTAACAGACGGTGTGCCATCAAGGTTCAGTGGCAGTGGATCAGGCACA
CAGTTTTCTCTGAAGATCAACAGCCTGCAGCCTGAAGATTTTGGGAGTTATTACTGTCAACATCATTAT
GGTACTCCGTGGACGTTCGGTGGAGGCACCAAGCTGGAAATCAAA
Clones: 80E11 SEQ ID Nos:47-56
   Heavy chain VH 
<-------------FR1-----------> CDR1 <----FR2-----> CDR2 <--
QVQLQQSGPQLVRPGASVKISCKASGYSFTSYWMHWVKQRPGQGLEWIGVIDPSDSETILNQKFKDKAT
-----------FR3-------------> CDR3 <---FR4--->
LTVDKSSSTAYMQFSSPTSEDSAVYYCARSLDGYYDYWGQGTTLTVSS
   Nucleotide sequence 
CAGGTTCAGCTGCAGCAGTCTGGGCCTCAGCTGGTTAGGCCTGGGGCTTCAGTGAAGATATCCTGCAAG
GCTTCTGGTTACTCATTCACCAGCTACTGGATGCACTGGGTGAAGCAGAGGCCTGGACAAGGTCTTGAG
TGGATTGGCGTGATTGATCCTTCCGATAGTGAGACTATATTAAATCAGAAGTTCAAGGACAAGGCCACA
TTGACTGTAGACAAATCCTCCAGTACAGCCTACATGCAATTCAGCAGCCCGACATCTGAGGACTCTGCG
GTCTATTACTGTGCAAGATCCCTTGATGGTTACTACGACTACTGGGGCCAAGGCACCACTCTCACAGTC
TCCTCA
   Light chain VK 
<-----------FR1--------->     CDR1     <-----FR2-----> CDR2 <-----------
DIQMTQSPASLSASVGETVTITCRAGENIYSYLAWYQQKQGKSPQLLVYNAKTLAEGVPSRFSGSASGT
---------FR3-------->     CDR3   <---FR4--->
QFSLKINSLQPEDFGSYYCQHFWGTPWAFGGGTRLEIK
   Nucleotide sequence 
GACATCCAGATGACACAGTCTCCAGCCTCCCTATCTGCATCTGTGGGAGAAACTGTCACCATCACATGT
CGAGCAGGTGAGAATATTTACAGTTATTTAGCATGGTATCAGCAGAAACAGGGAAAATCTCCTCAGCTC
CTGGTCTATAATGCAAAAACCTTAGCAGAAGGTGTGCCATCAAGGTTCAGTGGCAGTGCATCAGGCACA
CAGTTTTCTCTGAAGATCAACAGCCTGCAGCCTGAAGATTTTGGGAGTTATTACTGTCAACATTTTTGG
GGTACTCCGTGGGCGTTCGGTGGAGGCACCAGACTGGAAATAAAA
Clones: 80B12 SEQ ID Nos:57-66
   Heavy chain VH 
<-------------FR1-----------> CDR1 <----FR2-----> CDR2 <--
QVQLQQSGPQLVRPGASVKISCKASGYSFTTYWMHWVKQRPGQGLEWIGLIDPSDGEAGLNQNFKDKAT
-------------FR3-------------> CDR3 <---FR4--->
LTVDISSRTAYMQISSPTSEDSAVYFCARSLDGYYDYWGQGTTLTVSS
   Nucleotide sequence 
CAGGTCCAGCTGCAGCAGTCTGGGCCTCAGCTGGTTAGGCCTGGGGCTTCAGTGAAGATATCCTGCAAG
GCTTCTGGTTACTCATTCACCACCTACTGGATGCACTGGGTGAAGCAGAGGCCTGGACAAGGTCTTGAG
TGGATTGGCTTGATTGATCCTTCCGATGGTGAAGCTGGGTTAAATCAGAACTTCAAGGACAAGGCCACA
TTGACTGTAGACATTTCCTCCAGAACAGCCTACATGCAAATCAGCAGCCCGACATCTGAGGACTCTGCG
GTTTATTTCTGTGCAAGATCCCTTGATGGTTACTACGACTACTGGGGCCAAGGCACCACTCTCACAGTC
TCCTCA
   Light chain VK 
<-----------FR1--------->     CDR1    <-----FR2----->  CDR2  <-----------
DIQMTQSPASLSASVGETVTITCRASEDIYSYLAWYQQKQGKSPQLLVYNAKTLAEGVPSRFSGSGSGT
--------FR3------->     CDR3   <---FR4--->
QFSLKINSLQPEDFGSYYCQHFWSTPWTFGGGTKLELK
   Nucleotide sequence 
GACATCCAGATGACCCAGTCTCCAGCCTCCCTGTCTGCATCTGTGGGAGAAACTGTCACCATCACATGT
CGAGCAAGTGAGGATATTTACAGTTATTTAGCATGGTATCAGCAGAAACAGGGAAAATCTCCTCAGCTC
CTGGTCTATAATGCAAAAACCTTAGCAGAAGGTGTGCCATCAAGGTTCAGTGGCAGTGGATCAGGCACA
CAGTTTTCTCTGAAGATCAACAGCCTGCAGCCTGAAGATTTTGGGAGTTATTACTGTCAACATTTTTGG
AGTACTCCGTGGACGTTCGGTGGAGGCACCAAGCTGGAGCTGAAA
Clones: 39G5-H8 SEQ ID Nos:67-76
   Heavy chain VH 
<-------------FR1-----------> CDR1 <----FR2-----> CDR2 <--
QVQLLQSGPQLVRPGASVKISCKASGYSFISYWIHWVKQRPGQGLEWIGIIDPSDSDTSLNQKFKDKAT
------------FR3--------------> CDR3 <---FR4--->
LTVDKSSTTAYIQLSSPTSEDSAVYYCARSLDGYYDYWGQGTTLTVSS
   Nucleotide sequence 
CAGGTGCAACTACTGcAGTCTGGGCCTCAGCTGGTTAGGCCTGGGGCTTCAGTGAAGATATCCTGCAAG
GCTTCTGGTTACTCATTCATTAGTTACTGGATACACTGGGTGAAGCAGAGGCCTGGACAAGGTCTTGAG
TGGATTGGCATTATTGATCCTTCCGATAGTGACACTAGCTTAAATCAGAAGTTCAAGGACAAGGCCACA
TTGACTGTGGACAAATCCTCCACCACAGCCTACATTCAACTCAGCAGCCCGACATCTGAGGACTCTGCG
GTCTATTACTGTGCAAGATCCCTTGATGGTTACTACGACTACTGGGGCCAAGGCACCACTCTCACAGTC
TCCTCA
   Light chain VK 
<-----------FR1--------->     CDR1     <-----FR2----->  CDR2 <-----------
DIQMTQSPASLSASVGETVTITCRASENIYSYLAWYQQKQGKSPQLLVYNARTLPEGVPSRFSGSGSGT
---------FR3-------->     CDR3   <---FR4--->
QFSLKINSLQPEDFGIYYCQHHYTTPWTFGGGTKLEIK
   Nucleotide sequence

GACATCCAGATGACTCAGTCTCCAGCCTCCCTATCTGCATCTGTGGGAGAAACTGTCACCATCACATGT
CGAGCAAGTGAGAATATTTACAGTTATTTAGCATGGTATCAGCAGAAACAGGGAAAATCTCCTCAACTC
CTGGTCTATAATGCAAGAACCTTACCAGAAGGTGTGCCATCACGGTTCAGTGGCAGTGGATCAGGCACA
CAGTTTTCTCTGAAGATCAACAGCCTGCAGCCTGAAGATTTTGGGATTTATTACTGTCAACATCATTAT
ACTACTCCGTGGACGTTCGGTGGAGGCACCAAGCTGGAAATCAAA

### Example 6 Preparation of anti-TSLP chimeric antibodies

The heavy and light chain variable region sequence fragments obtained in Example 5 were PCR amplified, and the heavy chain variable region was cloned into a vector containing a human heavy chain constant region to express the entire IgG1 heavy chain in mammalian cells. Similarly, the light chain variable region was cloned into a vector containing a human light chain constant region to express the entire kappa light chain in mammalian cells. The vectors were verified by sequencing and then transfected into HEK293.6E mammalian cells, and IgG1 was expressed and secreted into the culture medium, and purified by Protein A chromatography, wherein the supernatant was merged, collected, filtered, and IgG was purified by Protein A chromatography, the eluted protein was concentrated by ultrafiltration, the concentration of IgG was determined by spectrophotometry, and the purity of IgG was analyzed by SDS-PAGE. Chimeric antibodies of 39G5-H8, 71G4, 79D6, 80B12, 80D12, and 80E11 were obtained.

### Example 7 Determination of affinity for chimeric antibodies

### 1) Binding of antibodies to human TSLP

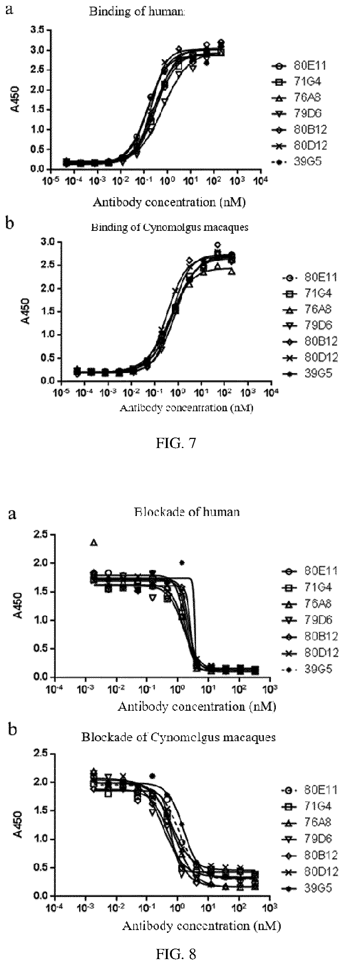

Human TSLP recombinant protein was coated onto a 96-well microtiter plate at 1 µg/mL in carbonate buffer overnight at 4°C. The plate was washed three times with PBS and blocked by adding 200 µL/well of PBS containing 2% skim milk for 1 hour. After washing once with PBS, 100 µL of anti-TSLP antibodies were added to each well and incubated at room temperature for 60 minutes; after washing with PBST and PBS three times, respectively, 100 µL of HRP-labeled anti-human IgG Fc secondary antibody was added to each well, and the mixture was incubated at room temperature for 60 minutes. After washing with PBST and PBS three times, respectively, 100 µL of TMB substrate was added to each well for color development at 37°C for 10 minutes, the reaction was terminated with 50 µL of 2M sulfuric acid solution per well, and the absorbance was read at a wavelength of 450 nm. FIG. 7-a and Table 3 show that each of the chimeric antibodies obtained in Example 6 bound to human TSLP.

### 2) Binding of antibodies to Cynomolgus macaques TSLP

Cynomolgus macaques TSLP recombinant protein was coated onto a 96-well microtiter plate at 1 ug/mL in carbonate buffer overnight at 4°C. The plate was washed three times with PBS and blocked by adding 200 µL/well of PBS containing 2% skim milk for 1 hour. After washing once with PBS, 100 µL of anti-TSLP antibodies were added to each well and incubated at room temperature for 60 minutes; after washing with PBST and PBS three times, respectively, 100 µL of HRP-labeled anti-human IgG Fc secondary antibody was added to each well, and the mixture was incubated at room temperature for 60 minutes. After washing with PBST and PBS three times, respectively, 100 µL of TMB substrate was added to each well for color development at 37°C for 10 minutes, the reaction was terminated with 50 µL of 2M sulfuric acid solution per well, and the absorbance was read at a wavelength of 450 nm. FIG. 7-b and Table 3 show that each of the chimeric antibodies obtained in Example 6 bound to Cynomolgus macaques TSLP.

**Table 3 ELISA detection of chimeric antibodies**

| | EC50 (nM) | | IC50 (nM) | |
|---|---|---|---|---|
| | Binding of Human | Binding of Cynomolgus macaques | Blockade of Human | Blockade of Cynomolgus macaques |
| 76A8 | 0.26 | 0.47 | 1.42 | 0.73 |
| 79D6 | 0.58 | 0.73 | 1.35 | 0.31 |
| 71G4 | 0.32 | 0.60 | 1.52 | 0.56 |
| 80B 12 | 0.34 | 0.55 | 2.58 | 0.54 |
| 80E11 | 0.15 | 0.57 | 1.80 | 1.15 |
| 80D12 | 0.21 | 0.35 | 2.44 | 0.63 |
| 39G5 | 0.15 | 0.54 | 3.42 | 1.74 |

### 3) Affinity assay by BIAcore

The kinetic binding activity of anti-TSLP antibodies to human TSLP was measured using the BIAcore T200 system. An anti-TSLP antibody at 2 µg/ml was immobilized on the sensor chip Protein A using 1× HBS-EP buffer as running buffer at a flow rate of 10 µL/min for 40 seconds. Various concentrations of human TSLP ranging from 0 to 21.7 nM were injected onto the surface of immobilized anti-TSLP antibody at a flow rate of 30 µL/min. After each injection cycle, the Protein A chip surface was regenerated with regeneration buffer 10 mM glycine (pH 2.0) at a flow rate of 30 µL/min.

The association rate constant (ka) and dissociation rate constant (kd) and equilibrium dissociation constant KD were analyzed by fitting the resulting data to a Langmuir 1:1 kinetics theoretical model using BIAevaluation software. Table 4 shows that each chimeric antibody binds to human TSLP with high affinity.

**Table 4 Affinity of chimeric antibodies**

| | ka (1/Ms) | kd (1/s) | KD (pM) | Rmax (RU) | Chi² (RU²) | Capture antibody (RU) |
|---|---|---|---|---|---|---|
| 79D6 | 4.31E+06 | 8.77E-05 | 20.4 | 16.94 | 0.128 | 61.2 |
| 80E11 | 6.62E+06 | 1.23E-04 | 18.5 | 21.91 | 0.0993 | 80.2 |
| 39G5 | 4.84E+06 | 4.60E-05 | 9.50 | 18.16 | 0.0742 | 62.3 |
| 76A8 | 3.22E+06 | 8.36E-05 | 26.0 | 25.36 | 0.22 | 88.7 |
| 80D12 | 4.77E+06 | 2.84E-05 | 5.96 | 22.82 | 0.246 | 86.2 |
| 80B12 | 4.82E+06 | 1.08E-04 | 22.4 | 16.04 | 0.573 | 58.2 |
| 71G4 | 3.15E+06 | 3.16E-05 | 10.0 | 20.78 | 0.182 | 78.3 |

### Example 8 Blockade at ELISA levels

### 1) Blockade of the binding of human TSLP to human TSLPR by antibodies

Human TSLPR recombinant protein was coated onto a 96-well microtiter plate at 1 µg/mL in carbonate buffer overnight at 4°C. The plate was washed three times with PBS and blocked by adding 200 µL/well of PBS containing 2% skim milk for 1 hour. Anti-TSLP antibodies were incubated with 60 ng/mL biotinylated human TSLP protein in a blocked U-shaped 96-well plate for 30 minutes at room temperature. The 96-well plate was washed three times with PBS, and 100 µL of the incubated mixture was added to each well and incubated for 1 hour at room temperature. After washing with PBST and PBS three times, respectively, 100 µL of SA-HRP was added to each well and incubated at room temperature for 30 minutes in the dark. After washing with PBST and PBS three times, respectively, 100 µL of TMB substrate was added to well for color development at 37°C for 10 minutes, the reaction was terminated with 50 µL of 2M sulfuric acid solution per well, and the absorbance was read at a wavelength of 450 nm. FIG. 8-a and Table 3 show the blockade of the binding of human TSLP to human TSLPR by each of the chimeric antibodies.

### 2) Blockade of the binding of Cynomolgus macaques TSLP to Cynomolgus macaques TSLPR by antibodies

Cynomolgus macaques TSLPR recombinant protein was coated onto a 96-well microtiter plate at 1 µg/mL in carbonate buffer overnight at 4°C. The plate was washed three times with PBS and blocked by adding 200 µL/well of PBS containing 2% skim milk for 1 hour. Anti-TSLP antibodies were incubated with 60 ng/mL biotinylated Cynomolgus macaques TSLP protein in a blocked U-shaped 96-well plate for 30 minutes at room temperature. The 96-well plate was washed three times with PBS, and 100 µL of the incubated mixture was added to each well and incubated for 1 hour at room temperature. After washing with PBST and PBS three times, respectively, 100 µL of SA-HRP was added to each well and incubated at room temperature for 30 minutes in the dark. After washing with PBST and PBS three times, respectively, 100 µL of TMB substrate was added to well for color development at 37°C for 10 minutes, the reaction was terminated with 50 µL of 2M sulfuric acid solution per well, and the absorbance was read at a wavelength of 450 nm. FIG. 8-b and Table 3 show the blockade of the binding of Cynomolgus macaques TSLP to Cynomolgus macaques TSLPR by each of chimeric antibodies.

### Example 9 Blockade at the cellular level

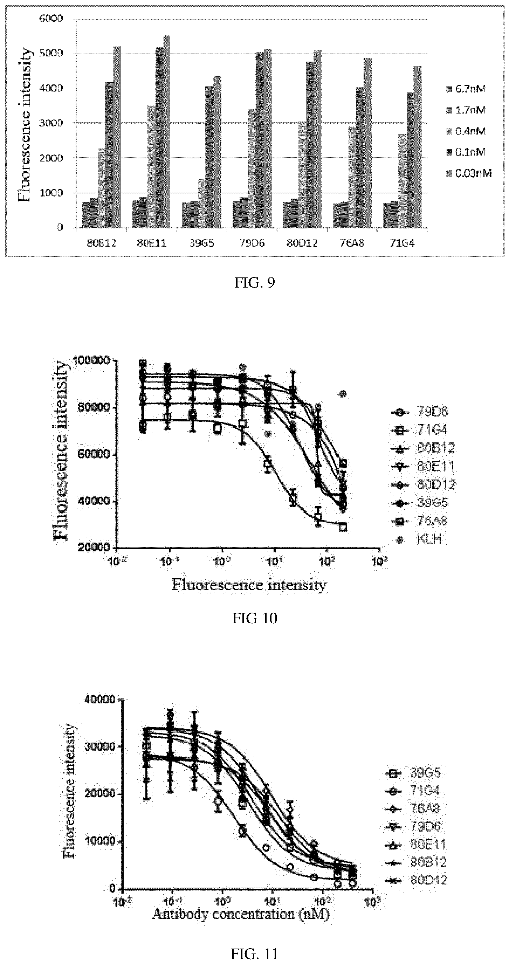

Ba/F3-hTSLPR-IL7Rα cells were washed three times with PBS, centrifuged at 300g for 3 minutes, and blocked with 3% BSA/PBS for 30 minutes. The cells were resuspended and added to a 96-well U-plate at 5×10⁴/well. The antibody was incubated with 60 ng/mL of biotinylated human TSLP protein at room temperature for 10 minutes, then added to the cells, the mixture was incubated at 4°C for 45 minutes and washed twice with 0.5% BSA/PBS, SA-PE was added, the mixture was incubated at 4°C for 45 minutes and washed twice with 0.5% BSA/PBS, PI dye was added, the mixture was incubated at 4°C for 10 minutes and washed twice with 0.5% BSA/PBS, the cells were resuspended in PBS, and the blocking effect of the antibody at the cellular level was detected by flow cytometry. FIG. 9 shows that each of the chimeric antibodies may block the binding of TSLP to cell surface TSLPR.

### Example 10 Inhibition of TSLP-stimulated proliferation of Ba/F3-hTSLPR-IL7Rα cells by chimeric antibodies

The assay for the inhibition of TSLP-induced Ba/F3-hTSLPR-IL7Rα cell proliferation by TSLP antibodies were performed in RPMI-1640 with 10% fetal bovine serum. Ba/F3-hTSLPR-IL7Rα cells were added to a 96-well cell culture plate one day in advance at a concentration of 2×10⁴ cells/well. 60 µL of the diluted anti-TSLP antibodies were pre-incubated with 60 µL of 5 ng/mL human TSLP for 15 minutes at room temperature. The pre-incubated antibody-cytokine was added to the cell culture plate at 100 µL/well. The 96-well plate was incubated in a 5% CO₂ incubator at 37°C for 72 hours. After the culture, CellTiter-Glo was added to each well for reaction 10 minutes. Luminescence values were read in a black 96-well plate. As shown in FIG. 10 and Table 5, each of the antibodies may inhibit cell proliferation, with 71G4 inhibition being the most significant.

**Table 5 Inhibition IC50 of cell proliferation by chimeric antibodies**

| | IC50 (nM) |
|---|---|
| 76A8 | Weak |
| 79D6 | Weak |
| 71G4 | 12.71 |
| 80B 12 | Weak |
| 80E11 | Weak |
| 80D12 | 44.69 |
| 39G5 | 39.13 |
| Isotype control KLH | - |

### Example 11 Inhibition of TSLP-activated STAT5 signaling by chimeric antibodies

Ba/F3-hTSLPR-IL7Rα-STAT5 luc cells were starved overnight, and the next day were plated onto a 96-well plate at 5×10⁴/well, with 50 µL per well; human TSLP was diluted and incubated at 37°C for 30 minutes in advance; 50µL of ligand/antibody mixture was pipetted to the cell plate for blending, and the mixture was incubated at 37°C for 6 hours; 100 µL of fluorescence detection reagent was added; the luminescence value of each well was read with a microplate reader. As shown in FIG. 11 and Table 6, each of the antibodies may inhibit the expression of reporter gene, with 71G4 inhibition being the most significant.

**Table 6 Inhibition IC50 of STAT5 reporter gene by chimeric antibodies**

| | IC50 (nM) |
|---|---|
| 76A8 | 9.78 |
| 79D6 | 9.61 |
| 71G4 | 1.67 |
| 80B 12 | 5.79 |
| 80E11 | 13.57 |
| 80D12 | 3.84 |
| 39G5 | 3.33 |

### Example 12 Humanization of antibodies

TSLP antibodies 39G5, 71G4, 76A8, 79D6, 80B12, 80D12, and 80E11 were derived from the same or similar mouse germline genes, and murine antibodies 39G5 and 71G4 with the strongest neutralization activity were selected for variable region sequence humanization.

Firstly, the sequence of murine antibody 39G5 was aligned with that of human antibody germline to find out the human germ-line light chain genes VK_1_39 and IGKJ1^{∗}01 and human germ-line heavy chain genes VH-1-69 and IGHJ4^{∗}01 which have good homology, are completely identical with the key amino acid sequences maintaining the antibody structure core (Upper hydrophobic core) and has high frequency of occurrence in the human body, and murine antibody CDR grafting was conducted; the source of murine anti -71G4 light and heavy chains was the same as that of 39G5, and only 1 or 2 amino acids of CDR 3 are different amino acid residues of the same type; therefore, CDR1 and CDR2 of 71G4 heavy chain are respectively substituted into humanized 39G5 heavy chain, and at the same time, 71G4 humanized light chain or 39G5 humanized light chain are respectively used for pairing expression with 39G5 humanized heavy chain to optimize the region surrounding the core binding site so as to improve the affinity and the distribution of molecular surface charge. Subsequently, in silico homology modeling was performed at the same time, and the framework amino acid sequence, molecular surface charge, and hydrophobic region distribution in the CDR region and its surrounding areas were analyzed, and different heavy chain derivatives and light chain derivatives were determined to obtain 27 humanized variants of TSLP antibodies: Ab1, Ab2, Ab3, Ab4, Ab5, Ab6, Ab7, Ab8, Ab9, Ab10, Ab11, Ab12, Ab13, Ab14, Ab15, Ab16, Ab17, Ab18, Ab19, Ab20, Ab21, Ab22, Ab23, Ab24, Ab25, Ab26, and Ab27. The heavy and light chains of these 27 humanized variants are shown in Table 7.

**Table 7 Humanization design of TSLP antibodies**

| Humanized antibody | Heavy chain | Light chain |
|---|---|---|
| Ab1 | h39G5VHv1 | h39G5VKv1 |
| Ab2 | h39G5VHv2 | h39G5VKv1 |
| Ab3 | h39G5VHv3 | h39G5VKv1 |
| Ab4 | h39G5VHv4 | h39G5VKv1 |
| Ab5 | h39G5VHv5 | h39G5VKv1 |
| Ab6 | h39G5VHv6 | h39G5VKv1 |
| Ab7 | h39G5VHv7 | h39G5VKv1 |
| Ab8 | h39G5VHv8 | h39G5VKv1 |
| Ab9 | h39G5VHv9 | h39G5VKv1 |
| Ab10 | h39G5VHv10 | h39G5VKv1 |
| Ab11 | h39G5VHv11 | h39G5VKv1 |
| Ab12 | h39G5VHv12 | h39G5VKv1 |
| Ab13 | h39G5VHv13 | h39G5VKv1 |
| Ab14 | h39G5VHv14 | h39G5VKv1 |
| Ab15 | h39G5VHv15 | h39G5VKv1 |
| Ab16 | h71G4VHv1 | h39G5VKv1 |
| Ab17 | h71G4VHv2 | h39G5VKv1 |
| Ab18 | h71G4VHv3 | h39G5VKv1 |
| Ab19 | h71G4VHv1 | h71G4VKv1 |
| Ab20 | h71G4VHv2 | h71G4VKv1 |
| Ab21 | h71G4VHv3 | h71G4VKv1 |
| Ab22 | h39G5VHv1 | h71G4VKv1 |
| Ab23 | h39G5VHv2 | h71G4VKv1 |
| Ab24 | h39G5VHv5 | h71G4VKv1 |
| Ab25 | h39G5VHv6 | h71G4VKv1 |
| Ab26 | h39G5VHv10 | h71G4VKv1 |
| Ab27 | h39G5VHv11 | h71G4VKv1 |
| | | |
| | | |

Full sequence of the light and heavy chain derivatives were synthesized, respectively, and cloned into vectors containing the constant region Ckappa of the antibody kappa chain or the constant region CH1-CH3 of human IgG1. The light and heavy chain derivative plasmids were paired in combination, and transfected into HEK293.6E cells for expression for 5-6 days, and the supernatant was collected for Protein A column purification.

The humanized antibody heavy/light chain variable region sequences are as follows:
h39G5VH v1: SEQ ID Nos:77-81 
<-------------FR1-------------> CDR1 <----FR2-----> CDR2 <--
QVQLVQSGAEVKKPGSSVKVSCKASGYSFISYWIHWVRQAPGQGLEWMGIIDPSDSDTSLNQKFQGRVT
-----------FR3-------------> CDR3 <---FR4--->
LTVDKSSTTAYMELSSLRSEDTAVYYCARSLDGYYDYWGQGTLVTVSS
Nucleotide sequence 
CAGGTTCAGCTGGTTCAGTCTGGCGCCGAAGTGAAGAAACCTGGCAGCAGCGTGAAGGTGTCCTGCAAG
GCTAGCGGCTACAGCTTCATCAGCTACTGGATCCACTGGGTCCGACAGGCCCCTGGACAAGGACTTGAG
TGGATGGGCATCATCGACCCCAGCGACAGCGACACAAGCCTGAACCAGAAATTCCAGGGCAGAGTGACC
ATCACCGCCGACGAGTCTAcCAGCACCGCCTACATgGAACTGAGCAGCCTGAGAAGCGAGGACACCGCC
GTGTACTACTGTGCCAGATCTCTGGACGGCTACTACGATTACTGGGGCCAGGGAACCCTGGTCACCGTT
TCTTCT
h39G5VH v2: SEQ ID Nos: 82-86 
<-------------FR1-----------> CDR1 <----FR2-----> CDR2 <--
QVQLVQSGAEVKKPGSSVKVSCKASGYSFISYWIHWVRQAPGQGLEWMGIIDPSDSDTSLNQKFQGRVT
-----------FR3---------------> CDR3 <---FR4--->
ITADESTSTAYMELSSLRSEDTAVYYCARSLDGYYDYWGQGTLVTVSS
Nucleotide sequence 
CAGGTTCAGCTGGTTCAGTCTGGCGCCGAAGTGAAGAAACCTGGCAGCAGCGTGAAGGTGTCCTGCAAG
GCTAGCGGCTACAGCTTCATCAGCTACTGGATCCACTGGGTCCGACAGGCCCCTGGACAAGGACTTGAG
TGGATGGGCATCATCGACCCCAGCGACAGCGACACAAGCCTGAACCAGAAATTCCAGGGCAGAGTGACC
ATCACCGCCGACGAGTCTAcCAGCACCGCCTACATgGAACTGAGCAGCCTGAGAAGCGAGGACACCGCC
GTGTACTACTGTGCCAGATCTCTGGACGGCTACTACGATTACTGGGGCCAGGGAACCCTGGTCACCGTT
TCTTCT
h39G5VH v3: SEQ ID Nos: 87-91 
<-------------FR1-----------> CDR1 <----FR2-----> CDR2 <--
EVQLVQSGAEVKKPGESLRISCKASGYSFISYWIHWVRQMPGKGLEWMGIIDPSDSDTSLNQSFQGHVT
-----------FR3--------------> CDR3 <---FR4--->
LSVDKSSTTAYLQWSSLKASDTAMYYCARSLDGYYDYWGQGTLVTVSS
Nucleotide sequence 
GAAGTGCAGCTGGTTCAGTCTGGCGCCGAAGTGAAGAAGCCTGGCGAGAGCCTGAGAATCAGCTGCAAG
GCCAGCGGCTACAGCTTCATCAGCTACTGGATCCACTGGGTCCGACAGATGCCTGGCAAAGGCCTGGAA
TGGATGGGCATCATCGACCCCAGCGACAGCGACACAAGCCTGAACCAGTCTTTCCAGGGCCACGTGACA
CTGAGCGTGGACAAGTCTAGCACCACCGCCTACCTGCAGTGGTCTAGCCTGAAGGCCTCTGACACCGCC
ATGTACTACTGCGCCAGATCTCTGGACGGCTACTACGATTACTGGGGCCAGGGAACCCTGGTCACCGTT
TCTTCT
h39G5VH v4: SEQ ID Nos: 92-96 
<-------------FR1-----------> CDR1 <----FR2-----> CDR2 <--
EVQLVQSGAEVKKPGESLRISCKASGYSFISYWIHWVRQMPGKGLEWMGIIDPSDSDTSLNQSFQGHVT
-----------FR3--------------> CDR3 <---FR4--->
ISADKSISTAYLQWSSLKASDTAMYYCARSLDGYYDYWGQGTLVTVSS
Nucleotide sequence 
GAAGTGCAGCTGGTTCAGTCTGGCGCCGAAGTGAAGAAGCCTGGCGAGAGCCTGAGAATCAGCTGCAAG
GCCAGCGGCTACAGCTTCATCAGCTACTGGATCCACTGGGTCCGACAGATGCCTGGCAAAGGCCTGGAA
TGGATGGGCATCATCGACCCCAGCGACAGCGACACAAGCCTGAACCAGTCTTTCCAGGGCCACGTGACA
ATCAGCGCCGACAAGAGCATCAGCACCGCCTACCTGCAGTGGTCTAGCCTGAAGGCCTCTGACACCGCC
ATGTACTACTGCGCCAGATCTCTGGACGGCTACTACGACTACTGGGGCCAGGGAACACTGGTCACCGTT
AGCTCT
h39G5VH v5: SEQ ID Nos: 97-101 
<-------------FR1-----------> CDR1 <----FR2-----> CDR2 <--
QVQLVQSGAEVKKPGSSVKVSCKASGYTFTDYWMHWVRQAPGQGLEWMGIIDPSDSDTSLNQKFQGRVT
-----------FR3---------------> CDR3 <---FR4--->
LTVDTSTSTAYMELSSLRSEDTAVYYCARSLDGYYDYWGQGTLVTVSS
Nucleotide sequence 
CAGGTTCAGCTGGTTCAGTCTGGCGCCGAAGTGAAGAAACCTGGCAGCAGCGTGAAGGTGTCCTGCAAG
GCTAGCGGCTACACATTCACCGACTACTGGATGCACTGGGTCCGACAGGCTCCAGGACAGGGACTTGAG
TGGATGGGCATCATCGACCCCAGCGACAGCGACACAAGCCTGAACCAGAAATTCCAGGGCAGAGTGACC
CTGACCGTGGACACCTCTACAAGCACCGCCTACATGGAACTGAGCAGCCTGAGAAGCGAGGACACCGCC
GTGTACTACTGTGCCAGATCTCTGGACGGCTACTACGATTACTGGGGCCAGGGAACCCTGGTCACCGTT
TCTTCT
h39G5VH v6: SEQ ID Nos: 102-106 
<-------------FR1-----------> CDR1 <----FR2-----> CDR2 <--
QVQLVQSGAEVKKPGSSVKVSCKASGYTFTDYWMHWVRQAPGQGLEWMGTIDNSDSDTSLNQKFQGRVT
-----------FR3--------------> CDR3 <---FR4--->
LTVDTSTSTAYMELSSLRSEDTAVYYCARSLDGYYDYWGQGTLVTVSS
Nucleotide sequence 
CAGGTTCAGCTGGTTCAGTCTGGCGCCGAAGTGAAGAAACCTGGCAGCAGCGTGAAGGTGTCCTGCAAG
GCTAGCGGCTACACATTCACCGACTACTGGATGCACTGGGTCCGACAGGCTCCAGGACAGGGACTTGAG
TGGATGGGCACCATCGACAACAGCGACAGCGACACAAGCCTGAACCAGAAATTCCAGGGCAGAGTGACC
CTGACCGTGGACACCTCTACAAGCACCGCCTACATGGAACTGAGCAGCCTGAGAAGCGAGGACACCGCC
GTGTACTACTGTGCCAGATCTCTGGACGGCTACTACGATTACTGGGGCCAGGGAACCCTGGTCACCGTT
TCTTCT
h39G5VH v7: SEQ ID Nos: 107-111 
<-------------FR1-----------> CDR1 <----FR2-----> CDR2 <--
QVQLVQSGAEVKKPGSSVKVSCKASGYTFTDYWMHWVRQAPGQGLEWMGTIDNSDSDTSLNQKFQGRVT
-----------FR3--------------> CDR3 <---FR4--->
LTVDESSSTAYMELSSLRSEDTAVYYCARSLDGYYDYWGQGTLVTVSS
Nucleotide sequence 
CAGGTTCAGCTGGTTCAGTCTGGCGCCGAAGTGAAGAAACCTGGCAGCAGCGTGAAGGTGTCCTGCAAG
GCTAGCGGCTACACATTCACCGACTACTGGATGCACTGGGTCCGACAGGCTCCAGGACAGGGACTTGAG
TGGATGGGCACCATCGACAACAGCGACAGCGACACAAGCCTGAACCAGAAATTCCAGGGCAGAGTGACC
CTGACCGTGGACACCTCTACAAGCACCGCCTACATGGAACTGAGCAGCCTGAGAAGCGAGGACACCGCC
GTGTACTACTGTGCCAGATCTCTGGACGGCTACTACGATTACTGGGGCCAGGGAACCCTGGTCACCGTT
TCTTCT
h39G5VH v8: SEQ ID Nos: 112-116 
<-------------FR1-----------> CDR1 <----FR2-----> CDR2 <--
EVQLVQSGAEVKKPGESLKISCKASGYTFTDYWMHWVRQMPGKGLEWMGIIDPSDSDTSLNQSFQGHVT
-----------FR3---------------> CDR3 <---FR4--->
LSVDKSSTTAYLQWSSLKASDTAMYYCARSLDGYYDYWGQGTLVTVSS
Nucleotide sequence 
GAAGTGCAGCTGGTTCAGTCTGGCGCCGAAGTGAAGAAGCCTGGCGAGAGCCTGAAGATCAGCTGCAAG
GCCAGCGGCTACACCTTCACCGACTACTGGATGCACTGGGTCCGACAGATGCCTGGCAAAGGCCTGGAA
TGGATGGGCATCATCGACCCCAGCGACAGCGACACAAGCCTGAACCAGTCTTTCCAGGGCCACGTGACA
CTGAGCGTGGACAAGTCTAGCACCACCGCCTACCTGCAGTGGTCTAGCCTGAAGGCCTCTGACACCGCC
ATGTACTACTGCGCCAGATCTCTGGACGGCTACTACGATTACTGGGGCCAGGGAACCCTGGTCACCGTT
TCTTCT
h39G5VH v9: SEQ ID Nos: 117-121 
<-------------FR1-----------> CDR1 <----FR2-----> CDR2 <--
EVQLVQSGAEVKKPGESLKISCKASGYTFTDYWMHWVRQMPGKGLEWMGTIDNSDSDTSLNQSFQGHVT
-----------FR3--------------> CDR3 <---FR4--->
LSVDKSSTTAYLQWSSLKASDTAMYYCARSLDGYYDYWGQGTLVTVSS
Nucleotide sequence 
GAAGTGCAGCTGGTTCAGTCTGGCGCCGAAGTGAAGAAGCCTGGCGAGAGCCTGAAGATCAGCTGCAAG
GCCAGCGGCTACACCTTCACCGACTACTGGATGCACTGGGTCCGACAGATGCCTGGCAAAGGCCTGGAA
TGGATGGGCACCATCGACAACAGCGACAGCGACACAAGCCTGAACCAGAGCTTTCAGGGCCACGTGACA
CTGAGCGTGGACAAGTCTAGCACCACCGCCTACCTGCAGTGGTCTAGCCTGAAGGCCTCTGACACCGCC
ATGTACTACTGCGCCAGATCTCTGGACGGCTACTACGATTACTGGGGCCAGGGAACCCTGGTCACCGTT
TCTTCT
h39G5VH v10: SEQ ID Nos: 122-126 
<-------------FR1-----------> CDR1 <----FR2-----> CDR2 <--
QVQLVQSGAEVKKPGSSVKVSCKASGYTFTDYWMHWVRQAPGQGLEWMGIIDPSDSDTSLNQKFQGRVT
-----------FR3--------------> CDR3 <---FR4--->
ITADTSTSTAYMELSSLRSEDTAVYYCARSLDGYYDYWGQGTLVTVSS
Nucleotide sequence 
CAGGTTCAGCTGGTTCAGTCTGGCGCCGAAGTGAAGAAACCTGGCAGCAGCGTGAAGGTGTCCTGCAAG
GCTAGCGGCTACACATTCACCGACTACTGGATGCACTGGGTCCGACAGGCTCCAGGACAGGGACTTGAG
TGGATGGGCATCATCGACCCCAGCGACAGCGACACAAGCCTGAACCAGAAATTCCAGGGCAGAGTGACC
ATCACCGCCGACACCTCTACAAGCACCGCCTACATGGAACTGAGCAGCCTGAGAAGCGAGGACACCGCC
GTGTACTACTGTGCCAGATCTCTGGACGGCTACTACGATTACTGGGGCCAGGGAACCCTGGTCACCGTT
TCTTCT
h39G5VH v11: SEQ ID Nos: 127-131 
<-------------FR1-----------> CDR1 <----FR2-----> CDR2 <--
QVQLVQSGAEVKKPGSSVKVSCKASGYTFTDYWMHWVRQAPGQGLEWMGTIDNSDSDTSLNQKFQGRVT
-----------FR3--------------> CDR3 <---FR4--->
ITADTSTSTAYMELSSLRSEDTAVYYCARSLDGYYDYWGQGTLVTVSS
Nucleotide sequence 
CAGGTTCAGCTGGTTCAGTCTGGCGCCGAAGTGAAGAAACCTGGCAGCAGCGTGAAGGTGTCCTGCAAG
GCTAGCGGCTACACATTCACCGACTACTGGATGCACTGGGTCCGACAGGCTCCAGGACAGGGACTTGAG
TGGATGGGCACCATCGACAACAGCGACAGCGACACAAGCCTGAACCAGAAATTCCAGGGCAGAGTGACC
ATCACCGCCGACACCTCTACAAGCACCGCCTACATGGAACTGAGCAGCCTGAGAAGCGAGGACACCGCC
GTGTACTACTGTGCCAGATCTCTGGACGGCTACTACGATTACTGGGGCCAGGGAACCCTGGTCACCGTT
TCTTCT
h39G5VH v12: SEQ ID Nos: 132-136 
<-------------FR1-----------> CDR1 <----FR2-----> CDR2 <--
QVQLVQSGAEVKKPGSSVKVSCKASGYTFTDYWMHWVRQAPGQGLEWMGTIDNSDSDTSLNQKFQGRVT
-----------FR3--------------> CDR3 <---FR4--->
ITADESSSTAYMELSSLRSEDTAVYYCARSLDGYYDYWGQGTLVTVSS
Nucleotide sequence 
CAGGTTCAGCTGGTTCAGTCTGGCGCCGAAGTGAAGAAACCTGGCAGCAGCGTGAAGGTGTCCTGCAAG
GCTAGCGGCTACACATTCACCGACTACTGGATGCACTGGGTCCGACAGGCTCCAGGACAGGGACTTGAG
TGGATGGGCACCATCGACAACAGCGACAGCGACACAAGCCTGAACCAGAAATTCCAGGGCAGAGTGACC
ATCACCGCCGACGAGTCTAGCAGCACCGCCTACATGGAACTGAGCAGCCTGAGAAGCGAGGACACCGCC
GTGTACTACTGTGCCAGATCTCTGGACGGCTACTACGATTACTGGGGCCAGGGAACCCTGGTCACCGTT
TCTTCT
h39G5VH v13: SEQ ID Nos: 137-141 
<-------------FR1-----------> CDR1 <----FR2-----> CDR2 <--
EVQLVQSGAEVKKPGESLKISCKASGYTFTDYWMHWVRQMPGKGLEWMGIIDPSDSDTSLNQSFQGHVT
-----------FR3---------------> CDR3 <---FR4--->
ISADKSISTAYLQWSSLKASDTAMYYCARSLDGYYDYWGQGTLVTVSS
Nucleotide sequence 
GAAGTGCAGCTGGTTCAGTCTGGCGCCGAAGTGAAGAAGCCTGGCGAGAGCCTGAAGATCAGCTGCAAG
GCCAGCGGCTACACCTTCACCGACTACTGGATGCACTGGGTCCGACAGATGCCTGGCAAAGGCCTGGAA
TGGATGGGCATCATCGACCCCAGCGACAGCGACACAAGCCTGAACCAGTCTTTCCAGGGCCACGTGACA
ATCAGCGCCGACAAGAGCATCAGCACCGCCTACCTGCAGTGGTCTAGCCTGAAGGCCTCTGACACCGCC
ATGTACTACTGCGCCAGATCTCTGGACGGCTACTACGACTACTGGGGCCAGGGAACACTGGTCACCGTT
AGCTCT
h39G5VH v14: SEQ ID Nos: 142-146 
<-------------FR1-----------> CDR1 <----FR2-----> CDR2 <--
EVQLVQSGAEVKKPGESLKISCKASGYTFTDYWMHWVRQMPGKGLEWMGTIDNSDSDTSLNQSFQGHVT
-----------FR3--------------> CDR3 <---FR4--->
ISADKSISTAYLQWSSLKASDTAMYYCARSLDGYYDYWGQGTLVTVSS
Nucleotide sequence 
GAAGTGCAGCTGGTTCAGTCTGGCGCCGAAGTGAAGAAGCCTGGCGAGAGCCTGAAGATCAGCTGCAAG
GCCAGCGGCTACACCTTCACCGACTACTGGATGCACTGGGTCCGACAGATGCCTGGCAAAGGCCTGGAA
TGGATGGGCACCATCGACAACAGCGACAGCGACACAAGCCTGAACCAGAGCTTTCAGGGCCACGTGACA
ATCAGCGCCGACAAGTCTATCAGCACCGCCTACCTGCAGTGGTCTAGCCTGAAGGCCTCTGACACCGCC
ATGTACTACTGCGCCAGATCTCTGGACGGCTACTACGACTACTGGGGCCAGGGAACACTGGTCACCGTT
AGCTCT
h39G5VH v15: SEQ ID Nos: 147-151 
<-------------FR1-----------> CDR1 <----FR2-----> CDR2 <--
EVQLVQSGAEVKKPGESLKISCKASGYTFTDYWMHWVRQMPGKGLEWMGTIDNSDSDTSLNQSFQGHVT
-----------FR3--------------> CDR3 <---FR4--->
ISADKSSTTAYLQWSSLKASDTAMYYCARSLDGYYDYWGQGTLVTVSS
Nucleotide sequence 
GAAGTGCAGCTGGTTCAGTCTGGCGCCGAAGTGAAGAAGCCTGGCGAGAGCCTGAAGATCAGCTGCAAG
GCCAGCGGCTACACCTTCACCGACTACTGGATGCACTGGGTCCGACAGATGCCTGGCAAAGGCCTGGAA
TGGATGGGCACCATCGACAACAGCGACAGCGACACAAGCCTGAACCAGAGCTTTCAGGGCCACGTGACA
ATCAGCGCCGACAAGTCTAGCACCACCGCCTACCTGCAGTGGTCTAGCCTGAAGGCCTCTGACACCGCC
ATGTACTACTGCGCCAGATCTCTGGACGGCTACTACGATTACTGGGGCCAGGGAACCCTGGTCACCGTT
TCTTCT
h71G4VH v1: SEQ ID Nos: 152-156 
<-----------FR1----------> CDR1 <----FR2-----> CDR2 <--
QVQLVQSGAEVKKPGSSVKVSCKASGYTFTDYWMHWVRQAPGQGLEWMGTIDNSDSDTTYNPSFQGHVT
-----------FR3--------------> CDR3 <---FR4--->
ISADKSISTAYLQWSSLKASDTAMYYCARSVDGYLDYWGQGTLVTVSS
Nucleotide sequence 
CAGGTTCAGCTGGTTCAGTCTGGCGCCGAAGTGAAGAAACCTGGCAGCAGCGTGAAGGTGTCCTGCAAG
GCTAGCGGCTACACATTCACCGACTACTGGATGCACTGGGTCCGACAGGCTCCAGGACAGGGACTTGAG
TGGATGGGCACCATCGACAACAGCGACAGCGACACAACCTACAACCcaTCTTTCCAGGGCCACGTGACA
ATCAGCGCCGACAAGAGCATCAGCACCGCCTACCTGCAGTGGTCTAGCCTGAAGGCCTCTGACACCGCC
ATGTACTACTGTGCCAGATCTGTGGACGGCTACCTGGATTACTGGGGCCAGGGAACCCTGGTCACCGTT
TCTTCT
h71G4VH v2: SEQ ID Nos: 157-161 
<-------------FR1-----------> CDR1 <----FR2-----> CDR2 <--
QVQLVQSGAEVKKPGSSVKVSCKASGYTFTDYWMHWVRQAPGQGLEWMGTIDNSDSDTTYNQKFQGRVT
-----------FR3--------------> CDR3 <---FR4--->
LTVDTSTSTAYMELSSLRSEDTAVYYCARSVDGYLDYWGQGTLVTVSS
Nucleotide sequence 
CAGGTTCAGCTGGTTCAGTCTGGCGCCGAAGTGAAGAAACCTGGCAGCAGCGTGAAGGTGTCCTGCAAG
GCTAGCGGCTACACATTCACCGACTACTGGATGCACTGGGTCCGACAGGCTCCAGGACAGGGACTTGAG
TGGATGGGCACCATCGACAACAGCGACAGCGACACAACCTACAACCAGAAATTCCAGGGCAGAGTGACC
CTGACCGTGGACACCTCTACAAGCACCGCCTACATGGAACTGAGCAGCCTGAGAAGCGAGGACACCGCC
GTGTACTACTGTGCCAGATCTGTGGACGGCTACCTGGATTACTGGGGCCAGGGAACCCTGGTCACCGTT
TCTTCT
h71G4VH v3: SEQ ID Nos: 162-166 
<-------------FR1-----------> CDR1 <----FR2-----> CDR2 <--
QVQLVQSGAEVKKPGSSVKVSCKASGYTFTDYWMHWVRQAPGQGLEWMGTIDNSDSDTTYNQKFQGRVT
-----------FR3---------------> CDR3 <---FR4--->
MTRDTSTSTVYMELSSLRSEDTAVYYCARSVDGYLDYWGQGTLVTVSS
Nucleotide sequence 
CAGGTTCAGCTGGTTCAGTCTGGCGCCGAAGTGAAGAAACCTGGCAGCAGCGTGAAGGTGTCCTGCAAG
GCTAGCGGCTACACATTCACCGACTACTGGATGCACTGGGTCCGACAGGCTCCAGGACAGGGACTTGAG
TGGATGGGCACCATCGACAACAGCGACAGCGACACAACCTACAACCAGAAATTCCAGGGCAGAGTGACC
aTGACCagaGACACCTCTACAAGCACCGtCTACATGGAACTGAGCAGCCTGAGAAGCGAGGACACCGCC
GTGTACTACTGTGCCAGATCTGTGGACGGCTACCTGGATTACTGGGGCCAGGGAACCCTGGTCACCGTT
TCTTCT
h39G5VK v1: SEQ ID Nos: 167-171 
<-----------FR1--------->   CDR1    <-----FR2----->  CDR2 <-----------
DIQMTQSPSSLSASVGDRVTITCRASENIYSYLAWYQQKPGKAPKLLIYNARTLPEGVPSRFSGSGSGT
-----FR3-----------> CDR3 <---FR4--->
DFTLTISSLQPEDFATYYCQHHYTTPWTFGQGTKVEIK
Nucleotide sequence 
GACATCCAGATGACACAGAGCCCTAGCAGCCTGTCTGCCAGCGTGGGAGACAGAGTGACCATCACCTGT
AGAGCCAGCGAGAACATCTACAGCTACCTGGCCTGGTATCAGCAGAAGCCTGGCAAGGCTCCCAAGCTG
CTGATCTACAACGCCAGAACACTGCCTGAGGGCGTGCCCTCTAGATTCAGCGGATCTGGCTCTGGCACC
GACTTCACCCTGACAATCTCTAGCCTGCAGCCTGAGGACTTCGCCACCTACTACTGCCAGCACCACTAC
ACCACACCTTGGACATTCGGCCAGGGCACCAAGGTGGAAATCAAG
h71G4VK v1: SEQ ID Nos: 172-176 
<-----------FR1--------> CDR1 <-----FR2-----> CDR2 <-----------
DIQMTQSPSSLSASVGDRVTITCRTSENIYSYLAWYQQKPGKAPKLLIYFAKTLTDGVPSRFSGSGSGT
-----FR3-----------> CDR3 <---FR4--->
DFTLTISSLQPEDFATYYCQHHYGTPWTFGQGTKVEIK
Nucleotide sequence

GACATCCAGATGACACAGAGCCCTAGCAGCCTGTCTGCCAGCGTGGGAGACAGAGTGACCATCACCTGT
AGAaCCAGCGAGAACATCTACAGCTACCTGGCCTGGTATCAGCAGAAGCCTGGCAAGGCTCCCAAGCTG
CTGATCTACttCGCCAagACACTGaccGAcGGCGTGCCCTCTAGATTCAGCGGATCTGGCTCTGGCACC
GACTTCACCCTGACAATCTCTAGCCTGCAGCCTGAGGACTTCGCCACCTACTACTGCCAGCACCACTAC
ggCACACCTTGGACATTCGGCCAGGGCACCAAGGTGGAAATCAAG

### Example 13 Determination of affinity for humanized antibodies

### 1) Binding of humanized antibodies to human TSLP

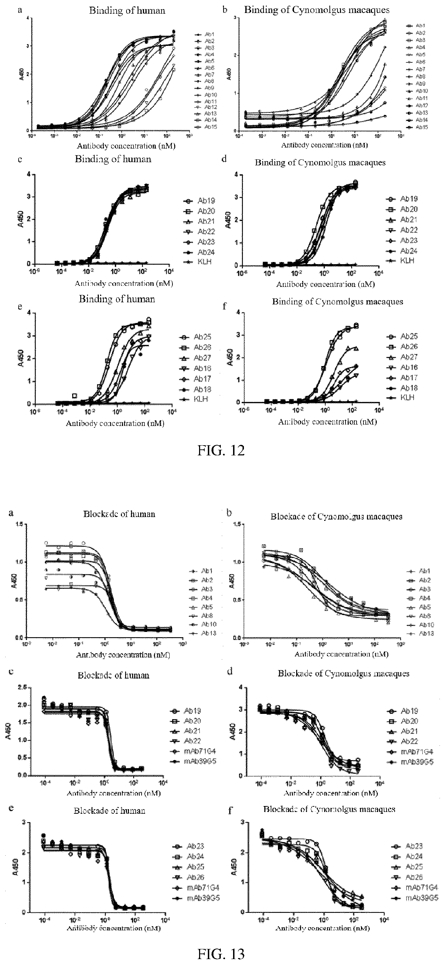

Human TSLP recombinant protein was coated onto a 96-well microtiter plate at 1 µg/mL in carbonate buffer overnight at 4°C. The plate was washed three times with PBS and blocked by adding 200 µL/well of PBS containing 2% skim milk for 1 hour. After washing once with PBS, 100 µL of anti-TSLP antibodies were added to each well and incubated at room temperature for 60 minutes; after washing with PBST and PBS three times, respectively, 100 µL of HRP-labeled anti-human IgG Fc secondary antibody was added to each well, and the mixture was incubated at room temperature for 60 minutes. After washing with PBST and PBS three times, respectively, 100 µL of TMB substrate was added to each well for color development at 37°C for 10 minutes, the reaction was terminated with 50 µL of 2M sulfuric acid solution per well, and the absorbance was read at a wavelength of 450 nm. FIGs. 12-a, c, and e and Table 8 show that each of the humanized antibodies obtained in Example 12 bound to human TSLP, with Ab20 having the strongest binding activity.

### 2) Binding of humanized antibodies to Cynomolgus macaques TSLP

Cynomolgus macaques TSLP recombinant protein was coated onto a 96-well microtiter plate at 1 µg/mL in carbonate buffer overnight at 4°C. The plate was washed three times with PBS and blocked by adding 200 µL/well of PBS containing 2% skim milk for 1 hour. After washing once with PBS, 100 µL of anti-TSLP antibodies were added to each well and incubated at room temperature for 60 minutes; after washing with PBST and PBS three times, respectively, 100 µL of HRP-labeled anti-human IgG Fc secondary antibody was added to each well, and the mixture was incubated at room temperature for 60 minutes. After washing with PBST and PBS three times, respectively, 100 µL of TMB substrate was added to each well for color development at 37°C for 10 minutes, the reaction was terminated with 50 µL of 2M sulfuric acid solution per well, and the absorbance was read at a wavelength of 450 nm. FIGs. 12-b, d, and f and Table 8 show that all antibodies except Ab14 and Ab15 bind to Cynomolgus macaques TSLP, with Ab20 having the strongest binding activity.

**Table 8 Detection the binding of humanized antibodies to Cynomolgus macaques TSLP by ELISA**

| Humanized antibody | Heavy chain/light chain | Human TSLP EC50 (nM) | Cynomolgus macaques TSLP EC50 (nM) |
|---|---|---|---|
| Ab1 | h39G5VHv1/h39G5VKv1 | 0.19 | 2.29 |
| Ab2 | h39G5VHv2/h39G5VKv1 | 0.21 | 2.53 |
| Ab3 | h39G5VHv3/h39G5VKv1 | 0.22 | 2.22 |
| Ab4 | h39G5VHv4/h39G5VKv1 | 0.39 | 5.54 |
| Ab5 | h39G5VHv5/h39G5VKv1 | 0.26 | 2.85 |
| Ab6 | h39G5VHv6/h39G5VKv1 | 2.79 | Weak |
| Ab7 | h39G5VHv7/h39G5VKv1 | 1.89 | Weak |
| Ab8 | h39G5VHv8/h39G5VKv1 | 0.4 | 5.51 |
| Ab9 | h39G5VHv9/h39G5VKv1 | 3.03 | 208.8 |
| Ab10 | h39G5VHv10/h39G5VKv1 | 0.32 | 2.238 |
| Ab11 | h39G5VHv11/h39G5VKv1 | 35.98 | Weak |
| Ab12 | h39G5VHv12/h39G5VKv1 | Weak | Weak |
| Ab13 | h39G5VHv13/h39G5VKv1 | 0.66 | 5.68 |
| Ab14 | h39G5VHv14/h39G5VKv1 | Weak | - |
| Ab15 | h39G5VHv15/h39G5VKv1 | 62.92 | - |
| Ab16 | h71G4VHv1/h39G5VKv1 | 5.87 | 12.64 |
| Ab17 | h71G4VHv2/h39G5VKv1 | 1.98 | 4.44 |
| Ab18 | h71G4VHv3/h39G5VKv1 | 2.15 | 17.86 |
| Ab19 | h71G4VHv1/h71G4VKv1 | 0.27 | 0.65 |
| Ab20 | h71G4VHv2/h71G4VKv1 | 0.17 | 0.27 |
| Ab21 | h71G4VHv3/h71G4VKv1 | 0.28 | 0.54 |
| Ab22 | h39G5VHv1/h71G4VKv1 | 0.29 | 0.77 |
| Ab23 | h39G5VHv2/h71G4VKv1 | 0.24 | 0.83 |
| Ab24 | h39G5VHv5/h71G4VKv1 | 0.22 | 1.37 |
| Ab25 | h39G5VHv6/h71G4VKv1 | 0.27 | 1.16 |
| Ab26 | h39G5VHv10/h71G4VKv1 | 0.18 | 0.95 |
| Ab27 | h39G5VHv11/h71G4VKv1 | 1.08 | 4.22 |

### 3) Affinity assay by BIAcore

The kinetic binding activity of anti-TSLP antibodies to human and Cynomolgus macaques TSLP was measured by surface plasmon resonance (SPR) using the BIAcore T200 system. Approximately 100 RU of anti-TSLP antibody was immobilized on the sensor chip Protein A using 1×HBS-EP buffer as running buffer at a flow rate of 10 µL/min. Various concentrations of human or Cynomolgus macaques TSLP ranging from 0 to 21.7 nM were injected onto the surface immobilized anti-TSLP antibody at a flow rate of 30 µL/min. After each injection cycle, the Protein A chip surface was regenerated with regeneration buffer 10 mM glycine (pH 2.0) at a flow rate of 30 µL/min. The association rate constant (ka) and dissociation rate constant (kd) and equilibrium dissociation constant KD were analyzed by fitting the resulting data to a Langmuir 1:1 kinetics theoretical model using BIAevaluation software.

Table 9 shows that each antibody has a higher affinity for both human and Cynomolgus macaques TSLP, with antibodies Ab22, Ab23, Ab24, and Ab26 having a higher affinity for human TSLP of 0.06 pM, 0.08 pM, 0.02 pM, and 2 pM, respectively, and for Cynomolgus macaques TSLP of 0.04 pM, 0.007 pM, 0.18 pM and 0.31 pM, respectively.

**Table 9 Affinity for humanized antibodies**

| | Affinity of human TSLP | | | Affinity of Cynomolgus macaques TSLP | | |
|---|---|---|---|---|---|---|
| | ka (1/Ms) | kd (1/s) | KD(M) | ka (1/Ms) | kd (1/s) | KD(M) |
| h39G5VHv1/h7IG4VKv1 hIgG1 | 1.68E+06 | 1.07E-07 | 6.40E-14 | 1.59E+06 | 6.70E-08 | 4.21E-14 |
| h39G5VHv2/h71G4VKv1 hIgG1 | 1.98E+06 | 1.61E-07 | 8.12E-14 | 1.93E+06 | 1.36E-08 | 7.08E-15 |
| h39G5VHv5/h71G4VKv1 hIgG1 | 1.70E+06 | 3.99E-08 | 2.34E-14 | 1.59E+06 | 2.85E-07 | 1.79E-13 |
| h39G5VHv10/h71G4VKv1 hIgG1 | 1.37E+06 | 2.79E-06 | 2.03E-12 | 1.24E+06 | 3.82E-07 | 3.08E-13 |

### Example 14 Blockade at ELISA levels

### 1) Blockade of the binding to human TSLP to human TSLPR by humanized antibodies

Human TSLPR recombinant protein was coated onto a 96-well microtiter plate at 1 µg/mL in carbonate buffer overnight at 4°C. The plate was washed three times with PBS and blocked by adding 200 µL/well of PBS containing 2% skim milk for 1 hour. Anti-TSLP antibodies were incubated with 60 ng/mL biotinylated human TSLP protein in a blocked U-shaped 96-well plate for 30 minutes at room temperature. The 96-well plate was washed three times with PBS, and 100 µL of the incubated mixture was added to each well and incubated for 1 hour at room temperature. After washing with PBST and PBS three times, respectively, 100 µL of SA-HRP was added to each well and incubated at room temperature for 30 minutes in the dark. After washing with PBST and PBS three times, respectively, 100 µL of TMB substrate was added to well for color development at 37°C for 10 minutes, the reaction was terminated with 50 µL of 2M sulfuric acid solution per well, and the absorbance was read at a wavelength of 450 nm. FIGs. 13-a, c, and e and Table 10 show that all of the humanized antibodies Ab1, Ab2, Ab3, Ab4, Ab5, Ab8, Ab10, Ab13, Ab19, Ab20, Ab21, Ab22, Ab23, Ab24, Ab25, and Ab26 block the binding of human TSLP to TSLPR.

### 2) Blockade of the binding of Cynomolgus macaques TSLP to Cynomolgus macaques TSLPR by humanized antibodies

Cynomolgus macaques TSLPR recombinant protein was coated onto a 96-well microtiter plate at 1 µg/mL in carbonate buffer overnight at 4°C. The plate was washed three times with PBS and blocked by adding 200 µL/well of PBS containing 2% skim milk for 1 hour. Anti-TSLP antibodies were incubated with 60 ng/mL biotinylated Cynomolgus macaques TSLP protein in a blocked U-shaped 96-well plate for 30 minutes at room temperature. The 96-well plate was washed three times with PBS, and 100 µL of the incubated mixture was added to each well and incubated for 1 hour at room temperature. After washing with PBST and PBS three times, respectively, 100 µL of SA-HRP was added to each well and incubated at room temperature for 30 minutes in the dark. After washing with PBST and PBS three times, respectively, 100 µL of TMB substrate was added to well for color development at 37°C for 10 minutes, the reaction was terminated with 50 µL of 2M sulfuric acid solution per well, and the absorbance was read at a wavelength of 450 nm. FIGs. 13-b, d, and f and Table 10 show that all of the humanized antibodies Ab1, Ab2, Ab3, Ab4, Ab5, Ab8, Ab10, Ab13, Ab19, Ab20, Ab21, Ab22, Ab23, Ab24, Ab25, and Ab26 block the binding of Cynomolgus macaques TSLP to TSLPR.

**Table 10 Blockade of the binding of TSLP to TSLPR by humanized antibodies**

| Humanized antibody | Heavy chain/light chain | Human TSLP IC50 (nM) | Cynomolgus macaques TSLP IC50 (nM) |
|---|---|---|---|
| Ab1 | h39G5VHv1/h39G5VKv1 | 1.70 | 1.78 |
| Ab2 | h39G5VHv2/h39G5VKv1 | 1.49 | 1.10 |
| Ab3 | h39G5VHv3/h39G5VKv1 | 1.65 | 0.62 |
| Ab4 | h39G5VHv4/h39G5VKv1 | 1.69 | 0.43 |
| Ab5 | h39G5VHv5/h39G5VKv1 | 1.69 | 0.28 |
| Ab8 | h39G5VHv8/h39G5VKv1 | 2.05 | 1.00 |
| Ab10 | h39G5VHv10/h39G5VKv1 | 1.36 | 0.37 |
| Ab13 | h39G5VHv13/h39G5VKv1 | 1.10 | 0.55 |
| Ab19 | h71G4VHv1/h71G4VK1 | 2.74 | 1.76 |
| Ab20 | h71G4VHv2/h71G4VK1 | 2.00 | 1.32 |
| Ab21 | h71G4VHv3/h71G4VK1 | 2.18 | 1.08 |
| Ab22 | h39G5VHv1/h71G4VK1 | 1.80 | 1.00 |
| Ab23 | h39G5VHv2/h71G4VK1 | 1.76 | 1.54 |
| Ab24 | h39G5VHv5/h71G4VK1 | 2.01 | 1.63 |
| Ab25 | h39G5VHv6/h71G4VK1 | 1.82 | 0.85 |
| Ab26 | h39G5VHv10/h71G4VK1 | 1.66 | 0.69 |
| Murine anti-71G4 | mAb71G4 | 1.98 | 0.47 |
| Murine anti-39G5 | mAb39G5 | 1.95 | 1.6 |

### Example 15 Detection of the blockade of the binding of TSLP to TSLPR by humanized antibodies by FACS

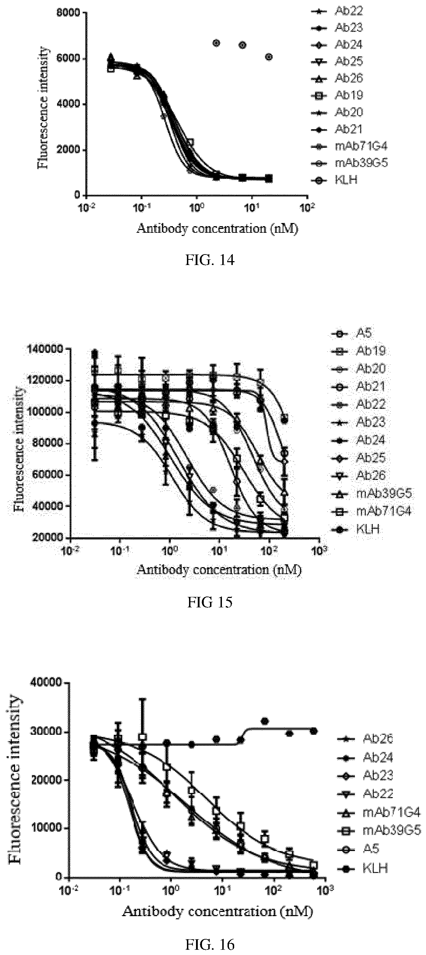

Ba/F3-hTSLPR-IL7Rα cells were washed three times with PBS, centrifuged at 300g for 3 minutes, and blocked with 3% BSA/PBS for 30 minutes. The cells were resuspended and added to a 96-well U-plate at 5×10⁴/well. The antibody was incubated with 60 ng/mL of biotinylated human TSLP protein at room temperature for 10 minutes, then added to the cells, the mixture was incubated at 4°C for 45 minutes and washed twice with 0.5% BSA/PBS, SA-PE was added, the mixture was incubated at 4°C for 45 minutes and washed twice with 0.5% BSA/PBS, PI dye was added, the mixture was incubated at 4°C for 10 minutes and washed twice with 0.5% BSA/PBS, the cells were resuspended in PBS, and the blocking effect of the antibody at the cellular level was detected by flow cytometry. FIG. 14 and Table 11 show that all of the humanized antibodies Ab19, Ab20, Ab21, Ab22, Ab23, Ab24, Ab25, and Ab26 block the binding of TSLP to TSLPR on the cell surface, and there is no significant difference between the antibodies.

**Table 11 Blockade of the binding of TSLPR to TSLP on cells by humanized antibodies**

| Humanized antibody | Heavy chain/light chain | Cellular blockade IC50 (nM) |
|---|---|---|
| Ab19 | h71G4VHv1/h71G4VKv1 | 0.45 |
| Ab20 | h71G4VHv2/h71G4VKv1 | 0.33 |
| Ab21 | h71G4VHv3/h71G4VKv1 | 0.36 |
| Ab22 | h39G5VHv1/h71G4VKv1 | 0.39 |
| Ab23 | h39G5VHv2/h71G4VKv1 | 0.33 |
| Ab24 | h39G5VHv5/h71G4VKv1 | 0.36 |
| Ab25 | h39G5VHv6/h71G4VKv1 | 0.35 |
| Ab26 | h39G5VHv10/h71G4VKv1 | 0.39 |
| Murine anti-71 G4 | mAb71G4 | 0.27 |
| Murine anti-39G5 | mAb39G5 | 0.36 |
| Isotype control | KLH hIgG1 | - |

### Example 16 Inhibition of TSLP-stimulated proliferation of Ba/F3-hTSLPR-IL7Rα cells by humanized antibodies

The ability of different anti-TSLP antibodies to biologically neutralize human TSLP was assessed by applying a short-term proliferation bioassay utilizing cells expressing recombinant human TSLPR.

Cells were added to a 96-well cell culture plate one day in advance at a concentration of 2×10⁴ cells/well. 60 µL of the diluted anti-TSLP antibodies were pre-incubated with 60 µL of 5 ng/mL human TSLP for 15 minutes at room temperature. The pre-incubated antibody-cytokine was added to the cell culture plate at 100 µL/well. The 96-well plate was incubated in a 5% CO₂ incubator at 37°C for 72 hours. After the culture, CellTiter-Glo was added to each well for reaction 10 minutes. Luminescence values were read in a black 96-well plate.

FIG. 15 and Table 12 show that the humanized antibodies Ab22, Ab23, Ab24, and Ab26 significantly inhibited TSLP-stimulated proliferation of Ba/F3-hTSLPR-IL7Rα cells (IC50 approximately equal to 2.20 nM, 1.10 nM, 1.04 nM, and 1.50 nM). The inhibitory effect of Ab22, Ab23, Ab24, and Ab26 was also superior to that of A5 (determined as IC50 = 16.55) when compared to antibody A5 of the same type (see patent US10287348 B2).

**Table 12 Inhibition of cell proliferation by humanized antibodies**

| Humanized antibody | Heavy chain/light chain | Inhibition of Cell Proliferation IC50 (nM) |
|---|---|---|
| Ab19 | h71G4VHv1/h71G4VKv1 | - |
| Ab20 | h71G4VHv2/h71G4VKv1 | 46.16 |
| Ab21 | h71G4VHv3/h71G4VKv1 | 178.3 |
| Ab22 | h39G5VHv1/h71G4VKv1 | 2.198 |
| Ab23 | h39G5VHv2/h71G4VKv1 | 1.101 |
| Ab24 | h39G5VHv5/h71G4VKv1 | 1.037 |
| Ab25 | h39G5VHv6/h71G4VKv1 | - |
| Ab26 | h39G5VHv10/h71G4VKv1 | 1.496 |
| Murine anti-71 G4 | mAb39G5 | 58.91 |
| Murine anti-39G5 | mAb71G4 | 30.71 |
| A5 | H5/L5 | 16.55 |
| Isotype control | KLH hIgG1 | - |

### Example 17 Detection of blockage of TSLP through STAT5 pathway conduction signal by humanized antibody by a reporter gene method

Ba/F3-hTSLPR-IL7Rα-STAT5 luc cells were starved overnight, and were plated onto a 96-well plate at 5×10⁴/well in the next day, with 50 µL per well; human TSLP was diluted and incubated at 37°C for 30 minutes in advance; 50µL of ligand/antibody mixture was pipetted to the cell plate for blending, and the mixture was incubated at 37°C for 6 hours; 100 µL of fluorescence detection reagent was added; the luminescence value of each well was read with a microplate reader.

The results in FIG. 16 and Table 13 show that the four humanized antibodies Ab22, Ab23, Ab24, and Ab26 significantly inhibited TSLP-stimulated signal transduction through the STAT 5 pathway (IC50 = 0.17 nM, 0.17 nM, 0.15 nM, and 0.18 nM), and the inhibitory effect was superior to that of A5 (determined IC50 = 1.93 nM, referring to Kenneth V, 2017 report, IC50 = 1.4 nM).

**Table 13 Inhibition IC50 of TSLP-induced activation of the STAT5 signaling pathway by humanized antibodies**

| Humanized antibody | Heavy chain/light chain | STAT5 signal inhibition IC50 (nM) |
|---|---|---|
| Ab22 | h39G5VHv1/h71G4VKv1 | 0.17 |
| Ab23 | h39G5VHv2/h71G4VKv1 | 0.17 |
| Ab24 | h39G5VHv5/h71G4VKv1 | 0.15 |
| Ab26 | h39G5VHv10/h71G4VKv1 | 0.18 |
| Murine anti-71 G4 | mAb71G4 | 1.14 |
| Murine anti-39G5 | mAb39G5 | 4.92 |
| A5 | H5/L5 | 1.93 |
| Isotype control | KLH hIgG1 | - |

Various changes may be made and equivalents may be substituted for elements thereof without departing from the spirit and scope of the disclosure. Any feature, step, or embodiment of an embodiment of the present disclosure may be used in combination with any other feature, step, or embodiment, unless the context indicates otherwise.

## Claims

1. An antibody or antigen-binding portion thereof that binds to TSLP, comprising a heavy chain CDR selected from the amino acid sequences of SEQ ID Nos: 8-10, 18-20, 28-30, 38-40, 48-50, 58-60, 68-70, 78-80, 83-85, 88-90, 93-95, 98-100, 103-105, 108-110, 113-115, 118-120, 123-125, 128-130, 133-135, 138-140, 143-145, 148-150, 153-155, 158-160, 163-165, or any variant thereof, and/or a light chain CDR selected from the amino acid sequences of SEQ ID NOs:13-15, 23-25, 33-35, 43-45, 53-55, 63-65, 73-75, 168-170, 173-175, or any variant thereof.

2. The antibody or antigen-binding portion thereof according to claim 1, comprising a heavy chain CDR1 selected from the amino acid sequences of SEQ ID NOs: 8, 18, 28, 38, 48, 58, 68, 78, 83,88, 93, 98, 103, 108, 113, 118, 123, 128, 133, 138, 143, 148, 153, 158, 163, or any variant thereof, a heavy chain CDR2 selected from the amino acid sequences of SEQ ID NOs: 9, 19, 29, 39, 49, 59, 69, 79, 84, 89, 94, 99, 104, 109, 114, 119, 124, 129, 134, 139, 144, 149, 154, 159, 164, or any variant thereof, and a heavy chain CDR3 selected from the amino acid sequences of SEQ ID NOs: 10, 20, 30, 40, 50, 60, 70, 80, 85, 90, 95, 100, 105, 110, 115, 120, 125, 130, 135, 140, 145, 150, 155, 160, 165, or any variant thereof; and/or a light chain CDR1 selected from the amino acid sequences of SEQ ID NOs: 13, 23, 33, 43, 53, 63, 73, 168, 173, or any variant thereof, a light chain CDR2 selected from the amino acid sequences of SEQ ID NOs: 14, 24, 34, 44, 54, 64, 74, 169, 174, or any variant thereof, and a light chain CDR3 selected from the amino acid sequences of SEQ ID NOs: 15, 25, 35, 45, 55, 65, 75, 170, 175, or any variant thereof.

3. The antibody or antigen-binding portion thereof according to claim 1 or 2, comprising a CDR combination of heavy and light chains selected from the group consisting of:
(1) the heavy chain CDR1, CDR2, and CDR3 sequences including SEQ ID NOs: 8-10, respectively, and/or the light chain CDR1, CDR2, and CDR3 sequences including SEQ ID NOs: 13-15, respectively;
(2) the heavy chain CDR1, CDR2, and CDR3 sequences including SEQ ID NOs: 18-20, respectively, and/or the light chain CDR1, CDR2, and CDR3 sequences including SEQ ID NOs: 23-25, respectively;
(3) the heavy chain CDR1, CDR2, and CDR3 sequences including SEQ ID NOs: 28-30, respectively, and/or the light chain CDR1, CDR2, and CDR3 sequences including SEQ ID NOs: 33-35, respectively;
(4) the heavy chain CDR1, CDR2, and CDR3 sequences including SEQ ID NOs: 38-40, respectively, and/or the light chain CDR1, CDR2, and CDR3 sequences including SEQ ID NOs: 43-45, respectively;
(5) the heavy chain CDR1, CDR2, and CDR3 sequences including SEQ ID NOs: 48-50, respectively, and/or the light chain CDR1, CDR2, and CDR3 sequences including SEQ ID NOs: 53-55, respectively;
(6) the heavy chain CDR1, CDR2, and CDR3 sequences including SEQ ID NOs: 58-60, respectively, and/or the light chain CDR1, CDR2, and CDR3 sequences including SEQ ID NOs: 63-65, respectively;
(7) the heavy chain CDR1, CDR2, and CDR3 sequences including SEQ ID NOs: 68-70, respectively, and/or the light chain CDR1, CDR2, and CDR3 sequences including SEQ ID NOs: 73-75, respectively;
(8) the heavy chain CDR1, CDR2, and CDR3 sequences including SEQ ID NOs: 78-80, respectively, and/or the light chain CDR1, CDR2, and CDR3 sequences including SEQ ID NOs: 168-170, respectively;
(9) the heavy chain CDR1, CDR2, and CDR3 sequences including SEQ ID NOs: 83-85, respectively, and/or the light chain CDR1, CDR2, and CDR3 sequences including SEQ ID NOs: 168-170, respectively;
(10) the heavy chain CDR1, CDR2, and CDR3 sequences including SEQ ID NOs: 88-90, respectively, and/or the light chain CDR1, CDR2, and CDR3 sequences including SEQ ID NOs: 168-170, respectively;
(11) the heavy chain CDR1, CDR2, and CDR3 sequences including SEQ ID NOs: 93-95, respectively, and/or the light chain CDR1, CDR2, and CDR3 sequences including SEQ ID NOs: 168-170, respectively;
(12) the heavy chain CDR1, CDR2, and CDR3 sequences including SEQ ID NOs: 98-100, respectively, and/or the light chain CDR1, CDR2, and CDR3 sequences including SEQ ID NOs: 168-170, respectively;
(13) the heavy chain CDR1, CDR2, and CDR3 sequences including SEQ ID NOs: 103-105, respectively, and/or the light chain CDR1, CDR2, and CDR3 sequences including SEQ ID NOs: 168-170, respectively;
(14) the heavy chain CDR1, CDR2, and CDR3 sequences including SEQ ID NOs: 108-110, respectively, and/or the light chain CDR1, CDR2, and CDR3 sequences including SEQ ID NOs: 168-170, respectively;
(15) the heavy chain CDR1, CDR2, and CDR3 sequences including SEQ ID NOs: 113-115, respectively, and/or the light chain CDR1, CDR2, and CDR3 sequences including SEQ ID NOs: 168-170, respectively;
(16) the heavy chain CDR1, CDR2, and CDR3 sequences including SEQ ID NOs: 118-120, respectively, and/or the light chain CDR1, CDR2, and CDR3 sequences including SEQ ID NOs: 168-170, respectively;
(17) the heavy chain CDR1, CDR2, and CDR3 sequences including SEQ ID NOs: 123-125, respectively, and/or the light chain CDR1, CDR2, and CDR3 sequences including SEQ ID NOs: 168-170, respectively;
(18) the heavy chain CDR1, CDR2, and CDR3 sequences including SEQ ID NOs: 128-130, respectively, and/or the light chain CDR1, CDR2, and CDR3 sequences including SEQ ID NOs: 168-170, respectively;
(19) the heavy chain CDR1, CDR2, and CDR3 sequences including SEQ ID NOs: 133-135, respectively, and/or the light chain CDR1, CDR2, and CDR3 sequences including SEQ ID NOs: 168-170, respectively;
(20) the heavy chain CDR1, CDR2, and CDR3 sequences including SEQ ID NOs: 138-140, respectively, and/or the light chain CDR1, CDR2, and CDR3 sequences including SEQ ID NOs: 168-170, respectively;
(21) the heavy chain CDR1, CDR2, and CDR3 sequences including SEQ ID NOs: 143-145, respectively, and/or the light chain CDR1, CDR2, and CDR3 sequences including SEQ ID NOs: 168-170, respectively;
(22) the heavy chain CDR1, CDR2, and CDR3 sequences including SEQ ID NOs: 148-150, respectively, and/or the light chain CDR1, CDR2, and CDR3 sequences including SEQ ID NOs: 168-170, respectively;
(23) the heavy chain CDR1, CDR2, and CDR3 sequences including SEQ ID NOs: 153-155, respectively, and/or the light chain CDR1, CDR2, and CDR3 sequences including SEQ ID NOs: 168-170, respectively;
(24) the heavy chain CDR1, CDR2, and CDR3 sequences including SEQ ID NOs: 158-160, respectively, and/or the light chain CDR1, CDR2, and CDR3 sequences including SEQ ID NOs: 168-170, respectively;
(25) the heavy chain CDR1, CDR2, and CDR3 sequences including SEQ ID NOs: 163-165, respectively, and/or the light chain CDR1, CDR2, and CDR3 sequences including SEQ ID NOs: 168-170, respectively;
(26) the heavy chain CDR1, CDR2, and CDR3 sequences including SEQ ID NOs: 153-155, respectively, and/or the light chain CDR1, CDR2, and CDR3 sequences including SEQ ID NOs: 173-175, respectively;
(27) the heavy chain CDR1, CDR2, and CDR3 sequences including SEQ ID NOs: 158-160, respectively, and/or the light chain CDR1, CDR2, and CDR3 sequences including SEQ ID NOs: 173-175, respectively;
(28) the heavy chain CDR1, CDR2, and CDR3 sequences including SEQ ID NOs: 163-165, respectively, and/or the light chain CDR1, CDR2, and CDR3 sequences including SEQ ID NOs: 173-175, respectively;
(29) the heavy chain CDR1, CDR2, and CDR3 sequences including SEQ ID NOs: 78-80, respectively, and/or the light chain CDR1, CDR2, and CDR3 sequences including SEQ ID NOs: 173-175, respectively;
(30) the heavy chain CDR1, CDR2, and CDR3 sequences including SEQ ID NOs: 83-85, respectively, and/or the light chain CDR1, CDR2, and CDR3 sequences including SEQ ID NOs: 173-175, respectively;
(31) the heavy chain CDR1, CDR2, and CDR3 sequences including SEQ ID NOs: 98-100, respectively, and/or the light chain CDR1, CDR2, and CDR3 sequences including SEQ ID NOs: 173-175, respectively;
(32) the heavy chain CDR1, CDR2, and CDR3 sequences including SEQ ID NOs: 103-105, respectively, and/or the light chain CDR1, CDR2, and CDR3 sequences including SEQ ID NOs: 173-175, respectively;
(33) the heavy chain CDR1, CDR2, and CDR3 sequences including SEQ ID NOs: 123-125, respectively, and/or the light chain CDR1, CDR2, and CDR3 sequences including SEQ ID NOs: 173-175, respectively; and
(34) the heavy chain CDR1, CDR2, and CDR3 sequences including SEQ ID NOs: 128-130, respectively, and/or the light chain CDR1, CDR2, and CDR3 sequences including SEQ ID NOs: 173-175, respectively.

4. The antibody or antigen-binding portion thereof according to claim 1 or 2, comprising a heavy chain variable region selected from the amino acid sequence of SEQ ID NOs: 7, 17, 27, 37, 47, 57, 67, 77, 82, 87, 92, 97, 102, 107, 112, 117, 122, 127, 132, 137, 142, 147, 152, 157, 162, or any variant thereof, and/or a light chain variable region selected from the amino acid sequence of SEQ ID NOs: 12, 22, 32, 42, 52, 62, 72, 167, 172, or any variant thereof;
preferably, comprising a heavy chain variable region of the amino acid sequence of SEQ ID NO: 7 or any variant thereof, and a light chain variable region of the amino acid sequence of SEQ ID NO: 12 or any variant thereof;
preferably, comprising a heavy chain variable region of the amino acid sequence of SEQ ID NO: 17 or any variant thereof, and a light chain variable region of the amino acid sequence of SEQ ID NO: 22 or any variant thereof;
preferably, comprising a heavy chain variable region of the amino acid sequence of SEQ ID NO: 27 or any variant thereof, and a light chain variable region of the amino acid sequence of SEQ ID NO: 32 or any variant thereof;
preferably, comprising a heavy chain variable region of the amino acid sequence of SEQ ID NO: 37 or any variant thereof, and a light chain variable region of the amino acid sequence of SEQ ID NO: 42 or any variant thereof;
preferably, comprising a heavy chain variable region of the amino acid sequence of SEQ ID NO: 47 or any variant thereof, and a light chain variable region of the amino acid sequence of SEQ ID NO: 52 or any variant thereof;
preferably, comprising a heavy chain variable region of the amino acid sequence of SEQ ID NO: 57 or any variant thereof, and a light chain variable region of the amino acid sequence of SEQ ID NO: 62 or any variant thereof;
preferably, comprising a heavy chain variable region of the amino acid sequence of SEQ ID NO: 67 or any variant thereof, and a light chain variable region of the amino acid sequence of SEQ ID NO: 72 or any variant thereof;
preferably, comprising a heavy chain variable region of the amino acid sequence of SEQ ID NO: 77 or any variant thereof, and a light chain variable region of the amino acid sequence of SEQ ID NO: 167 or any variant thereof;
preferably, comprising a heavy chain variable region of the amino acid sequence of SEQ ID NO: 82 or any variant thereof, and a light chain variable region of the amino acid sequence of SEQ ID NO: 167 or any variant thereof;
preferably, comprising a heavy chain variable region of the amino acid sequence of SEQ ID NO: 87 or any variant thereof, and a light chain variable region of the amino acid sequence of SEQ ID NO: 167 or any variant thereof;
preferably, comprising a heavy chain variable region of the amino acid sequence of SEQ ID NO: 92 or any variant thereof, and a light chain variable region of the amino acid sequence of SEQ ID NO: 167 or any variant thereof;
preferably, comprising a heavy chain variable region of the amino acid sequence of SEQ ID NO: 97 or any variant thereof, and a light chain variable region of the amino acid sequence of SEQ ID NO: 167 or any variant thereof;
preferably, comprising a heavy chain variable region of the amino acid sequence of SEQ ID NO: 102 or any variant thereof, and a light chain variable region of the amino acid sequence of SEQ ID NO: 167 or any variant thereof;
preferably, comprising a heavy chain variable region of the amino acid sequence of SEQ ID NO: 107 or any variant thereof, and a light chain variable region of the amino acid sequence of SEQ ID NO: 167 or any variant thereof;
preferably, comprising a heavy chain variable region of the amino acid sequence of SEQ ID NO: 112 or any variant thereof, and a light chain variable region of the amino acid sequence of SEQ ID NO: 167 or any variant thereof;
preferably, comprising a heavy chain variable region of the amino acid sequence of SEQ ID NO: 117 or any variant thereof, and a light chain variable region of the amino acid sequence of SEQ ID NO: 167 or any variant thereof;
preferably, comprising a heavy chain variable region of the amino acid sequence of SEQ ID NO: 122 or any variant thereof, and a light chain variable region of the amino acid sequence of SEQ ID NO: 167 or any variant thereof;
preferably, comprising a heavy chain variable region of the amino acid sequence of SEQ ID NO: 127 or any variant thereof, and a light chain variable region of the amino acid sequence of SEQ ID NO: 167 or any variant thereof;
preferably, comprising a heavy chain variable region of the amino acid sequence of SEQ ID NO: 132 or any variant thereof, and a light chain variable region of the amino acid sequence of SEQ ID NO: 167 or any variant thereof;
preferably, comprising a heavy chain variable region of the amino acid sequence of SEQ ID NO: 137 or any variant thereof, and a light chain variable region of the amino acid sequence of SEQ ID NO: 167 or any variant thereof;
preferably, comprising a heavy chain variable region of the amino acid sequence of SEQ ID NO: 142 or any variant thereof, and a light chain variable region of the amino acid sequence of SEQ ID NO: 167 or any variant thereof;
preferably, comprising a heavy chain variable region of the amino acid sequence of SEQ ID NO: 147 or any variant thereof, and a light chain variable region of the amino acid sequence of SEQ ID NO: 167 or any variant thereof;
preferably, comprising a heavy chain variable region of the amino acid sequence of SEQ ID NO: 152 or any variant thereof, and a light chain variable region of the amino acid sequence of SEQ ID NO: 167 or any variant thereof;
preferably, comprising a heavy chain variable region of the amino acid sequence of SEQ ID NO: 157 or any variant thereof, and a light chain variable region of the amino acid sequence of SEQ ID NO: 167 or any variant thereof;
preferably, comprising a heavy chain variable region of the amino acid sequence of SEQ ID NO: 162 or any variant thereof, and a light chain variable region of the amino acid sequence of SEQ ID NO: 167 or any variant thereof;
preferably, comprising a heavy chain variable region of the amino acid sequence of SEQ ID NO: 152 or any variant thereof, and a light chain variable region of the amino acid sequence of SEQ ID NO: 172 or any variant thereof;
preferably, comprising a heavy chain variable region of the amino acid sequence of SEQ ID NO: 157 or any variant thereof, and a light chain variable region of the amino acid sequence of SEQ ID NO: 172 or any variant thereof;
preferably, comprising a heavy chain variable region of the amino acid sequence of SEQ ID NO: 162 or any variant thereof, and a light chain variable region of the amino acid sequence of SEQ ID NO: 172 or any variant thereof;
preferably, comprising a heavy chain variable region of the amino acid sequence of SEQ ID NO: 77 or any variant thereof, and a light chain variable region of the amino acid sequence of SEQ ID NO: 172 or any variant thereof;
preferably, comprising a heavy chain variable region of the amino acid sequence of SEQ ID NO: 82 or any variant thereof, and a light chain variable region of the amino acid sequence of SEQ ID NO: 172 or any variant thereof;
preferably, comprising a heavy chain variable region of the amino acid sequence of SEQ ID NO: 97 or any variant thereof, and a light chain variable region of the amino acid sequence of SEQ ID NO: 172 or any variant thereof;
preferably, comprising a heavy chain variable region of the amino acid sequence of SEQ ID NO: 102 or any variant thereof, and a light chain variable region of the amino acid sequence of SEQ ID NO: 172 or any variant thereof;
preferably, comprising a heavy chain variable region of the amino acid sequence of SEQ ID NO: 122 or any variant thereof, and a light chain variable region of the amino acid sequence of SEQ ID NO: 172 or any variant thereof;
preferably, comprising a heavy chain variable region of the amino acid sequence of SEQ ID NO: 127 or any variant thereof, and a light chain variable region of the amino acid sequence of SEQ ID NO: 172 or any variant thereof.

5. A nucleic acid molecule encoding an antibody or antigen-binding portion thereof according to any one of claims 1 to 4, or a nucleic acid molecule having at least greater than 60%, 65%, 70%, 75%, 80%, 85%, 90%, 95%, 96%, 97%, 98%, 99% or more sequence identity thereto; preferably, the nucleic acid molecule comprises an antibody heavy chain nucleotide sequence selected from SEQ ID NOs: 11, 21, 31, 41, 51, 61, 71, 81, 86, 91, 96, 101, 106, 111, 116, 121, 126, 131, 136, 141, 146, 151, 156, 161, 166, or any variant thereof, and/or an antibody light chain nucleotide sequence selected from SEQ ID NOs: 16, 26, 36, 46, 56, 66, 76, 171, 176, or any variant thereof.

6. A vector comprising the nucleic acid molecule according to claim 5.

7. A cell containing the nucleic acid molecule according to claim 6 or the vector according to claim 8.

8. A composition, comprising the antibody or antigen-binding portion thereof according to any one of claims 1 to 4, the nucleic acid molecule according to claim 5, the vector according to claim 6, and/or the cell according to claim 7.

9. A kit comprising the antibody or antigen-binding portion thereof according to any one of claims 1 to 4, the nucleic acid molecule according to claim 5, the vector according to claim 6, the cell according to claim 7, and/or the composition according to claim 8.

10. Use of the antibody or antigen-binding portion thereof according to any one of claims 1 to 4, the nucleic acid molecule according to claim 5, the vector according to claim 6, the cell according to claim 7, and/or the composition according to claim 8 in the manufacture of a medicament or kit for the treatment of a TSLP-related disorder, preferably the TSLP-related disorder is selected from a TSLP-related inflammatory disorder and an autoimmune disease; preferably, the TSLP-related inflammatory disorder is selected from allergic inflammation, asthma, chronic obstructive pulmonary disease, atopic dermatitis, or eosinophilic esophagitis; preferably, the allergic inflammation is selected from allergic rhinitis, allergic sinusitis or allergic conjunctivitis; preferably, the autoimmune disease is selected from rheumatoid arthritis or multiple sclerosis.
